(19) Europäisches Patentamt
European Patent Office
Office européen des brevets

(11) EP 1 923 075 B1

(12) EUROPEAN PATENT SPECIFICATION

(45) Date of publication and mention of the grant of the patent:
**11.11.2015 Bulletin 2015/46**

(51) Int Cl.:
***C08G 85/00*** *(2006.01)* ***A61K 49/04*** *(2006.01)*

(21) Application number: **08004369.8**

(22) Date of filing: **27.09.2004**

(54) **Radiopaque polymeric stents**

Röntgendichte Polymerstents

Endoprothèses polymériques radiopaques

(84) Designated Contracting States:
**AT BE BG CH CY CZ DE DK EE ES FI FR GB GR HU IE IT LI LU MC NL PL PT RO SE SI SK TR**

(30) Priority: **13.08.2004 US 601743 P**

(43) Date of publication of application:
**21.05.2008 Bulletin 2008/21**

(62) Document number(s) of the earlier application(s) in accordance with Art. 76 EPC:
**04789146.0 / 1 789 097**

(73) Proprietor: **Rutgers, The State University New Brunswick, NJ 08909 (US)**

(72) Inventors:
- **Kohn, Joachim B.
Plainfield, NJ 07080 (US)**
- **Bolikal, Durgadas
Edison, NJ 08820 (US)**
- **Zeltinger, Joan
Encinitas, CA 92024 (US)**
- **Pesnell, Aaron, F.
Cherry Hill, NJ 08002 (US)**
- **Brandom, Donald K.
Davis, CA 95617 (US)**
- **Schmid, Eric
San Diego, CA 92128 (US)**

(74) Representative: **Zacco GmbH
Bayerstrasse 83
80335 München (DE)**

(56) References cited:
**EP-A- 1 158 014**
**WO-A-00/05243**
**WO-A-00/78791**
**WO-A-99/24391**
**DE-A1- 4 411 360**
**US-A- 5 849 839**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF INVENTION

Preferred embodiments of the present invention relate to polymeric medical devices, such as stents. More particularly, the polymeric compositions disclosed herein comprise iodine-containing, tyrosine-derived diphenols, optionally in conjunction with other groups, such as dicarboxylic acids and/or poly(alkylene oxide), such that the stents made from these polymeric compositions are bioresorbable and radiopaque, and exhibit physicomechanical properties consistent with their intended uses.

### BACKGROUND

Vascular stents are used widely in a variety of applications, including, especially, in the treatment of heart disease. It has been reported that in 1998, about 61 million Americans had some form of heart disease, which since about 1990 has been the single leading cause of death in the United States. One type of heart disease, coronary artery disease (CAD), is characterized, at least in part, by the inhibition of blood flow through the arteries that supply blood to the heart muscle due to the buildup of plaque (arteriosclerosis) in the arteries. CAD is suspected to account for 1 out of every 5 deaths that occur in the U.S.A. In 2001, about 1.1 million people had a new or recurrent myocardial infarction (heart attack due to coronary arterial disease). See, for example, Report by the American Heart Association, "Heart and Stroke Statistical Update", 2001, American Heart Association, Dallas, TX. Currently more than 500,000 Americans are treated annually for blocked coronary arteries. This number is expected to double over the next 10 years in light of the aging population.

Vascular stents generally comprise a mesh tube, which is inserted into an artery to keep the artery open after it has been stretched with a balloon during the course of an angioplasty procedure. Typically, the vascular stent is mounted on a balloon catheter that is inserted via the femoral artery and pushed to the desired location in the coronary artery. There, the balloon is inflated, thus expanding the stent and pressing it against the vessel wall to lock it in place.

Most stents are constructed from metal, including, for example, stainless steel or nitinol. While such metal stents possess certain desirable characteristics, such as sufficient radial strength to hold open a subject artery and radio-opacity (allowing an implanted stent to be seen and monitored by X-ray radiography/fluoroscopy), metal stents also exhibit a number of significant disadvantages. For example, the insertion and expansion of a metal stent in an artery tends to further injure the diseased vessel, potentially leading to the development of intimal hyperplasia and further occlusion of the vessel by the resulting in in-growth of smooth muscle cells and matrix proteins through the stent struts. Another disadvantage associated with use of metal stents is that once deployed, they become permanent residents within the vessel walls- long after their usefulness has passed. Indeed, the useful lifespan of a stent is estimated to be in the range of about 6 to 9 months. After this time, the chronic stresses and strains imposed on the vessel architecture by the permanent metal implants are believed to promote in-stent restenosis. Another disadvantage associated with the use of metal stents is that the placement of multiple permanent metal stents within a vessel can be a barrier to subsequent surgical bypass. Further, the deployment of a first metal stent may become a physical hurdle to the later delivery of a second stent at a distal site within the same vessel. In contrast to a metal stent, a bioresorbable stent may not outlive its usefulness within the vessel. Moreover, a bioresorbable stent may be used to deliver a greater dose of a therapeutic, as a drug and/or biological agent could be coated on the stent as well as embedded in the device itself. Further, such a stent could deliver multiple drugs and/or biological agents, at the same time or at various times of its life cycle, to treat specific aspects or events of vascular disease. Additionally, a bioresorbable stent may also allow for repeat treatment of the same approximate region of the blood vessel.

Accordingly, there remains an important unmet need to develop temporary (i.e., bioresorbable) and radiopaque stents, wherein the polymeric materials used to fabricate these stents have the desirable qualities of metal (e.g., sufficient radial strength and radiopacity, etc.), while circumventing or alleviating the many disadvantages or limitations associated with the use of permanent metal stents.

U.S. Patent No. 6,475,477 ("the '477 patent") discloses stents formed from radiopaque biocompatible polymers with hydrolytically unstable polymer backbones and pendant free carboxylic acid groups that promote polymer degradation and resorption. Not only are many of the disclosed polymers less than ideal for use in stents, the polymers with free carboxylic acid groups are prepared from monomers with benzyl-protected free acid moieties that are selectively removed from the polymer via hydrogenolysis in the presence of a palladium catalyst and hydrogen. While such a method is effective for removing the benzyl protecting groups with little or no cleaving of the polymer backbone, the palladium catalyst used therein is relatively expensive, and traces of palladium are difficult to remove from the polymer product.

Because the presence of free carboxylic acid groups is a highly desirable feature, new synthetic methods are needed for the preparation of polymers comprising both free carboxylic acid groups and bioresorbable polymer backbones to meet the heretofore unsatisfied need for bioresorbable and radiopaque stents having the desirable properties of metal

stents.

## SUMMARY OF THE INVENTION

For purposes of summarizing the invention, certain aspects, advantages and novel features of the invention have been described herein above. Of course, it is to be understood that not necessarily all such advantages may be achieved in accordance with any particular embodiment of the invention. Thus, the invention may be embodied or carried out in a manner that achieves or optimizes one advantage or group of advantages as taught or suggested herein without necessarily achieving other advantages as may be taught or suggested herein.

The present invention is defined in the appended independent claim. Preferred embodiments are defined in the dependent claims. A radiopaque, bioresorbable stent is disclosed (not claimed). The stent comprises a bioresorbable polymer comprising sufficient halogen atoms to render the stent inherently radiopaque. The stent may further comprise a configuration selected from the group consisting of a sheet stent, a braided stent, a self-expanding stent, a wire stent, a deformable stent, and a slide-and-lock stent. In another variation, the stent is balloon expandable and comprises at least two substantially non-deforming elements arranged to form a tubular member, the non-deforming elements being slidably or rotationally interconnected for allowing the tubular member to expand from a collapsed diameter to an expanded diameter.

Also disclosed (but not part of the invention) is a radiopaque, bioresorbable stent, comprising a polymer comprising one or more units described by Formula I:

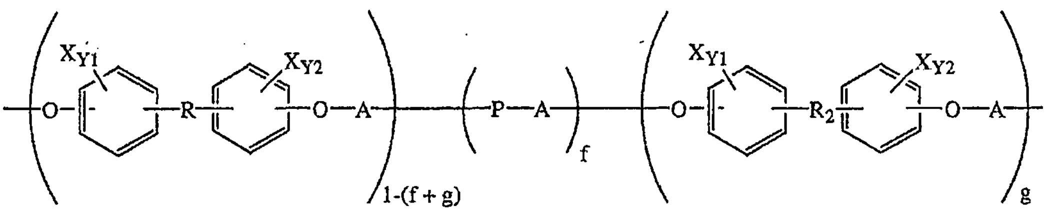
(I)

wherein each X is independently I or Br, Y1 and Y2 for each diphenol unit are independently between 0 and 4, inclusive, and Y1 + Y2 for each diphenol unit is between 1 and 8, inclusive.
wherein each R and $R_2$ are independently an alkyl, aryl or alkylaryl group containing up to 18 carbon atoms and from 0 to 8 heteroatoms selected from O and N, and $R_2$ further comprises a pendant free carboxylic acid group;
wherein A is either:

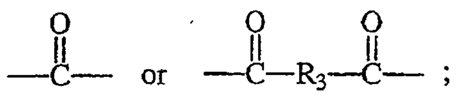
or ;

wherein $R_3$ is a saturated or unsaturated, substituted or unsubstituted alkyl, aryl, or alkylaryl group containing up to about 18 carbon atoms and 0 to 8 heteroatoms selected from O and N;
wherein P is a poly($C_1$-$C_4$ alkylene glycol) unit; f is from 0 to less than 1; g is from 0 to 1, inclusive; and f + g ranges from 0 to 1, inclusive.

Preferably, iodine and bromine are both present as ring substituents. Further, all X groups are preferably ortho-directed. Y1 and Y2 may independently be 2 or less, and Y1 + Y2 =1, 2, 3 or 4. In another variation, Y1 + Y2 = 2 or 3. All X groups are preferably iodine.

In another variation , the weight fraction of the poly($C_1$-$C_4$ alkylene glycol) unit is less than about 75 wt%. In a preferred variation, the weight fraction of the poly($C_1$-$C_4$ alkylene glycol) unit is less than about 50 wt%. More preferably, the poly($C_1$-$C_4$ alkylene glycol) is poly(ethylene glycol) with a weight fraction of less than about 40 wt%. Most preferably, the weight fraction of the poly(ethylene glycol) unit is between about 1 and 25 wt%. P may independently be $C_1$ up to $C_4$ or copolymers of $C_1$-$C_4$.

In another variation , f may vary between about 0 and 0.5, inclusive. Preferably, f is less than about 0.25. More preferably, f is less than about 0.1. More preferably yet, f varies from about 0.001 to about 0.08. Most preferably, f varies between about 0.025 and about 0.035.

In another variation , g is greater than 0 and typically varies between greater than 0 and about 0.5, inclusive.

Preferably, g is greater than about 0.1 to about 0.35. More preferably, g is from about 0.2 to about 0.3. More preferably yet, g varies between about 0.01 and about 0.25. Most preferably, g is between about 0.05 and about 0.15.

In another variation , both R and $R_2$ comprise a pendant $COOR_1$ group; wherein for R, the subgroup $R_1$ is independently an alkyl group ranging from 1 to about 18 carbon atoms containing from 0 to 5 heteroatoms selected from O and N; and

wherein for $R_2$, the subgroup $R_1$ is a hydrogen atom. In another preferred embodiment, each R and $R_2$ independently has the structure:

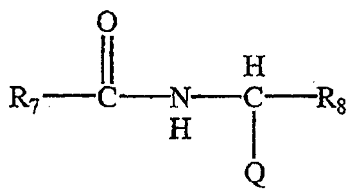

wherein $R_7$ is selected from the group consisting of -CH=CH-, $-CHJ_1-CHJ_2-$ and $(-CH_2-)a$; wherein $R_8$ is selected from the group consisting of -CH=CH-, $-CHJ_1-CHJ_2-$ and $(-CH_2-)n$; wherein a and n are independently between 0 and 8 inclusive; and $J_1$ and $J_2$ are independently Br or I; and wherein, for each $R_2$, Q comprises a free carboxylic acid group, and, for each R, Q is independently selected from the group consisting of hydrogen and carboxylic acid esters and amides, wherein said esters and amides are selected from the group consisting of esters and amides of alkyl and alkylaryl groups containing up to 18 carbon atoms and esters and amides of biologically active compounds.

In another variation, each R and $R_2$ independently has the structure:

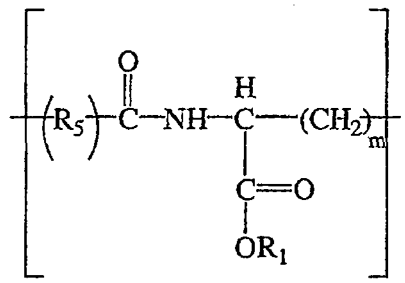

wherein $R_5$ is an alkyl group containing up to 18 carbon atoms and from 0 to 5 heteroatoms selected from O and N; and wherein m is an integer from 1 to 8 inclusive; and

wherein, for each $R_2$, $R_1$ is hydrogen, and, for each R, $R_1$ is independently an alkyl group ranging from 1 to about 18 carbon atoms containing from 0 to 5 heteroatoms selected from O and N.

In another variation, each R and $R_2$ independently has the structure:

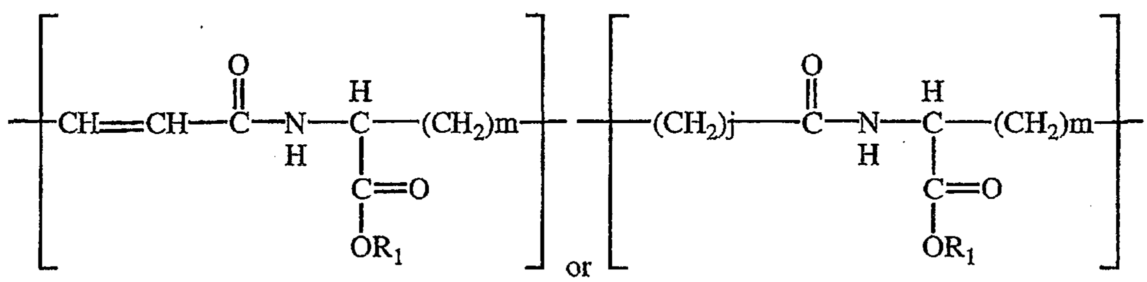

wherein j and m are independently an integer from 1 to 8, inclusive, and wherein, for each $R_2$, $R_1$ is hydrogen, and, for each R, $R_1$ is independently an alkyl group ranging from 1 to about 18 carbon atoms containing from 0 to 5 heteroatoms selected from O and N.

Preferably, each $R_1$ subgroup for R is independently an alkyl group ranging from 1 to about 18 carbon atoms and containing from 0 to 5 heteroatoms selected from O and N. More preferably, each $R_1$ subgroup for R is independently either ethyl or butyl.

In another variation, A is a -C(=O)- group. Alternatively, A may be:

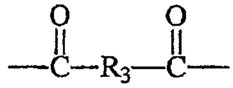

wherein $R_3$ is a $C_4$-$C_{12}$ alkyl, $C_8$ - $C_{14}$ aryl, or $C_8$ - $C_{14}$ alkylaryl. Preferably, $R_3$ is selected so that A is a moiety of a dicarboxylic acid that is a naturally occurring metabolite. More preferably, $R_3$ is selected from the group consisting of $-CH_2-C(=O)-$, $-CH_2-CH_2-C(=O)-$, -CH=CH- and $(-CH_2-)_z$; and wherein z is an integer from 0 to 8, inclusive. More preferably, z is an integer from 1 to 8, inclusive.

In another variation, the stent further comprises an effective amount of a therapeutic agent. Preferably, the amount is sufficient to inhibit restenosis, thrombosis, plaque formation, plaque rupture, and inflammation, and/or promote healing. In another variation, the polymer forms a coating on at least a portion of the stent. The polymer coating is preferably adapted to promote a selected biological response.

In accordance with another variation, a polymer is disclosed comprising one or more units described by Formula I:

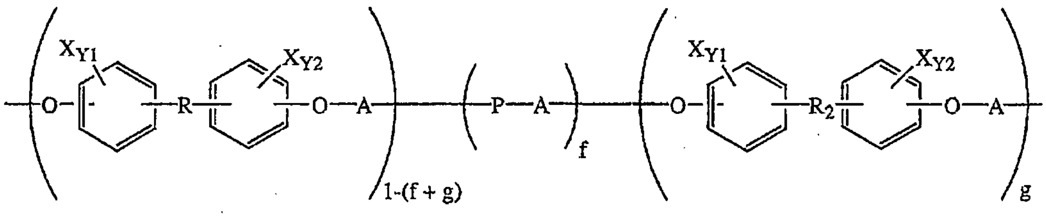

(I)

wherein each X is independently I or Br, Y1 and Y2 for each diphenol unit are independently between 0 and 4, inclusive, and Y1 + Y2 for each diphenol unit is between 1 and 8, inclusive;
wherein each R and $R_2$ are independently an alkyl, aryl or alkylaryl group containing up to 18 carbon atoms and from 0 to 8 heteroatoms selected from O and N, and $R_2$ further comprises a pendant free carboxylic acid group;
wherein A is either:

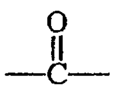 or 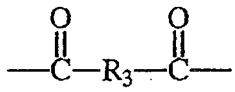 ;

wherein $R_3$ is a saturated or unsaturated, substituted or unsubstituted alkyl, aryl, or alkylaryl group containing up to about 18 carbon atoms and 0 to 8 heteroatoms selected from O and N;
wherein P is a poly($C_1$-$C_4$ alkylene glycol) unit; f is from 0 to less than 1; g is from 0 to 1, inclusive; and f + g ranges from 0 to 1, inclusive.

Preferably, Y1 and Y2 are independently be 2 or less, and Y1 + Y2 = 1, 2, 3 or 4. All X groups are also preferably ortho-directed. In another variation to the polymer of Formula I, Y1 + Y2 = 2 or 3. All X groups are preferably iodine.

Preferably, the weight fraction of the poly($C_1$-$C_4$ alkylene glycol) unit is less than about 75 wt%. In a preferred variation of the polymer of Formula I, the weight fraction of the poly($C_1$-$C_4$ alkylene glycol) unit is less than about 50 wt%. More preferably, P is a poly(ethylene glycol) unit with a weight fraction of less than about 40 wt%. Most preferably, the weight fraction of the poly(ethylene glycol) unit is between about 1 and 25 wt%. P may independently be $C_1$ up to $C_4$ or copolymers of $C_1$-$C_4$.

In another variation to the polymer of Formula I, f may vary between about 0 and 0.5, inclusive. Preferably, f is less than about 0.25. More preferably, f is less than about 0.1. More preferably yet, f varies from about 0.001 to about 0.08. Most preferably, f varies between about 0.025 and about 0.035.

In another variation to the polymer of Formula I, g is greater than 0 and typically varies between greater than 0 and about 0.5, inclusive. Preferably, g is greater than about 0.1 to about 0.35. More preferably, g is from about 0.2 to about 0.3. More preferably yet, g varies between about 0.01 and about 0.25. Most preferably, g is between about 0.05 and about 0.15.

In another variation to the polymer of Formula I, both R and $R_2$ comprise a pendant $COOR_1$ group; wherein for R, the subgroup $R_1$ is independently an alkyl group ranging from 1 to about 18 carbon atoms containing from 0 to 5 heteroatoms selected from O and N; and wherein for $R_2$, the subgroup $R_1$ is a hydrogen atom. In another preferred

embodiment, each R and $R_2$ independently has the structure:

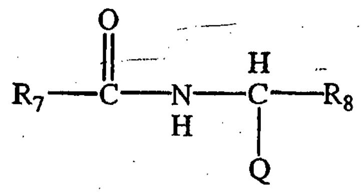

wherein $R_7$ is selected from the group consisting of -CH=CH-, $-CHJ_1-CHJ_2-$ and $(-CH_2-)a$; wherein $R_8$ is selected from the group consisting of -CH=CH-, $-CHJ_1-CHJ_2-$ and $(-CH_2-)n$; wherein a and n are independently between 0 and 8 inclusive; and $J_1$ and $J_2$ are independently Br or I; and wherein, for each $R_2$, Q comprises a free carboxylic acid group, and, for each R, Q is independently selected from the group consisting of hydrogen and carboxylic acid esters and amides, wherein said esters and amides are selected from the group consisting of esters and amides of alkyl and alkylaryl groups containing up to 18 carbon atoms and esters and amides of biologically active compounds.

In a preferred variation, each R and $R_2$ independently has the structure:

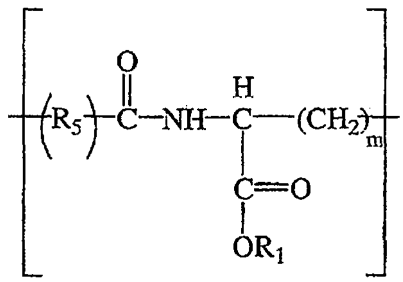

wherein $R_5$ is an alkyl group containing up to 18 carbon atoms and from 0 to 5 heteroatoms selected from O and N; and wherein m is an integer from 1 to 8 inclusive; and wherein, for each $R_2$, $R_1$ is hydrogen, and, for each R, $R_1$ is independently an alkyl group ranging from 1 to about 18 carbon atoms containing from 0 to 5 heteroatoms selected from O and N.

In a more preferred variation to the polymer of Formula I, each R and $R_2$ independently has the structure:

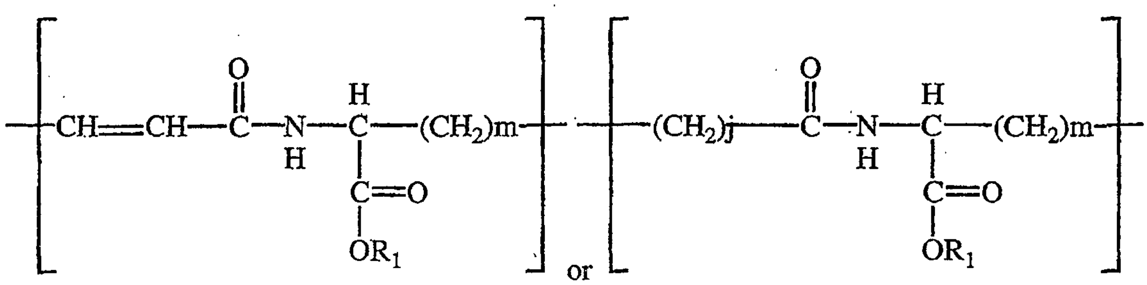

wherein j and m are independently an integer from 1 to 8, inclusive, wherein each $R_1$ subgroup for R is independently an alkyl group ranging from 1 to about 18 carbon atoms and containing from 0 to 5 heteroatoms selected from O and N, and wherein each $R_1$ subgroup for $R_2$ is a hydrogen atom;

In a variation to the polymer of Formula I, each $R_1$ subgroup for R is ethyl or butyl.

In another variation to the polymer of Formula I, A is -C(=O)-. Alternatively, A is:

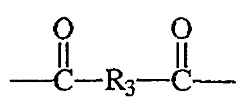

In another variation to the polymer of Formula I, $R_3$ is $C_4$ - $C_{12}$ alkyl, $C_8$ - $C_{14}$ aryl, or $C_8$ - $C_{14}$ alkylaryl. More

preferably, $R_3$ is selected from the group consisting of $-CH_2-C(=O)-$, $-CH_2-CH_2-C(=O)-$, -CH=CH- and $(-CH_2-)z$, wherein z is an integer from 0 to 8, inclusive.

A system is disclosed for treating a site within a body lumen. The system comprises a catheter having a deployment means, and a radiopaque, bioresorbable stent, wherein the catheter is adapted to deliver the stent to the site and the deployment means is adapted to deploy the stent. In preferred embodiments of the system, the catheter is selected from the group consisting of over-the-wire catheters, coaxial rapid-exchange catheters, and multi-exchange delivery catheters.

The present invention relates to a method for the selective removal of a tert-butyl protection group from a hydrolytically unstable polymer to form a new polymer composition having a free carboxylic acid group in place of said tert-butyl protection group. The method comprises dissolving the hydrolytically unstable polymer in a solvent comprising an amount of an acid having a pKa from about 0 to about 4 that is effective to selectively remove by acidolysis the tert-butyl ester group to form the new polymer composition having a free carboxylic acid group.

Preferably, the hydrolytically unstable polymer is soluble in said solvent. In one embodiment, the solvent consists essentially of the acid. In variations, the solvent is selected from the group consisting of chloroform, methylene chloride, tetrahydrofuran, dimethylformamide, and mixtures thereof. The acid may be selected from the group consisting of formic acid, trifluoroacetic acid, chloroacetic acid, and mixtures thereof. Preferably, the acid is formic acid.

In a variation to the method (not specifically claimed), the hydrolytically unstable polymer comprises one or more units described by Formula II:

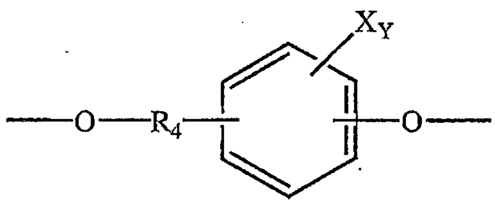

(II)

wherein X for each polymer unit is independently Br or I, Y is between 0 and 4, inclusive, and $R_4$ is an alkyl, aryl or alkylaryl group with up to 18 carbon atoms and from 0 to 8 heteroatoms selected from O and N, and further comprising a pendent tert-butyl ester group.

In a further variation to the method, the hydrolytically unstable polymer is copolymerized with up to about 75 wt% of a poly($C_1$-$C_4$ alkylene glycol). Typically, the poly($C_1$-$C_4$ alkylene glycol) weight fraction is less than about 50 wt%. A poly(ethylene glycol) weight fraction of less than about 40 wt% is preferred, with a weight fraction less than about 25 wt% more preferred. Hydrolytically unstable polymers for stent applications preferably contain a molar fraction of poly(ethylene glycol) between about 0.001 and 0.08.

In a further variation to the method, all X groups are ortho-directed, and Y is 1 or 2. Preferably, every X is iodine.

In a further variation to the method (not specifically claimed), $R_4$ is an alkyl group. $R_4$ may have the structure:

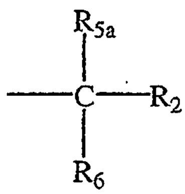

wherein $R_2$ is independently an alkyl, aryl or alkylaryl group containing up to 18 carbon atoms and from 0 to 8 heteroatoms selected from O or N, and further comprises a pendant t-butyl ester group; and $R_{5a}$ and $R_6$ are each independently selected from hydrogen and straight and branched alkyl groups having up to 18 carbon atoms and from 0 to 8 heteroatoms selected from O and N.

Alternatively, $R_2$ may comprise:

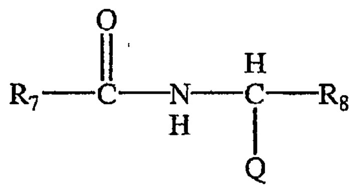

wherein $R_7$ is selected from the group consisting of -CH=CH-, $-CHJ_1-CHJ_2-$ and $(-CH_2-)a$; wherein $R_8$ is selected from the group consisting of -CH=CH-, $-CHJ_1-CHJ_2-$ and $(-CH_2-)n$; wherein a and n are independently between 0 and 8 inclusive; and $J_1$ and $J_2$ are independently Br or I; and Q comprises a carboxylic acid tert-butyl ester.

In a variation to the method (not specifically claimed), $R_2$ has the structure:

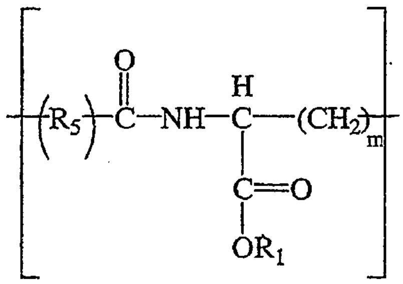

wherein $R_5$ is an alkyl group containing up to 18 carbon atoms and from 0 to 5 heteroatoms selected from O and N; and wherein m is an integer from 1 to 8 inclusive; and $R_1$ is a tert-butyl ester group

In a preferred variation to the method, $R_2$ has the structure:

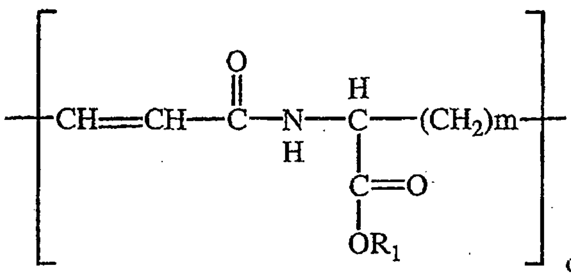

or

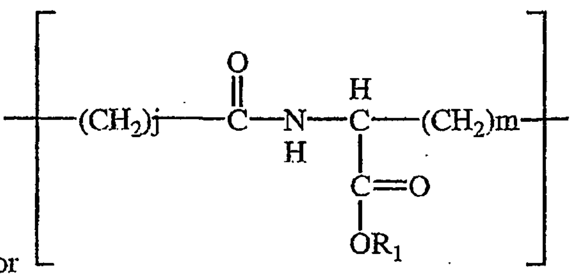

wherein j and m are independently an integer from 1 to 8, inclusive, and $R_1$ is a tert-butyl group.

In a further variation to the method, $R_4$ is an aryl or alkylaryl group. The units described by Formula II comprise a diphenol unit. More specifically, $R_4$ is an alkylaryl group and the diphenol unit is described by Formula III:

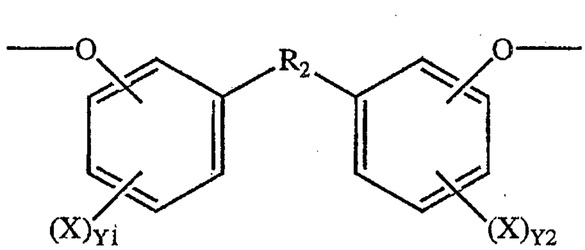

(III)

wherein X for each polymer unit is independently Br or I, Y1 and Y2 are independently between 0 and 4 inclusive, Y1 + Y2 is between 0 and 8, inclusive, and $R_2$ for each unit is independently an alkyl, aryl or alkylaryl group containing up to 18 carbon atoms and from 0 to 8 heteroatoms selected from O and N, and $R_2$ further comprises a pendant t-butyl protection group.

In a further variation to the method, $R_2$ comprises:

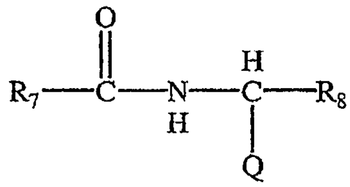

wherein $R_7$ is selected from the group consisting of -CH=CH-, $-CHJ_1-CHJ_2-$ and $(-CH_2-)a$; wherein $R_8$ is selected from the group consisting of -CH=CH-, $-CHJ_1-CHJ_2-$ and $(-CH_2-)n$; wherein a and n are independently between 0 and 8 inclusive; and $J_1$ and $J_2$ are independently Br or I; and Q comprises a carboxylic acid tert-butyl ester.

In a preferred variation to the method, $R_2$ has the structure:

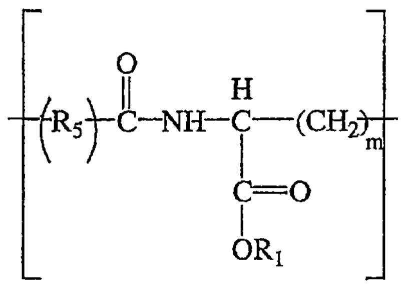

wherein $R_5$ is an alkyl group containing up to 18 carbon atoms and from 0 to 5 heteroatoms selected from O and N; and wherein m is an integer from 1 to 8 inclusive; and $R_1$ is a tert-butyl group

In a more preferred variation to the method, $R_2$ has the structure:

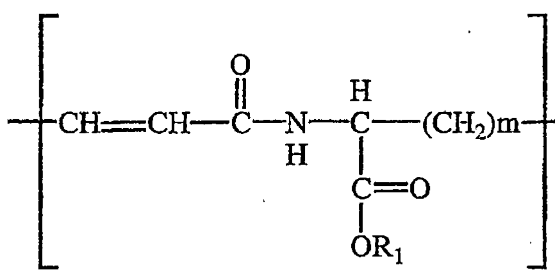

or

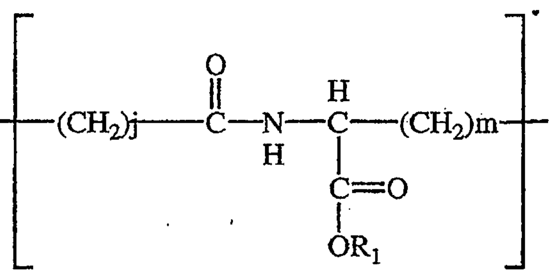

wherein j and m are independently an integer from 1 to 8, inclusive, and $R_1$ is a tert-butyl group.

In a further variation to the method, the hydrolytically unstable polymer is copolymerized with up to about 75 wt% of a poly($C_1$-$C_4$ alkylene glycol). Typically, the poly($C_1$-$C_4$ alkylene glycol) weight fraction is less than about 50 wt%. A poly(ethylene glycol) weight fraction of less than about 40 wt% is preferred, with a weight fraction less than about 25 wt% more preferred. Hydrolytically unstable polymers for stent applications preferably contain a molar fraction of poly(ethylene glycol) between about 0.001 and 0.08.

All X groups are preferably ortho-directed, and Y1 +Y2 = 1, 2, 3 or 4. Every X is preferably iodine.

In a further variation to the method, the hydrolytically unstable polymer may comprise one or more units defined by Formula I:

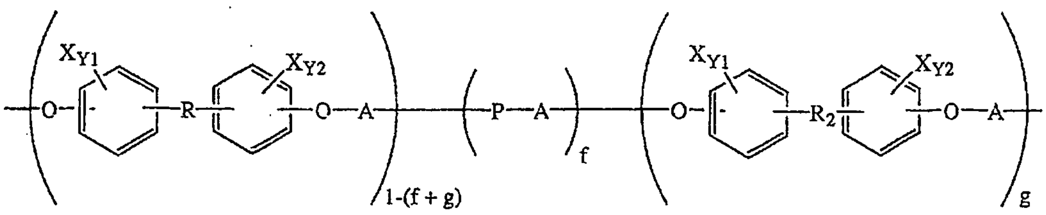

(I)

wherein each X is independently I or Br, Y1 and Y2 for each diphenol unit are independently between 0 and 4, inclusive, and Y1 + Y2 for each diphenol unit is between 0 and 8, inclusive.

wherein R and $R_2$ for each unit are independently an alkyl, aryl or alkylaryl group containing up to 18 carbon atoms and from 0 to 8 heteroatoms selected from O and N, and $R_2$ further comprises a pendant t-butyl ester group;

wherein A is either:

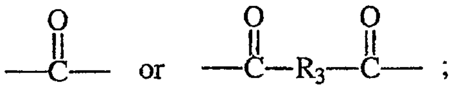

wherein $R_3$ is a saturated or unsaturated, substituted or unsubstituted alkyl, aryl, or alkylaryl group containing up to about 18 carbon atoms and 0 to 8 heteroatoms selected from O and N;

wherein P is a poly($C_1$-$C_4$ alkylene glycol) unit having a weight fraction less than about 75 wt%; f is from 0 to less than 1; g is from 0 to 1, inclusive; and f + g ranges from 0 to1, inclusive.

R and $R_2$ may comprise:

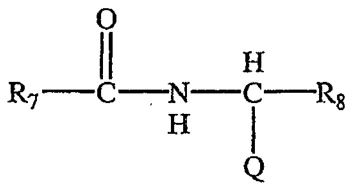

wherein $R_7$ is selected from the group consisting of -CH=CH-, $-CHJ_1-CHJ_2-$ and $(-CH_2-)a$; wherein $R_8$ is selected from the group consisting of -CH=CH-, $-CHJ_1-CHJ_2-$ and $(-CH_2-)n$; wherein a and n are independently between 0 and 8 inclusive; and $J_1$ and $J_2$ are independently Br or I; and wherein, for $R_2$, Q comprises a carboxylic acid t-butyl ester, and, for each R, Q is independently selected from the group consisting of hydrogen and carboxylic acid esters and amides, wherein said esters and amides are selected from the group consisting of esters and amides of alkyl and alkylaryl groups containing up to 18 carbon atoms and esters and amides of biologically active compounds.

In a variation to the method (not specifically claimed), R and $R_2$ independently have the structure:

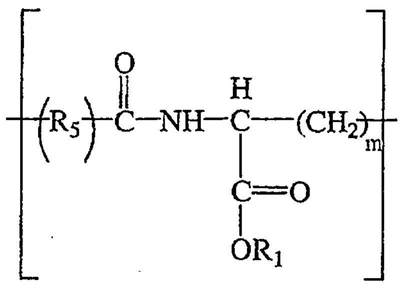

wherein $R_5$ is an alkyl group containing up to 18 carbon atoms and from 0 to 5 heteroatoms selected from O and N; and wherein m is an integer from 1 to 8 inclusive; and wherein, for $R_2$, $R_1$ is a tert-butyl group, and for each R, $R_1$ is independently an alkyl group ranging from 1 to about 18 carbon atoms containing from 0 to 5 heteroatoms selected from O and N.

In a more preferred variation to the method (not specifically claimed), R and $R_2$ independently have the structure:

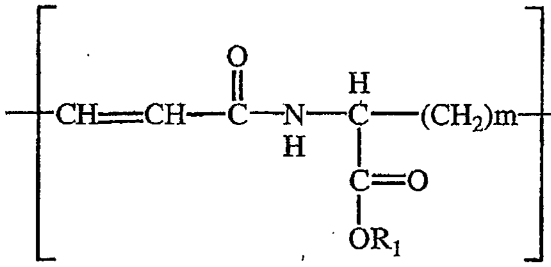

or

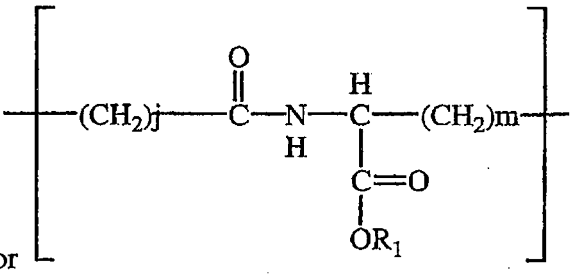

wherein j and m are independently an integer from 1 to 8, inclusive, and wherein, for each R, the subgroup $R_1$ is independently a straight-chain or branched alkyl group ranging from 1 to about 18 carbon atoms containing from 0 to 5 heteroatoms selected from O and N; and, for $R_2$, the subgroup $R_1$ is a tert-butyl (tB) group.

A polymer is disclosed comprising one or more units described by Formula II:

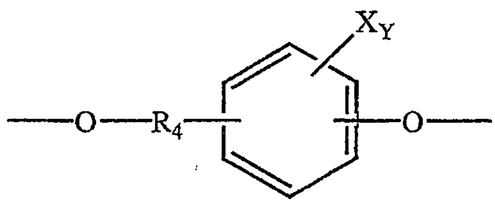

(II)

wherein X for each polymer unit is independently Br or I, Y is between 0 and 4, inclusive, and $R_4$ is an alkyl, aryl or alkylaryl group with up to 18 carbon atoms and from 0 to 8 heteroatoms selected from O and N, and further comprising a pendent tert-butyl ester group.

The polymer may be copolymerized with up to about 75 wt% of a poly($C_1$-$C_4$ alkylene glycol). Typically, the poly($C_1$-$C_4$ alkylene glycol) weight fraction is less than about 50 wt%. A poly(ethylene glycol) weight fraction of less than about 40 wt% is preferred, with a weight fraction less than about 25 wt% more preferred. Polymers for stent applications preferably contain a molar fraction of poly(ethylene glycol) between about 0.001 and 0.08.

Preferably, all X groups are ortho-directed, Y is 1 or 2, and every X is iodine.

In a further variation to the polymer of Formula II, $R_4$ is an alkyl group.

More preferably, $R_4$ has the structure:

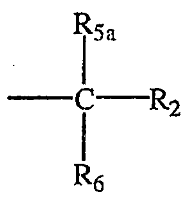

wherein $R_2$ is independently an alkyl, aryl or alkylaryl group containing up to 18 carbon atoms and from 0 to 8 heteroatoms selected from O or N, and further comprises a pendant t-butyl ester group; and $R_{5a}$ and $R_6$ are each independently selected from hydrogen and straight and branched alkyl groups having up to 18 carbon atoms and from 0 to 8 heteroatoms selected from O and N.

More preferably still, $R_2$ comprises:

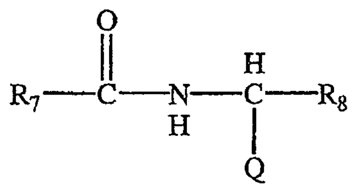

wherein $R_7$ is selected from the group consisting of -CH=CH-, $-CHJ_1-CHJ_2-$ and $(-CH_2-)a$; wherein $R_8$ is selected from the group consisting of -CH=CH-, $-CHJ_1-CHJ_2-$ and $(-CH_2-)n$; wherein a and n are independently between 0 and 8 inclusive; and $J_1$ and $J_2$ are independently Br or I; and Q comprises a carboxylic acid tert-butyl ester.

In a variation, $R_2$ has the structure:

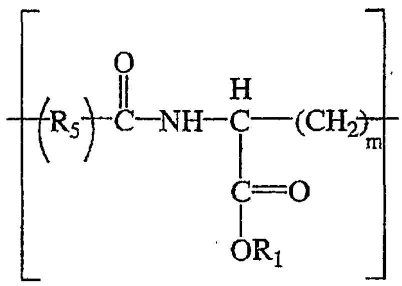

wherein $R_5$ is an alkyl group containing up to 18 carbon atoms and from 0 to 5 heteroatoms selected from O and N; and wherein m is an integer from 1 to 8 inclusive; and $R_1$ is a tert-butyl ester group

In a variation, $R_2$ has the structure:

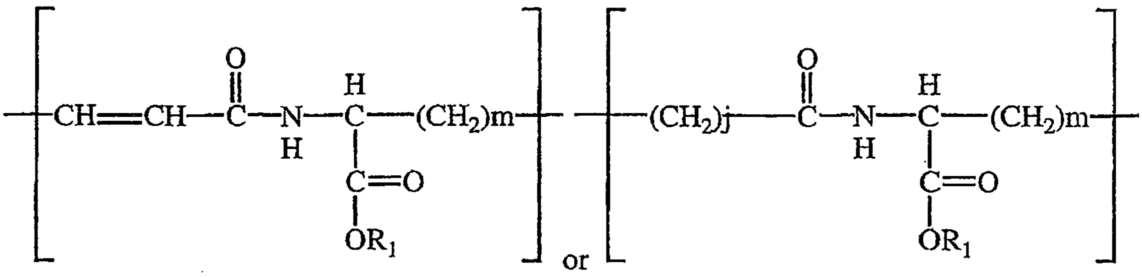

wherein j and m are independently an integer from 1 to 8, inclusive, and wherein each $R_1$ is a tert-butyl ester group.

In a further variation to the polymer of Formula II, $R_4$ may be an aryl or alkylaryl group. The units may also comprise a diphenol unit. In one preferred variation, $R_4$ is an alkylaryl group and the diphenol unit is described by Formula III:

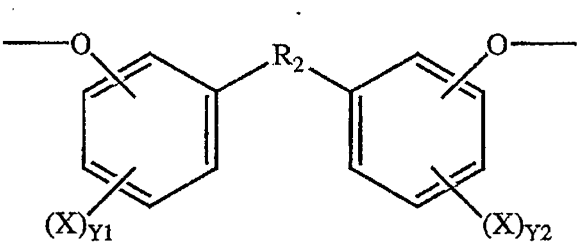

(III)

wherein X for each polymer unit is independently Br or I, Y1 and Y2 are independently between 0 and 4 inclusive, Y1 + Y2 is between 0 and 8, inclusive, and $R_2$ for each unit is independently an alkyl, aryl or alkylaryl group containing up to 18 carbon atoms and from 0 to 8 heteroatoms selected from O and N, and $R_2$ further comprises a pendant t-butyl ester

group. Species of Formula III polymers include the polymers of Formula I with free carboxylic acid groups, in which the carboxylic acid groups are protected with tert-butyl esters. Halogen-free polymers according to Foprmula I afre also included.

In a further variation, $R_2$ comprises:

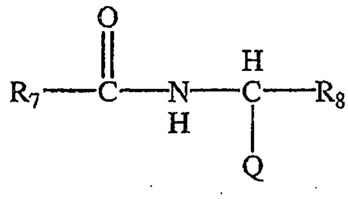

wherein $R_7$ is selected from the group consisting of -CH=CH-, $-CHJ_1-CHJ_2-$ and $(-CH_2-)a$; wherein $R_8$ is selected from the group consisting of -CH=CH-, $-CHJ_1-CHJ_2-$ and $(-CH_2-)n$; wherein a and n are independently between 0 and 8 inclusive; and $J_1$ and $J_2$ are independently Br or I; and Q comprises a carboxylic acid tert-butyl ester.

In a preferred variation, $R_2$ has the structure:

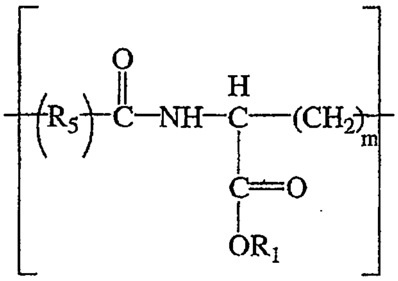

wherein $R_5$ is an alkyl group containing up to 18 carbon atoms and from 0 to 5 heteroatoms selected from O and N; and wherein m is an integer from 1 to 8 inclusive; and $R_1$ is a tert-butyl protection group

In a more preferred variation, $R_2$ has the structure:

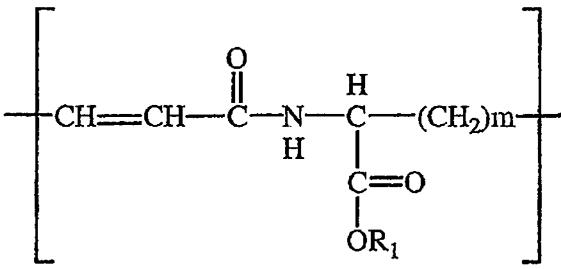

or

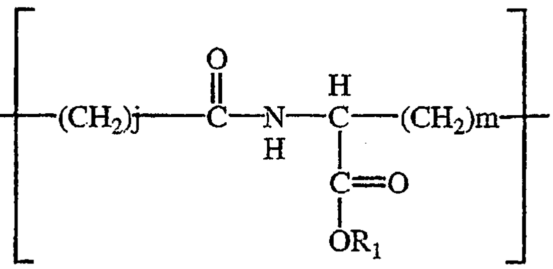

wherein j and m are independently an integer from 1 to 8, inclusive, and $R_1$ is a tert-butyl protection group.

In a further variation, the polymer is copolymerized with up to about 75 wt% of a poly($C_1$-$C_4$ alkylene glycol). Typically, the poly($C_1$-$C_4$ alkylene glycol) weight fraction is less than about 50 wt%. A poly(ethylene glycol) weight fraction of less than about 40 wt% is preferred, with a weight fraction less than about 25 wt% more preferred. Polymers for stent applications preferably contain a molar fraction of poly(ethylene glycol) between about 0.001 and 0.08.'

All X groups are preferably ortho-directed, and Y1 +Y2 = 1, 2, 3 or 4. Every X is preferably iodine.

In a further variation, a hydrolytically unstable polymer may comprise one or more units defined by Formula I:

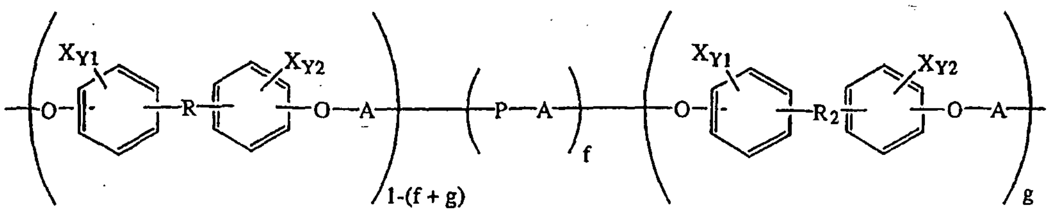

(I)

wherein each X is independently I or Br, Y1 and Y2 for each diphenol unit are independently between 0 and 4, inclusive, and Y1 + Y2 for each diphenol unit is between 0 and 8, inclusive.
wherein R and $R_2$ for each unit are independently an alkyl, aryl or alkylaryl group containing up to 18 carbon atoms and from 0 to 8 heteroatoms selected from O and N, and $R_2$ further comprises a pendant t-butyl ester group;
wherein A is either:

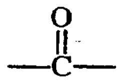

or

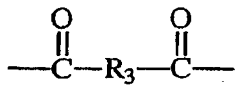

;

wherein $R_3$ is a saturated or unsaturated, substituted or unsubstituted alkyl, aryl, or alkylaryl group containing up to about 18 carbon atoms and 0 to 8 heteroatoms selected from O and N;
wherein P is a poly($C_1$-$C_4$ alkylene glycol) unit having a weight fraction less than about 75 wt%; f is from 0 to less than 1; g is from 0 to 1, inclusive; and f + g ranges from 0 to1, inclusive.

More preferably, R and $R_2$ may comprise:

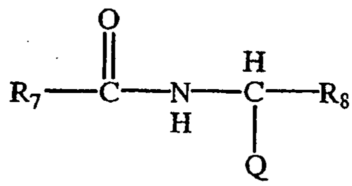

wherein $R_7$ is selected from the group consisting of -CH=CH-, -$CHJ_1$-$CHJ_2$- and (-$CH_2$-)a; wherein $R_8$ is selected from the group consisting of -CH=CH-, -$CHJ_1$-$CHJ_2$- and (-$CH_2$-)n; wherein a and n are independently between 0 and 8 inclusive; and $J_1$ and $J_2$ are independently Br or I; and wherein, for $R_2$, Q comprises a carboxylic acid t-butyl ester, and, for each R, Q is independently selected from the group consisting of hydrogen and carboxylic acid esters and amides, wherein said esters and amides are selected from the group consisting of esters and amides of alkyl and alkylaryl groups containing up to 18 carbon atoms and esters and amides of biologically active compounds. In a preferred variation, R and $R_2$ independently have the structure:

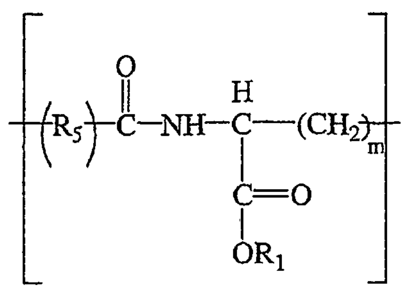

wherein $R_5$ is an alkyl group containing up to 18 carbon atoms and from 0 to 5 heteroatoms selected from O and N; and wherein m is an integer from 1 to 8 inclusive; and wherein, for $R_2$, $R_1$ is a tert-butyl protection group, and for each R, $R_1$ is independently an alkyl group ranging from 1 to about 18 carbon atoms containing from 0 to 5 heteroatoms selected from O and N.

In a more preferred variation, R and $R_2$ independently have the structure:

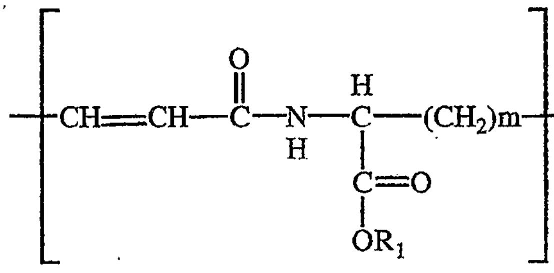

or

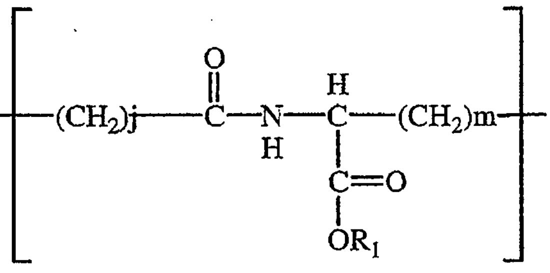

wherein j and m are independently an integer from 1 to 8, inclusive, and wherein, for each R, the subgroup $R_1$ is independently a straight-chain or branched alkyl group ranging from 1 to about 18 carbon atoms containing from 0 to 5 heteroatoms selected from O and N; and, for $R_2$, the subgroup $R_1$ is a tert-butyl (tB) protection group.

A compound is disclosed (but not claimed) having a structure described by Formula IIa:

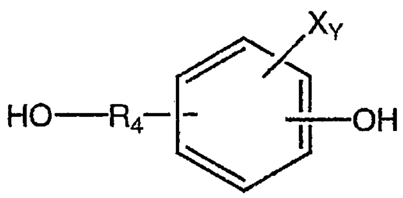

(IIa)

wherein X is Br or I, Y is between 0 and 4, inclusive, and $R_4$ is an alkyl, aryl or alkylaryl group with up to 18 carbon atoms and from 0 to 8 heteroatoms selected from O and N, and further comprises a pendent tert-butyl ester group.

All X groups are preferably ortho-directed, Y =1, 2, 3 or 4, and every X group is iodine.

The $R_4$ may be an alkyl group, preferably, having the structure:

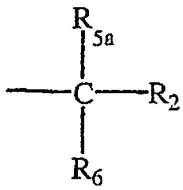

wherein $R_2$ is independently an alkyl, aryl or alkylaryl group containing up to 18 carbon atoms and from 0 to 8 heteroatoms selected from O or N, and further comprises a pendant t-butyl ester group; and $R_{5a}$ and $R_6$ are each independently selected from hydrogen and straight and branched alkyl groups having up to 18 carbon atoms and from 0 to 8 heteroatoms selected from O and N.

In a variation to the compound of Formula IIa, $R_2$ comprises:

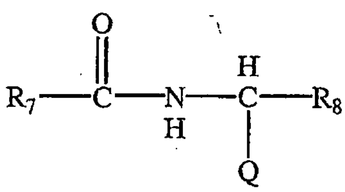

wherein $R_7$ is selected from the group consisting of -CH=CH-, $-CHJ_1-CHJ_2-$ and $(-CH_2-)a$; wherein $R_8$ is selected from the group consisting of -CH=CH-, $-CHJ_1-CHJ_2-$ and $(-CH_2-)n$; wherein a and n are independently between 0 and 8 inclusive; and $J_1$ and $J_2$ are independently Br or I; and Q comprises a carboxylic acid tert-butyl ester.

In a variation, $R_2$ has the structure:

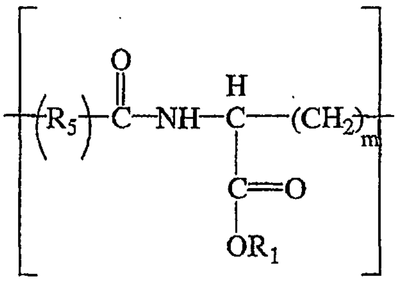

wherein $R_5$ is an alkyl group containing up to 18 carbon atoms and from 0 to 5 heteroatoms selected from O and N; and wherein m is an integer from 1 to 8 inclusive; and $R_1$ is a tert-butyl protection group

In a variation, $R_2$ has the structure:

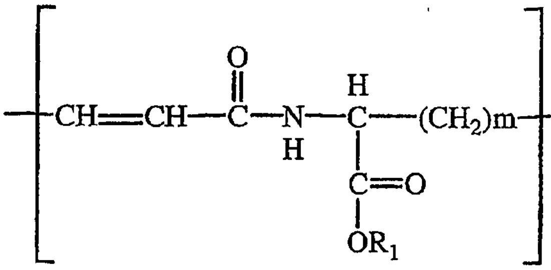

or

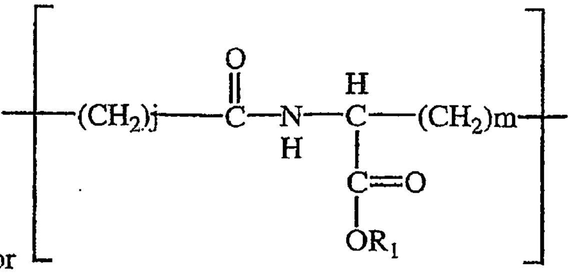

wherein j and m are independently an integer from 1 to 8, inclusive, and wherein each $R_1$ is a tert-butyl protection group.

In another variation, $R_4$ of the compound of Formula IIa is selected so that the compound comprises a diphenol unit, preferably as described by Formula IIIa:

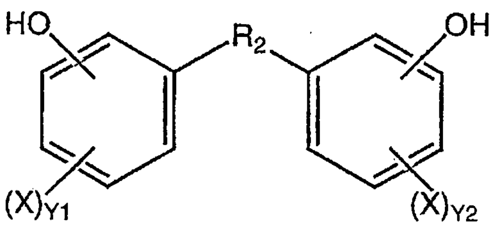

(IIIa)

wherein each X is independently Br or I, Y1 and Y2 are independently between 0 and 4 inclusive, Y1 + Y2 is between 0 and 8, inclusive, and $R_2$ is independently an alkyl, aryl or alkylaryl group containing up to 18 carbon atoms and from 0 to 8 heteroatoms selected from O and N, and $R_2$ further comprises a pendant t-butyl ester group.

In a variation to the diphenol, $R_2$ comprises:

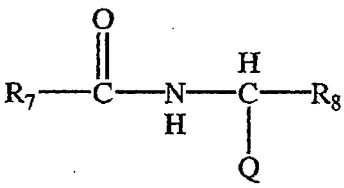

wherein $R_7$ is selected from the group consisting of -CH=CH-, $-CHJ_1-CHJ_2-$ and $(-CH_2-)a$; wherein $R_8$ is selected from the group consisting of -CH=CH-, $-CHJ_1-CHJ_2-$ and $(-CH_2-)n$; wherein a and n are independently between 0 and 8 inclusive; and $J_1$ and $J_2$ are independently Br or I; and Q comprises a carboxylic acid tert-butyl ester.

In a variation, $R_2$ has the structure:

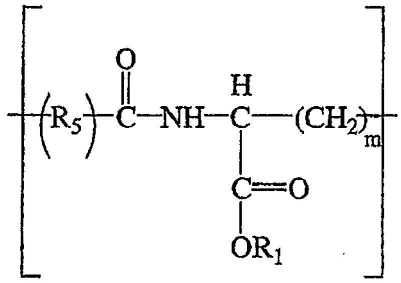

wherein $R_5$ is an alkyl group containing up to 18 carbon atoms and from 0 to 5 heteroatoms selected from O and N; and wherein m is an integer from 1 to 8 inclusive; and $R_1$ is a tert-butyl ester group

In a variation, $R_2$ has the structure:

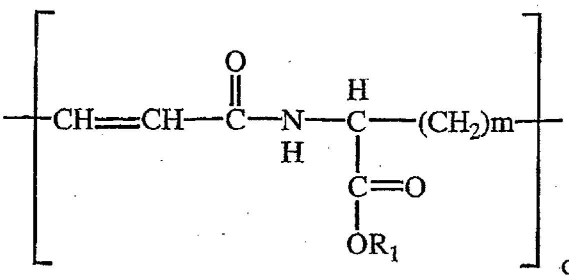

or

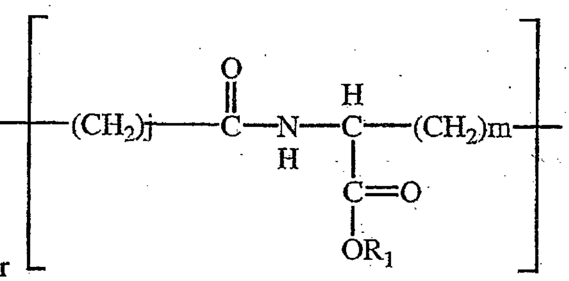

wherein j and m are independently an integer from 1 to 8, inclusive, and $R_1$ is a tert-butyl protection group.

A compound is disclosed (not claimed) having the structure:

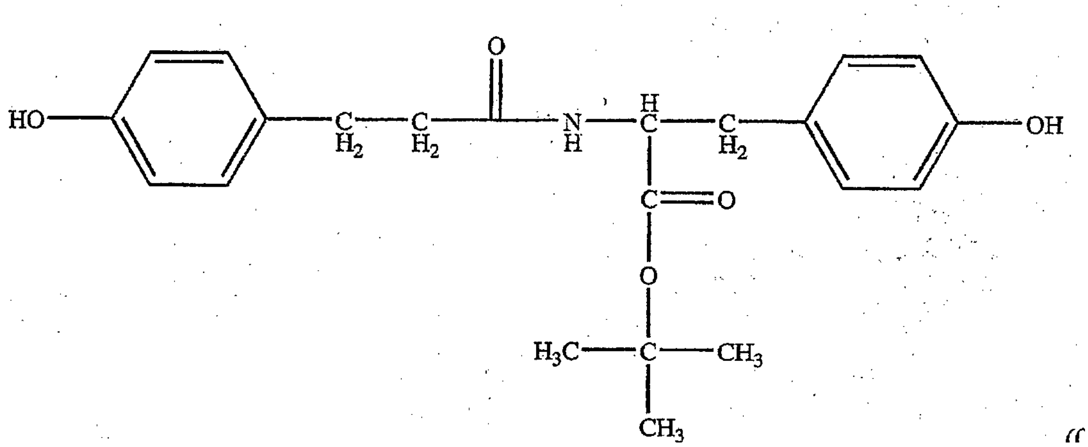

Another compound is disclosed, having the structure:

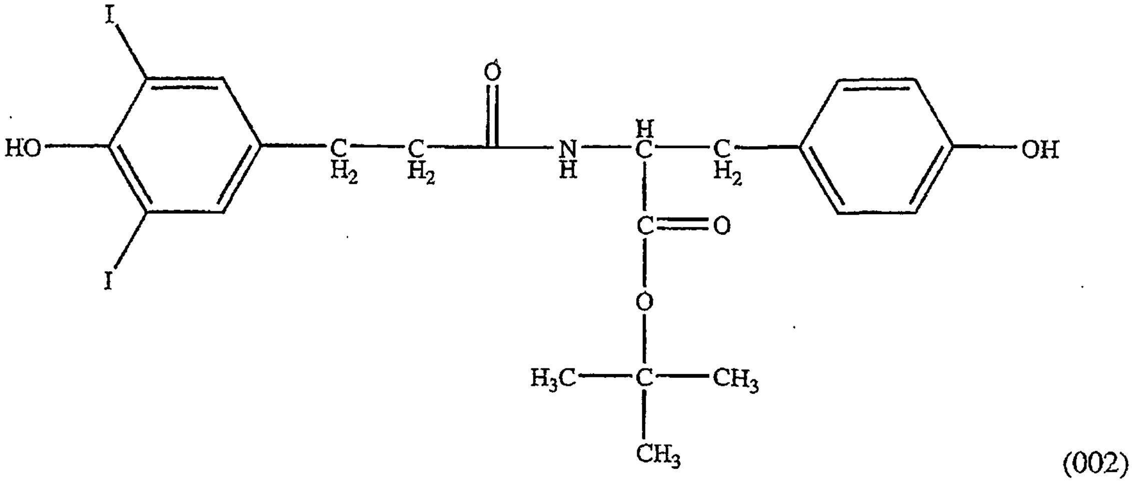

(002)

Another compound is disclosed, having the structure:

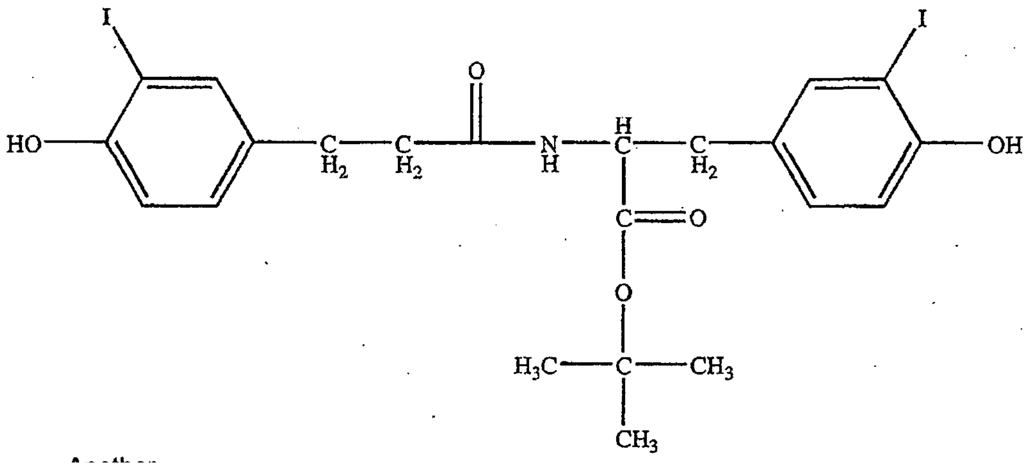

(003)

Another compound is disclosed having the structure:

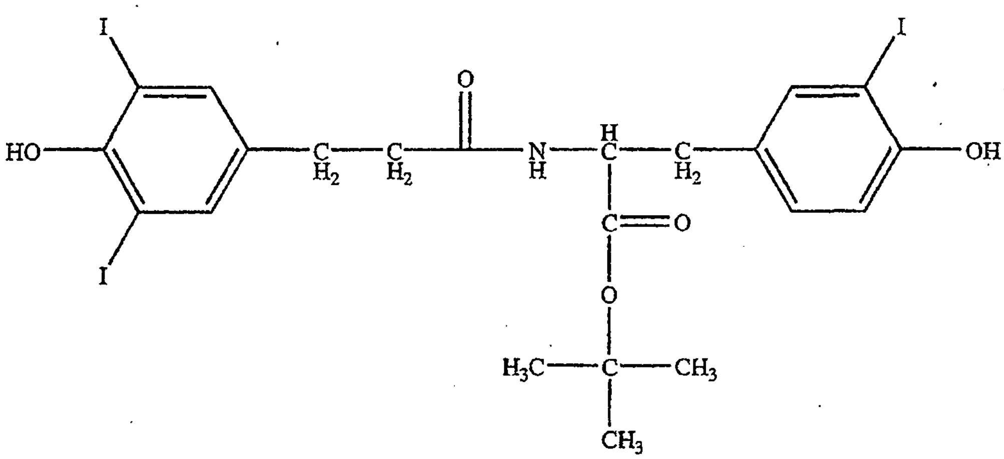

(004)

A compound is disclosed, having the structure:

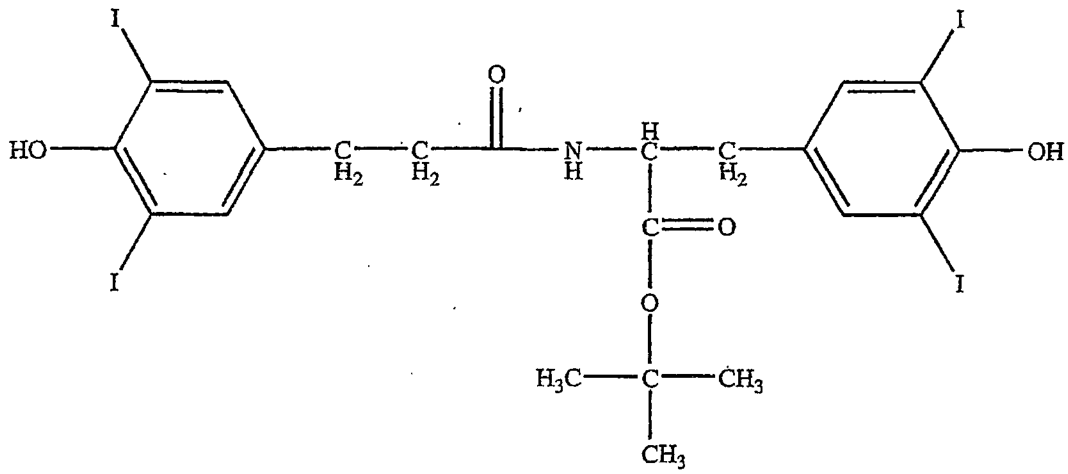

(005)

A method for retreatment of a body lumen is disclosed. The method comprises the steps of: deploying a first device comprising a radiopaque, bioresorbable stent along a region within the body lumen, wherein the first device resides therein for a first treatment period until the stent is bioresorbed; and deploying a second device subsequent to the first treatment period along the region, such that the body lumen is retreated.

In a variation to the radiopaque, bioresorbable stent, the polymer is not naturally occurring. In another variation, the polymer further comprises an amino acid.

## BRIEF DESCRIPTION OF THE DRAWINGS

FIG. 1 depicts an X-ray comparison of a polymer stent according to one preferred embodiment of the present invention to a prior art steel stent in a pig heart;

FIG. 2A is a light micrograph depicting magnified sections of a poly($I_2$-DTE carbonate) stent;

FIG. 2B is a light micrograph depicting magnified sections of a poly($I_2$-DTE-co-2.5%PEG2K carbonate) stent according to another preferred embodiment of the present invention;

FIG. 3 depicts the dissolution of paclitaxel out of poly-DTE-carbonate coatings into PBS with Tween 20 at 37C; and

FIG. 4a-b depicts an X-ray comparison of a radiopaque bioresorbable tri-iodinated tyrosine-derived polycarbonate films showing the radiopacity according to one preferred embodiment of the present invention.

## BEST MODES OF CARRYING OUT THE INVENTION

An inherently radiopaque, bioresorbable stent, comprising a bioresorbable polymer having sufficient halogen atoms to render the stent visible by conventional x-ray fluoroscopy is disclosed (but not part of the invention). New compositions and methods for the preparation of halogenated, bioresorbable polymers exhibiting uniquely optimized properties are also disclosed herein.

Within this framework, a particular challenge was the synthesis of polymers comprising a preselected proportion of repeat units having free carboxylic acid groups. It is well-known among synthetic polymer chemists that polymers containing free carboxylic acids groups as pendent chains cannot be synthesized by condensation-type polymerization reactions as the free carboxylic acid groups have a strong tendency to interfere with most condensation reactions. Therefore, an indirect route of synthesis was employed. First, monomers were prepared that comprise carboxylic acid groups chemically rendered inactive by a selectively removable "protecting group", as described for peptides in general and for the amino acid L-tyrosine in particular by M. Bodanszky (Principles of peptide synthesis, 1984, Springer Verlag, Berlin, Germany). Next, the protected monomers were subjected to the condensation polymerization reactions, resulting in the formation of a polymer comprising protected carboxylic acid groups. In the final reaction step, the protecting groups were selectively removed without cleavage of the polymer backbone and without causing other, non-desirable structural changes to the polymer. When the polymer is designed to be bioresorbable, the polymer backbone is intentionally configured to be readily degradable - making it extremely challenging to remove the protecting groups without concomitant damage to the polymer backbone. Applicants describe herein compositions and methods which meet this challenge and yield polymeric stents having desirable and unexpected properties.

An optimized polymer for use in the fabrication of a stent should fulfill at least some of the following criteria:

- Radiopacity is preferably sufficient to ensure visibility of the stent structure against the background of a human chest by X-ray fluoroscopy, the standard method used in the clinic;
- Stent struts are preferably as thin as possible, preferably 635 micrometer or less in thickness, and more preferably 100 micrometers or less in thickness, yet strong enough to prevent the collapse of the blood vessel and resistant to crushing forces. According to one preferred embodiment, the stent may exhibit an elastic modulus of about 50,000 to 500,000 PSI, and more preferably at least about 200,000 PSI, and a tensile strength at yield of greater than about 1,000 PSI, and more preferably greater than about 5,000 PSI.
- The stents are preferably hemocompatible to prevent acute thrombosis. Accordingly, the device surfaces are preferably resistant to protein adsorption and platelet/monocyte attachment. Further, the device surfaces ideally favor endothelial overgrowth but discourage attachment and growth of smooth muscle cells (which are responsible for the occurrence of restenosis).
- Stents preferably maintain their mechanical strength (e.g., hoop strength) for a period of about 1-24 months, more preferably about 3-18 months, more preferably still about 3-12 months, and most preferably about 3-6 months.
- Stents preferably have a desirable biodegradation and bioresorption profile such that the stents reside for a period of time in the body lumen such that at a later time any stent, bioresorbable or metal or other, may be used to re-treat the approximate same region of the blood vessel or allow for other forms of vessel re-intervention such as vessel bypass.

The term "stent" is used broadly herein to designate embodiments of an expandable tubular member for placement in (1) vascular body lumens (i.e., arteries and/or veins) such as coronary vessels, neurovascular vessels and peripheral vessels for instance renal, iliac, femoral, popliteal, subclavian and carotid; and in (2) nonvascular body lumens such as those treated currently i.e., digestive lumens (e.g., gastrointestinal, duodenum and esophagus, biliary ducts), respiratory lumens (e.g., tracheal and bronchial), and urinary lumens (e.g., urethra); (3) additionally such embodiments may be useful in lumens of other body systems such as the reproductive, endocrine, hematopoietic and/or the integumentary, musculoskeletal/orthopedic and nervous systems (including auditory and ophthalmic applications); and (4) finally, stent embodiments may be useful for expanding an obstructed lumen and for inducing an obstruction (e.g., as in the case of blocking off an aneurysm sac).

The term "bioresorbable" is used herein to designate polymers that undergo biodegradation (through the action of water and/or enzymes to be chemically degraded) and at least some of the degradation products are eliminated and/or absorbed by the body. The term "radiopaque" is used herein to designate an object or material comprising the object visible by in vivo analysis techniques for imaging such as, but not limited to, methods such as x-ray radiography, fluoroscopy, other forms of radiation, MRI, electromagnetic energy, - structural imaging (such as computed or computerized tomography), and functional imaging (such as ultrasonography). The term, "inherently radiopaque", is used herein to designate polymer that is intrinsically radiopaque due to the covalent bonding of halogen species to the polymer. Accordingly, the term does not encompass a polymer which is simply blended with a halogenated species or other radiopacifying agents such as metals and their complexes.

In order to meet the important needs with respect to development of bioresorbable, radiopaque stents, Applicants have developed certain preferred polymers containing combinations of structural units selected from dicarboxylic acids, halogenated (e.g., iodinated or brominated) derivatives of desaminotyrosyl-tyrosine and poly(alkylene glycols), which exhibit desirable physicomechanical and physicochemical properties that are consistent with their use in fabrication of medical devices, including stents. Significantly, while Applicants previously described in U.S. Patent No. 6,475,477, a wide variety of polymers having various combinations of properties and characteristics, Applicants have discovered that the particular polymers of the instant invention exhibit a combination of properties that is significantly and surprisingly superior to the previously-described polymers, and particularly well suited for use in implantable medical devices. Accordingly, the stents described in accordance with preferred embodiments of the present invention: (a) are sufficiently radiopaque to be visible by conventional X-ray fluoroscopy; (b) are of sufficient strength to support medically relevant levels of radial compression within an artery or surrounding tissue; (c) have surface properties which minimize fibrinogen adsorption on the polymer surface and thus reduce the occurrence of acute thrombosis as well as decrease the potential for smooth muscle cell proliferation and attachment; and (d) have a desirable resorption profile that can be adjusted to account for the needs of a range of applications requiring the presence of a stent for different lengths of time or for the elution of therapeutics.

Although Applicants do not wish to be bound by or to any particular theory of operation, Applicants believe that the beneficial combination of properties associated with the medical devices of the present invention are attributable, at least in part, to certain characteristics of the polymers of Formula I, from which the devices may be made. Specifically, Applicants believe that the particular level of halogen (e.g., iodine) substitution, the particular ratio of desaminotyrosyl-tyrosine alkyl ester to desaminotyrosyl-tyrosine used, and the particular amount and molecular weight of poly(alkylene

glycol) (e.g., poly(ethylene glycol); "PEG") units incorporated into the preferred polymers contribute to the significantly superior combination of properties.

The term, "ortho-directed", is used herein to designate orientation of the halogen atom(s) relative to the phenoxy alcohol group.

It is also understood that the presentation of the various polymer formulae that polymer structures represented may include homopolymers and heteropolymers which includes stereoisomers. Homopolymer is used herein to designate a polymer comprised of all the same type of monomers. Heteropolymer is used herein to designate a polymer comprised of two or more different types of monomer, which is also called a co-polymer. A heteropolymer or co-polymer may be of a kind known as block, random and alternating. Further with respect to the presentation of the various polymer formulae, products according to embodiments of the present invention may be comprised of a homopolymer, heteropolymer and/or a blend of such polymers.

**Preferred Polymers**

Therefore, a halogen-substituted polymer is disclosed containing one or more units described by Formula I:

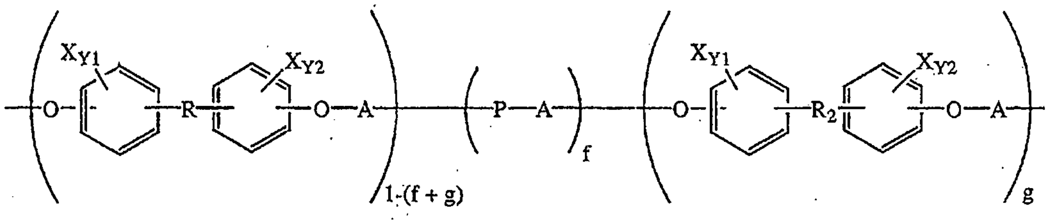

(I)

wherein each X is independently I or Br, Y1 and Y2 for each diphenol unit are independently between 0 and 4, inclusive, and Y1 + Y2 for each diphenol unit is between 1 and 8, inclusive.

wherein each R and $R_2$ are independently an alkyl, aryl or alkylaryl group containing up to 18 carbon atoms and from 0 to 8 heteroatoms selected from O and N, and $R_2$ further comprises a pendant free carboxylic acid group;

wherein A is either:

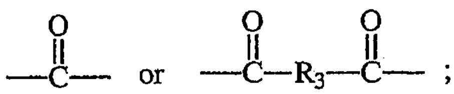

wherein $R_3$ is a saturated or unsaturated, substituted or unsubstituted alkyl, aryl, or alkylaryl group containing up to about 18 carbon atoms and 0 to 8 heteroatoms selected from O and N;

wherein P is a poly($C_1$-$C_4$ alkylene glycol) unit; f is from 0 to less than 1; g is from 0 to 1, inclusive; and f + g ranges from 0 to 1, inclusive.

In preferred variations to Formula I, iodine and bromine are both present as ring substituents. In other preferred variations, all X groups are onho-directed. Preferably, Y1 and Y2 are independently 2 or less, and Y1 + Y2 = 1, 2, 3 or 4, and more preferably 2 or 3. In more preferred variations to Formula I, all X groups are iodine.

In a variation of Formula I, the weight fraction of P, i.e., the poly($C_1$-$C_4$ alkylene glycol), is less than about 75 wt%, and more preferably, less than about 50 wt%. The poly(alkylene glycol) preferably has a molecular weight of 10,000 or less. In another variation, the poly($C_1$-$C_4$ alkylene glycol) is poly(ethylene glycol) with a weight fraction of less than about 40 wt%, and most preferably, between about 1 and 25 wt%. It is understood that P may independently be $C_1$ up to $C_1$ as well as copolymers of $C_1$-$C_4$, the later of which are represented in any combination.

f may vary between about 0 and 0.5, inclusive, more preferably, f is less than about 0.25 and yet more preferably, less than about 0.1. In a more preferred variations, f may vary from greater than about 0.001 to about 0.08, and most preferably, between about 0.025 and about 0.035.

In certain variations of Formula I, g is greater than 0 and typically varies between about 0 and 0.5. More preferably, g is greater than about 0.1 to about 0.35, and yet more preferably, g is from about 0.2 to about 0.3. In more preferred variations, g may vary between about 0.01 and about 0.25, and more preferably, between about 0.05 and about 0.15.

In other preferred variations to Formula I, both R and $R_2$ comprise a pendant $COOR_1$ group; wherein for each R, the subgroup $R_1$ is independently an alkyl group ranging from 1 to about 18 carbon atoms containing from 0 to 5

heteroatoms selected from O and N; and wherein for $R_2$, the subgroup $R_1$ is a hydrogen atom.

In other preferred variations to Formula I, each R and $R_2$ independently has the structure:

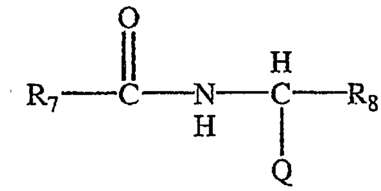

wherein $R_7$ is selected from the group consisting of -CH=CH-, $-CHJ_1-CHJ_2-$ and $(-CH_2-)a$, wherein $R_8$ is selected from the group consisting of -CH=CH-, $-CHJ_1-CHJ_2-$ and $(-CH_2-)n$, wherein a and n are independently between 0 and 8 inclusive; and $J_1$ and $J_2$ are independently Br or I; and wherein, for $R_2$, the subgroup Q comprises a free carboxylic acid group, and, for each R, the subgroup Q is independently selected from the group consisting of hydrogen and carboxylic acid esters and amides, wherein said esters and amides are selected from the group consisting of esters and amides of alkyl and alkylaryl groups containing up to 18 carbon atoms and esters and amides of biologically and pharmaceutically active compounds.

In other preferred variations to Formula I, each R and $R_2$ independently has the structure:

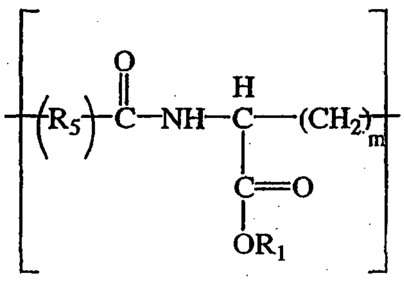

wherein $R_5$ is an alkyl group containing up to 18 carbon atoms and from 0 to 5 heteroatoms selected from O and N; and wherein m is an integer from 1 to 8 inclusive; and wherein, for $R_2$, the subgroup $R_1$ is a hydrogen atom, and, for each R, $R_1$ is independently an alkyl group ranging from 1 to about 18 carbon atoms containing from 0 to 5 heteroatoms selected from O and N.

In more preferred variations to Formula I, each R and $R_2$ independently has the structure:

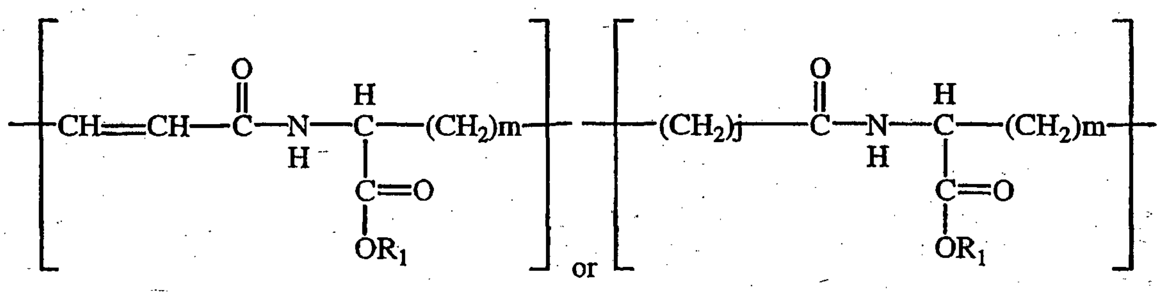

or

wherein j and m are independently an integer from 1 to 8, inclusive, and wherein, for $R_2$, the subgroup $R_1$ is a hydrogen atom, and, for each R, $R_1$ is independently an alkyl group ranging from 1 to about 18 carbon atoms containing from 0 to 5 heteroatoms selected from O and N. Preferably, each $R_1$ subgroup for R is independently an alkyl group ranging from 1 to about 18 carbon atoms and containing from 0 to 5 heteroatoms selected from O and N, and more preferably either ethyl or butyl.

In other preferred variations to Formula I, A is a -C(=O)- group. In another preferred variation to Formula I, A is:

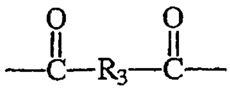

wherein $R_3$ is a $C_4$-$C_{12}$ alkyl, $C_8$ - $C_{14}$ aryl, or $C_8$ - $C_{14}$ alkylaryl. Preferably, $R_3$ is selected so that A is a moiety of a dicarboxylic acid that is a naturally occurring metabolite. More preferably, $R_3$ is a moiety selected from $-CH_2-C(=O)-$, $-CH_2-CH_2-C(=O)-$, -CH=CH- and $(-CH_2-)_z$, wherein z is an integer from 0 to 8, and more preferably, from 1 to 8, inclusive.

Also disclosed (but not claimed) are polymers comprising one or more units described by Formula II are disclosed:

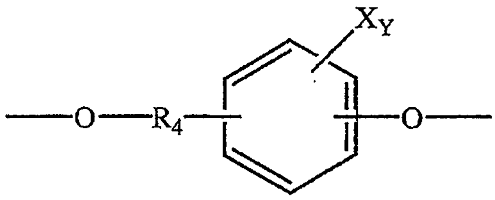

(II)

wherein X for each polymer unit is independently Br or I, Y is between 0 and 4, inclusive, and $R_4$ is an alkyl, aryl or alkylaryl group with up to 18 carbon atoms and from 0 to 8 heteroatoms selected from O and N, and further includes a pendent tert-butyl ester group.

When $R_4$ is an alkyl, it preferably has the structure:

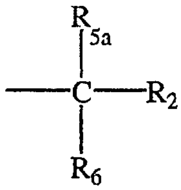

wherein $R_2$ is as defined herein with respect to Formula II, including all disclosed variations; and $R_{5a}$ and $R_6$ are each independently selected from hydrogen and straight and branched alkyl groups having up to 18 carbon atoms and from 0 to 8 heteroatoms independently selected from O and N.

The hydrolytically unstable polymer is optionally copolymerized with up to about 75 wt% of a poly($C_1$-$C_4$ alkylene glycol). Typically, the poly($C_1$-$C_4$ alkylene glycol) weight fraction is less than about 50 wt%. A poly(ethylene glycol) weight fraction of less than about 40 wt% is preferred, with a weight fraction less than about 25 wt% more preferred. Hydrolytically unstable polymers for stent applications preferably contain a molar fraction of poly(ethylene glycol) between about 0.001 and 0.08.

Preferred $R_4$ aryl or alkylaryl species are selected so that the unit described by Formula II is a diphenol. In even more preferred species, the $R_4$ phenolic ring is iodinated or brominated to provide a radiopaque polymer.

Also disclosed are polymers comprising one or more diphenolic units described by Formula III are disclosed:

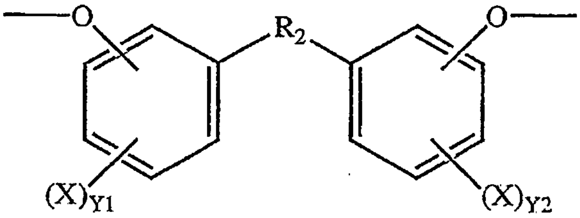

(III)

wherein X, Y1, Y2 and $R_2$ are the same as described herein with respect to Formula III, including all disclosed variations, and Y1 + Y2 is between 0 and 8, inclusive. The polymer may be optionally copolymerized with up to about 75 wt% of a poly($C_1$-$C_4$ alkylene glycol). Typically, the poly($C_1$-$C_4$ alkylene glycol) weight fraction is less than about 50 wt%. A

poly(ethylene glycol) weight fraction of less than about 40 wt% is preferred, with a weight fraction less than about 25 wt% more preferred. Hydrolytically unstable polymers for stent applications preferably contain a molar fraction of poly(ethylene glycol) between about 0.001 and 0.08.

Species of Formula III polymers include the polymers of Formula I with free carboxylic acid groups, in which the carboxylic acid groups are protected with tert-butyl esters. The Formula I polymers may also be halogen-free. Thus polymers according to Formula I are disclosed in which X, Y1, Y2, R, $R_2$, P, A, f and g are the same as described above with respect to Formula I, including all disclosed variations, except that the free carboxylic acid groups of $R_2$ are tert-butyl protected, and Y1 + Y2 can also equal zero.

Also disclosed are t-butyl protected polymers of Formulae I, II and III, which possess heretofore-unknown utility in the preparation of polymers with hydrolytically unstable backbones and pendant carboxylic acid groups, as well as the t-butyl protected monomers from which the polymers are polymerized.

Monomers are disclosed having a structure described by Formula IIa:

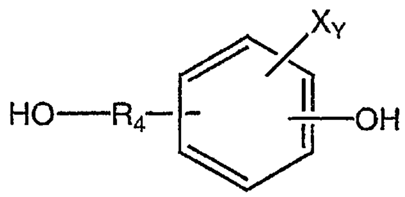

(IIa)

wherein X is Br or I, Y is between 0 and 4, inclusive, and $R_4$ is an alkyl, aryl or alkylaryl group with up to 18 carbon atoms and from 0 to 8 heteroatoms selected from O and N, and further comprises a pendent tert-butyl ester group.

All X groups are preferably ortho-directed, Y = 1, 2, 3 or 4, and every X group is iodine.

The $R_4$ may be an alkyl group, preferably, having the structure:

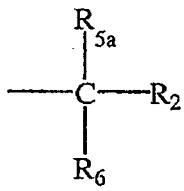

wherein $R_2$ and the variations thereof are the same as described above with respect to Formula IIa; and $R_{5a}$ and $R_6$ are each independently selected from hydrogen and straight and branched alkyl groups having up to 18 carbon atoms and from 0 to 8 heteroatoms selected from O and N.

In another variation, $R_4$ of the compound of Formula IIa is selected so that the compound comprises a diphenol unit, preferably as described by Formula IIIa:

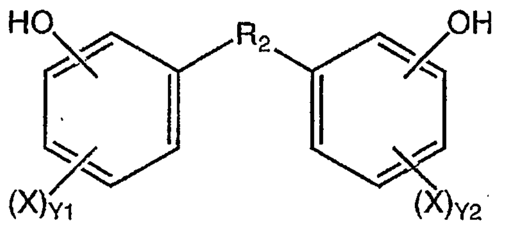

(IIIa)

wherein each X is independently Br or I, Y1 and Y2 are independently between 0 and 4 inclusive, Y1 + Y2 is between 0 and 8, inclusive, and $R_2$ and the variations thereof are the same as described above with respect to Formula IIIa.

The following monomers are described:

Compound 001:

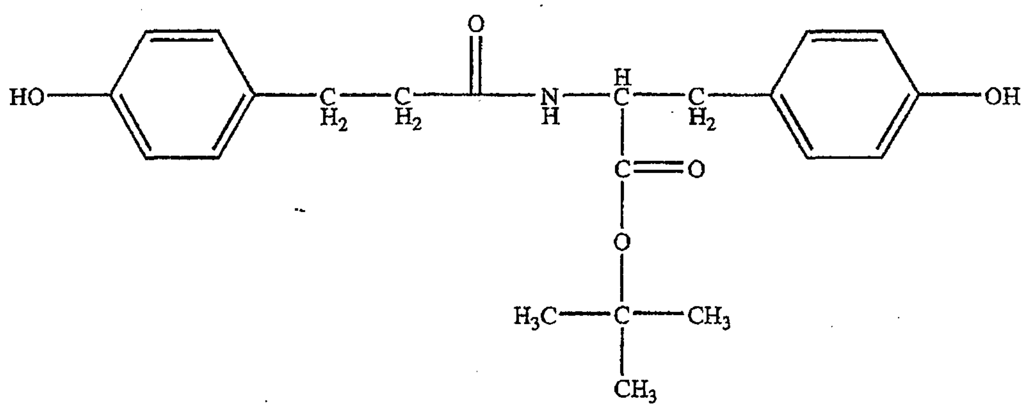

Compound 002:

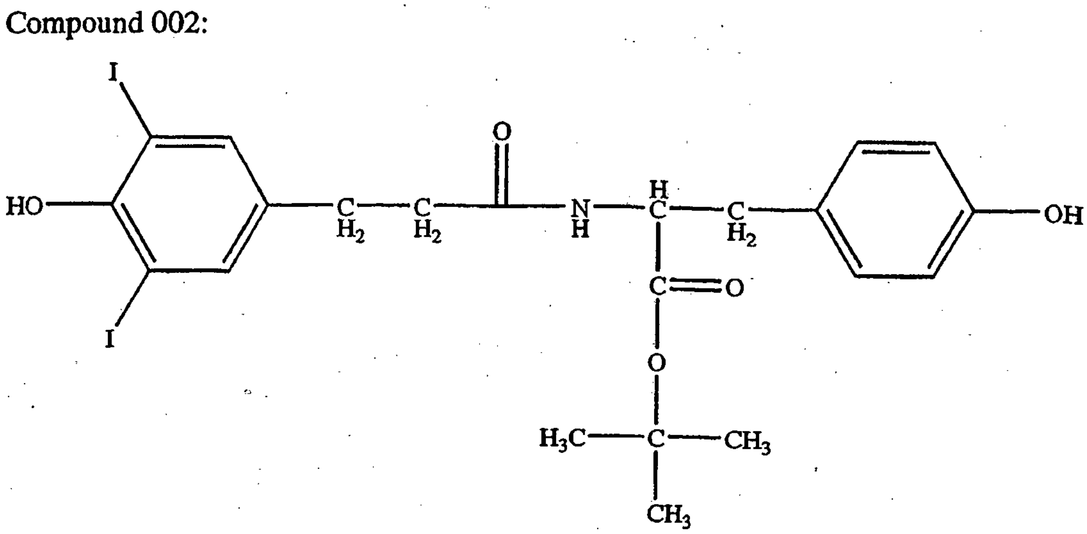

Compound 003

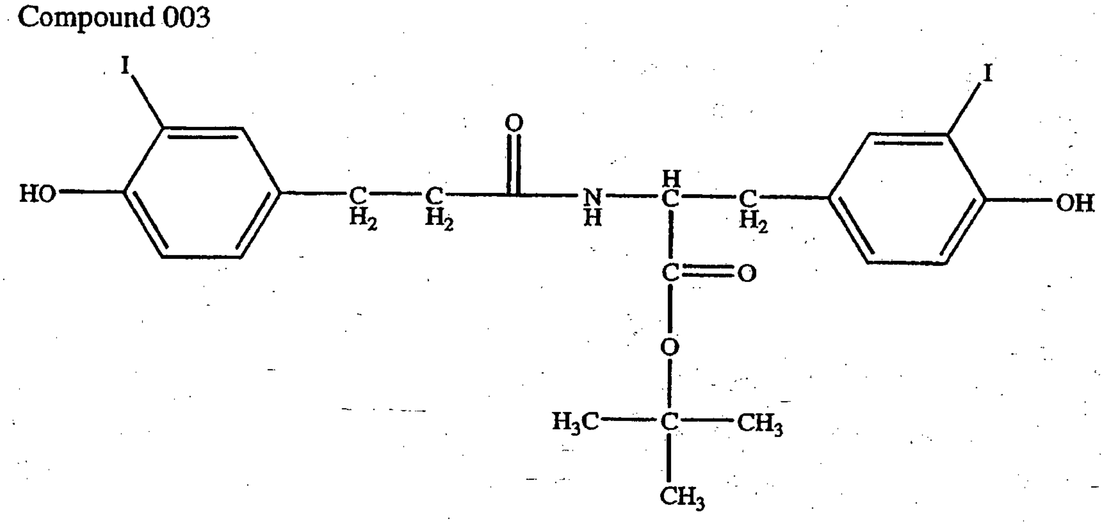

Compound 004:

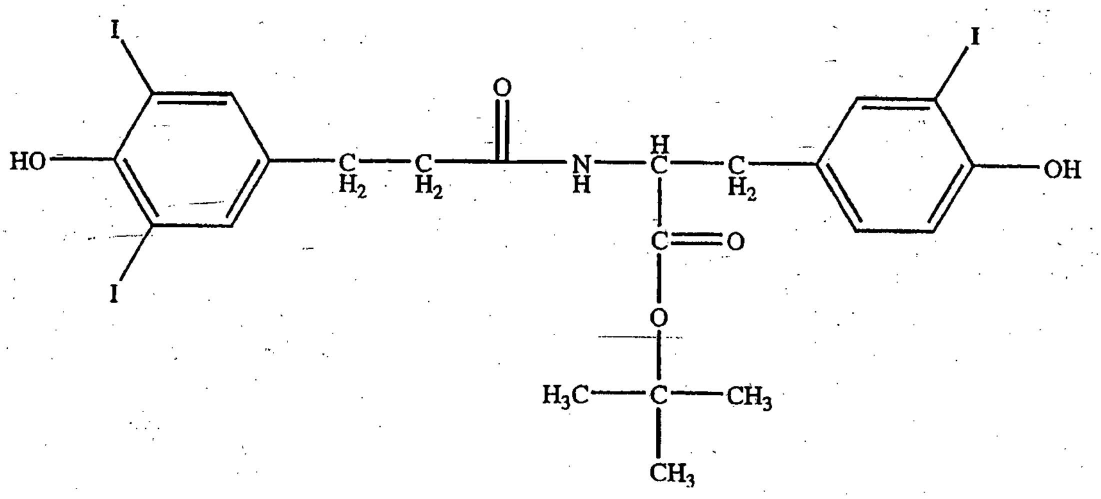

Compound 005

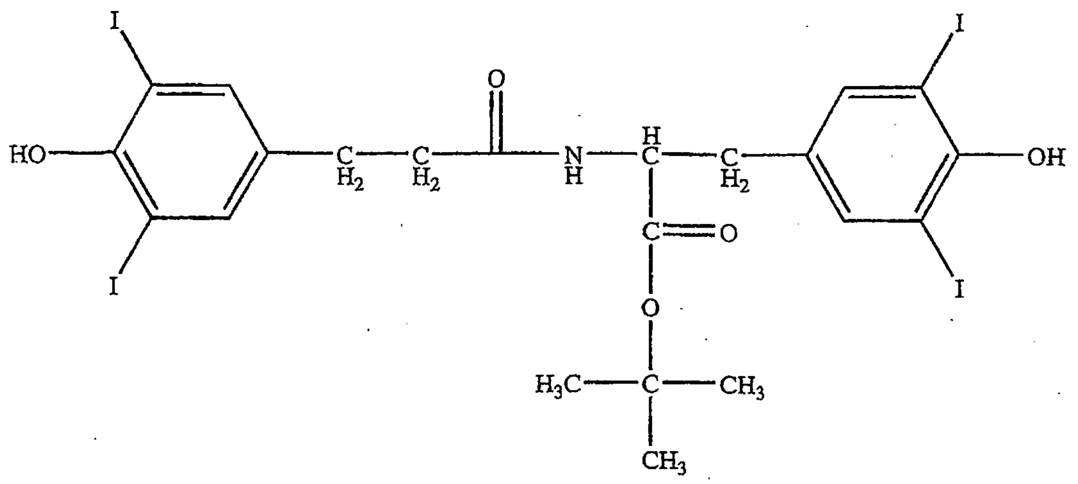

When radiopacity is either not a desired characteristic or when the radiopacity is conveyed by non-polymeric components of the stent (e.g., coated metal stents), the polymers disclosed herein need not be inherently radiopaque. Independent of radiopacity considerations, the polymers need also not contain poly(alkylene glycol) blocks (e.g., PEG).

In certain variations of Formula I, including polymers with t-butyl protected free carboxylic acid groups, $R_1$ of R is either ethyl or butyl, and $R_3$ is either $-CH_2-CH_2-$ or $-CH_2-CH_2-CH_2-CH_2-$. It is further understood that the presentation of Formula I is schematic and that the polymer structures represented are random copolymers with respect to the position of P so that the different subunits can occur in random sequence throughout the polymeric backbone except that A is always connected to either P or a phenolic ring.

For all of the depicted polymers, when A is a carbonyl (C=O), the resulting polymers comprise polycarbonates. When A is:

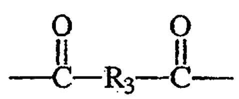

,

the resulting polymers comprise polyarylates. The polymers of Formulae II and II also include polycarbonates and

polyarylates, as well as any other polymer that can be polymerized from monomers with two terminal -OH groups, and particularly, any polymer that can be polymerized from a diphenol. The Formulae II and III polyarylates include polymers of the dicarboxylic acids disclosed for the preparation of the polyarylates of Formula I.

In certain preferred embodiments, it is understood that alkyl groups can be branched (such as isopropyl or tert-butyl), or straight-chain, and can contain heteroatoms such as O, N and S.

It is understood that the presentation of Formula I is schematic and that the polymer structures represented are random copolymers with respect to the position of P, so that the different subunits can occur in random sequence throughout the polymeric backbone except that A is preferably connected to either P or a phenolic ring.

In variations wherein Formula I defines a polyarylate, $R_3$ is preferably a saturated or unsaturated, substituted or unsubstituted alkyl, aryl, or alkylaryl group containing up to about 18 carbon atoms. In certain variations, $R_3$ is an alkyl group containing between about 2 and about 12 carbon atoms, either in a straight or branched chain. The $R_3$ groups may be substituted with any suitable functional group that does not, or tends not, to cross-react with other monomeric compounds during polymerization or otherwise interfere significantly with the formation of the present polymers via polymerization as described below. In certain variations, $R_3$ is selected such that the polyarylate A-moieties in Formula I are derived from dicarboxylic acids that are either naturally occurring metabolites or highly biocompatible compounds. For example, in some variations, $R_3$ is selected such that the polyarylate A-moieties in Formula I are derived from the intermediate dicarboxylic acids of the cellular respiration pathway known as the Krebs Cycle. Such dicarboxylic acids include alpha- ketoglutaric acid, succinic acid, fumaric acid, maleic acid, and oxalacetic acid. Other preferred biocompatible dicarboxylic acids include sebacic acid, adipic acid, oxalic acid, malonic acid, glutaric acid, pimelic acid, suberic acid and azelaic acid. Stated another way, $R_3$ is more preferably a moiety selected from -CH=CH- and $(-CH_2-)_z$, wherein z is an integer from 0 to 8, preferably 2 to 8, inclusive.

Among the preferred aromatic dicarboxylic acids are terephthalic acid, isophthalic acid, and bis(p-carboxyphenoxy) alkanes such as bis(p-carboxyphenoxy) propane.

P in Formula I is a poly(alkylene glycol), and preferably a poly(ethylene glycol) block/unit typically having a molecular weight of less than about 10,000 per unit. More typically, the poly(ethylene glycol) block/unit has a molecular weight less than about 4000 per unit. The molecular weight is preferably between about 1000 and about 2000 per unit.

The same poly(alkylene glycols), and preferred species thereof, my be optionally copolymerized in the polymers of Formulae II and Iii.

The molar fraction of poly(ethylene glycol) units in preferred embodiments of Formula I, (f), may vary between 0 and less than 1, typically between 0 and about 0.5, inclusive. More preferably, f is less than about 0.25 and yet more preferably, less than about 0.1. In a more preferred variations, f may vary from greater than about 0.001 to about 0.08, and most preferably, between about 0.025 and about 0.035. The same molar fractions apply to the polymers of Formulae II and III when copolymerized with a poly(alkylene glycol).

As illustrated in Formula I, and unless otherwise indicated, the molar fractions reported herein are based on the total molar amount of diphenolic carboxylic acid ester monomeric units, diphenolic free carboxylic acid units, and poly(alkylene glycol) units in the polymeric units of Formula I.

Applicants have recognized that the molar fraction of free carboxylic acid units, such as DT units, in the polymers of the present invention can be adjusted according to the present invention to likewise adjust the degradation/resorbability of the device made from such polymers. For example, applicants have recognized that while polymers comprising about 35% free carboxylic acid units (a molar fraction of 0.35) are 90% resorbed in about 15 days, polymers with lower amounts of free carboxylic acid will have desirably longer lifetimes in the body. Furthermore, by otherwise adjusting the amount of free carboxylic acid in the polymers across the range of preferred molar fraction, the resulting polymers can be adapted for use in various applications requiring different device lifetimes. In general, the higher the molar fraction of free carboxylic acid units, the shorter the lifetime of the device in the body and more suitable such devices are for applications wherein shorter lifetimes are required. In certain embodiments where lifetimes of 6 months or more are required, polymers of the presently preferred ranges of free carboxylic acid units tend to be desirable. According to preferred embodiments, the molar fraction, (g), of repeating units in Formula I derived from free carboxylic acids ranges between about 0 and 0.5, more preferably, g is greater than about 0.1 to about 0.35, and yet more preferably, g is from about 0.2 to about 0.3. In more preferred variations, g may vary between about 0.01 and about 0.25, and most preferably, between about 0.05 and about 0.15.

Applicants have also recognized that the polymer glass transition temperature increases as the degree of halogenation and the molar fraction of free carboxylic acid units increases. Higher weight percentages of poly(alkylene oxide) are typically used in polymers with higher levels of iodination and/or with higher molar fractions of free carboxylic acid units to maintain the polymer glass transition temperature within a range considered acceptable by those of ordinary skill in the art of polymeric stent design.

In certain preferred embodiments, the copolymers disclosed herein have weight-average molecular weights (Mw) of from about 20,000 to about 500,000, preferably from about 50,000 to about 300,000, and more preferably from about 75,000 to about 200,000. The polydispersity ($P_d$) values of the copolymers is in the range of about 0.5 to about

10, more preferably about 1.5 to 2.5, and most preferably about 2. The corresponding number-average molecular weights (Mn) of the polymers of the present invention can be calculated using:

$$Mn = Mw/P_d$$

Accordingly, the Mn values of the copolymers of the present invention are from about 10,000 to about 250,000, more preferably from about 25,000 to about 150,000, and even more preferably from about 37,500 to about 100,000. The molecular weights are measured by gel permeation chromatography (GPC) relative to polystyrene standards without further correction.

It is further understood that the polymers according to certain preferred embodiments of the present invention include not only the polymers from which the stents (or other medical devices are fabricated), but also the precursor polymers with t-butyl protected carboxylic acid groups, i.e., polymers according to Formula I wherein $R_1$ of $R_2$ is a t-butyl group.

**Stents and Stent Systems**

An inherently radiopaque, biocompatible, bioresorbable stent is disclosed (not claimed). The stent comprises a tubular member and further comprises any of the above-described polymers, wherein the tubular member comprises a configuration selected from the group consisting of a sheet stent, a braided stent, a self-expanding stent, a wire stent, a deformable stent, and a slide-and-lock stent. In some variations, the polymer is a coating on a metal stent. More preferably, the stent is balloon expandable and comprises at least two substantially non-deforming elements arranged to form a tubular member, the non-deforming elements being slidably or rotationally interconnected for allowing the tubular member to expand from a collapsed diameter to an expanded diameter. In another variation the tubular member comprises a series of slideably engaged radial elements and at least one locking mechanism which permits one-way sliding of the radial elements from a first collapsed diameter to a second expanded diameter.

A collapsed stent mounted on a delivery catheter is referred to herein as a stent system. Catheters include but are not limited to over-the-wire catheters, coaxial rapid-exchange designs and multi-exchange delivery platforms, e.g., the Medtronic Zipper Technology. Such catheters may include for instance those described in Bonzel U.S. Patent Nos. 4,762,129 and 5,232,445 and by Yock U.S. Patent Nos. 4,748,982; 5,496,346; 5,626,600; 5,040,548; 5,061,273; 5,350,395; 5,451,233 and 5,749,888. Additionally, catheters may include for instance those as described in U.S. Patent Nos. 4,762,129; 5,092,877; 5,108,416; 5,197,978; 5,232,445; 5,300,085; 5,445,646; 5,496,275; 5,545,135; 5,545,138; 5,549,556; 5,755,708; 5,769,868; 5,800,393; 5,836,965; 5,989,280; 6,019,785; 6,036,715; 5,242,399; 5,158,548; and 6,007,545.

Catheters may be specialized for various purposes such as to produce an ultrasound effect, electric field, magnetic field, light and/or temperature effect. Heating catheters may include for example those described in U.S. Patent No. 5,151,100, 5,230,349; 6,447,508; and 6,562,021 as well as WO9014046A1. Infrared light emitting catheters may include for example those described in U. S. Patent Nos. 5,910,816 and 5,423,321.

A stent produced in accordance with preferred aspects of the present invention may be of any design (e.g., slide-and-lock stents, sheet stents (sometimes referred to as jelly-roll stents), deformable stents, and self-expanding stents) suitable for a given application. Preferably, the stents of the present invention are designed to be readily implantable in the artery or tissue of an animal, such as a human, and to be expandable and/or suitable for holding open an artery, after said artery is opened via a medical procedure, such as an angioplasty. Examples of suitable stent designs for use in the present invention include "slide-and-lock" stents, including those disclosed in U.S. Patent Nos. 6,033,436; 6,224,626 and 6,623,521, and co-pending US Application No. 10/897,235 filed on July 21, 2004.

Other suitable designs adaptable for use herein include those used traditionally in metal and polymeric stents, including various mesh, jelly-roll, sheet, zigzag, and helical coil designs, e.g., the deformable stents by Palmaz such as U.S. Pat. No. 4,733,665 and its successors which have controllable expansion and a portion of the prosthesis that deforms with a force in excess of the elastic limit. Other stent designs include the following designs and their successors: U.S. Pat. No. 5,344,426 by Lau, U.S. Pat. Nos. 5,549,662 and 5,733,328 by Fordenbacher, U.S. Pat. Nos. 5,735,872 and 5,876,419 by Carpenter, U.S. Pat. No. 5,741,293 by Wijay, U.S. Pat. No. 5,984,963 by Ryan, U.S. Pat. Nos. 5,441,515 and 5,618,299 by Khosravi, U.S. Pat. Nos. 5,059,211; 5,306,286 and 5,527,337 by Stack, U.S. Pat. No. 5,443,500 by Sigwart, U.S. Patent No. 5,449,382 by Dayton, U.S. Patent No. 6,409,752 by Boatman, and the like.

Disclosed herein are expandable medical implants for maintaining support of a body lumen. Over the years, a wide variety of stent types have been proposed. Although the structures of stents may vary substantially, virtually all stents are configured to be expandable from a collapsed condition having a small diameter to an expanded condition having a larger diameter. While in the collapsed condition, the stent is delivered usually via catheter through the blood

vessel, or other body lumen, to the treatment site. After the treatment site is reached, the stent is radially expanded to an implantable size for supporting the vessel wall. Expansion of the stent from the collapsed condition to the expanded condition can be achieved in a variety of different ways. Various types of stents are described below based on their configurations and means for expansion. For additional information, a variety of stents types are described by Balcon et al., "Recommendations on Stent Manufacture, Implantation and Utilization," European Heart Journal (1997), vol. 18, pages 1536-1547, and Phillips, et al., "The Stenter's Notebook," Physician's Press (1998), Birmingham, Michigan.

Balloon expandable stents are manufactured in the collapsed condition and are expanded to a desired diameter with a balloon. During delivery, a balloon expandable stent is typically mounted on the exterior of an inflatable balloon located along the distal end portion of a catheter. After reaching the treatment site, the stent is expanded from the collapsed condition to the expanded condition by inflating the balloon. The stent is typically expanded to a diameter that is greater than or equal to the inner diameter of the body lumen. The expandable stent structure may be held in the expanded condition by mechanical deformation of the stent as taught in, for example, U.S. Patent No. 4,733,665 to Palmaz. Alternatively, balloon expandable stents may be held in the expanded condition by engagement of the stent walls with respect to one another as disclosed in, for example, U.S. Patent Nos. 4,740,207 to Kreamer, 4,877,030 to Beck et al., and 5,007,926 to Derbyshire. Further still, the stent may be held in the expanded condition by one-way engagement of the stent walls together with endothelial growth into the stent, as shown in U.S. Patent No. 5,059,211 to Stack et al.

The term "radial strength," as used herein, describes the external pressure that a stent is able to withstand without incurring clinically significant damage. Due to their high radial strength, balloon expandable stents are commonly used in the coronary arteries to ensure patency of the vessel. During deployment in a body lumen, the inflation of the balloon can be regulated for expanding the stent to a particular desired diameter. Accordingly, balloon expandable stents may be used in applications wherein precise placement and sizing are important. Balloon expandable stents may also be commonly used for direct stenting, wherein there is no pre-dilation of the vessel before stent deployment. Rather, during direct stenting, the expansion of the inflatable balloon dilates the vessel while also expanding the stent.

One of the first self-expanding stents used clinically is the braided "WallStent," as described in U.S. Patent No. 4,954,126 to Wallsten. The WallStent generally comprises a metallic mesh in the form of a Chinese finger cuff. The cuff provides a braided stent that is not superelastic, but technically still falls in the self-expanding stent family. Another example of a self-expanding stent is disclosed in U.S. Patent No. 5,192,307 to Wall wherein a stent-like prosthesis is formed of polymeric or sheet metal that is expandable or contractible for placement. The stent may be biased in an open position and lockable in a closed position or, alternatively, may be biased towards a closed position and lockable in an open position. In the former case, a pin may be used to hold the stent in the collapsed condition. The pin is removed to allow the stent to assume the expanded condition. One or more hooks may be formed into the wall for locking the stent. The hooks engage complementary recesses formed in an opposing wall to mechanically interlock the rolled up sheet forming the stent.

Heat expandable stents are similar in nature to self-expanding stents. However, this type of stent utilizes the application of heat to produce expansion of the stent structure. Stents of this type may be formed of a shape memory alloy, such as Nitinol. Still other types of heat expandable stents may be formed with a tin-coated, heat expandable coil. Heat expandable stents may be delivered to the affected area on a catheter capable of receiving a heated fluid. Heated saline or other fluid may be passed through the portion of the catheter on which the stent is located, thereby transferring heat to the stent and causing the stent to expand.

It is desirable that a stent be balloon expandable for providing accurate placement and sizing at a treatment site. It is also desirable that such a stent has sufficient radial strength to maintain patency of the lumen while subjected to substantial external forces. It is also desirable that such a stent be configured to exhibit little or no longitudinal shortening during radial expansion. It is also desirable that such a stent be sufficiently flexible along the longitudinal axis to conform to the curved shape of a body lumen. It is also desirable that such a stent has the capability to conform to the interior of the body lumen.

While various stent configurations; including without limitation, sheet stents, braided stents, self-expanding stents, wire stents, deformable stents, and a slide-and-lock stents, are known in the art, it will be appreciated that the description is illustrative only and should not be construed in any way as limiting the invention. Indeed, the radiopaque, bioresorbable polymers described herein may be applicable to a variety of other stent designs that are known in the art. Furthermore, various applications of the invention, and modifications thereto, which may occur to those who are skilled in the art, are also encompassed by the general concepts described herein.

Some preferred variations relate to an expandable slide-and-lock stent having a plurality of modules. The modules have a plurality of sliding and locking elements permitting one-way sliding of the radial elements from a collapsed diameter to an expanded/deployed diameter, but inhibiting radial recoil from the expanded diameter. One advantage is that the stent design elements of the modules and interlocks can be varied to customize the functional features of strength, compliance, radius of curvature at deployment and expansion ratio. In some preferred embodiments, the stent comprises the polymer described in Formula I, such that the stent comprises a radiopaque, bioresorbable material,

which is adapted to vanish over time. In some embodiments, the stent serves as a therapeutic delivery platform.

Some variations relate to a radially expandable stent used to open, or to expand a targeted area in a body lumen. In some embodiments, the assembled stent comprises a tubular member having a length in the longitudinal axis and a diameter in the radial axis, of appropriate size to be inserted into the body lumen. The length and diameter of the tubular member may vary considerably for deployment in different selected target lumens depending on the number and configuration of the structural components, described below. The tubular member is adjustable from at least a first collapsed diameter to at least a second expanded diameter. One or more stops and engaging elements or tabs are incorporated into the structural components of the tubular member whereby recoil (i.e., collapse from an expanded diameter to a more collapsed diameter) is minimized to less than about 5%.

The tubular member in accordance with some variations has a "clear through-lumen," which is defined as having no structural elements protruding into the lumen in either the collapsed or expanded diameters. Further, the tubular member has smooth marginal edges to minimize the trauma of edge effects. The tubular member is preferably thin-walled (wall thickness depending on the selected materials ranging from less than about 635 to less than about 100 micrometers) and flexible (e.g., less than about 0.01 Newtons force/millimeter deflection) to facilitate delivery to small vessels and through tortuous vasculature. The thin walled design will also minimize blood turbulence and thus risk of thrombosis. The thin profile of the deployed tubular member in accordance with some embodiments also facilitates more rapid endothelialization of the stent.

The wall of the tubular member comprises at least one module, which comprises a series of sliding and locking radial elements. Preferably, a plurality of modules are connected in the longitudinal axis via linkage elements which couple at least some of the radial elements between adjacent modules. The radial elements are configured within each module so as to define the circumference of the tubular member. Each radial element within a module is preferably a discrete, unitary structure, comprising one or more circumferential ribs bowed in the radial axis to form a fraction of the total circumference of the tubular member. At least one of the ribs in each radial element has one or more stops disposed along the length of the rib. At least some of the radial elements also have at least one articulating mechanism for slideably engaging the rib(s) from adjacent, circumferentially offset radial elements. In one aspect of the invention, the articulating mechanism includes a tab for engaging the stops disposed along the slideably engaged adjacent rib. The articulating between the tab from one radial element and the stops from an adjacent radial element is such that a locking or ratcheting mechanism is formed, whereby the adjacent radial elements may slide circumferentially apart from one another, but are substantially prevented from sliding circumferentially toward one another. Accordingly, the tubular member may be radially expanded from a smaller diameter to a larger diameter, but recoil to a smaller diameter is minimized by the locking mechanism. The amount of recoil can be customized for the application by adjusting the size and spacing between the stops along the ribs. Preferably the recoil is less than about 5%.

Some aspects of embodiments of stents are disclosed in U.S. Patent Nos. 6,033,436, 6,224,626 and 6,623,521 all of which are issued to Steinke et al.;

Although a stent formed from a single integral element is described above as having particular mechanical characteristics for locking the stent in the expanded condition, a variety of other "slide and lock" mechanisms may be used. For example, other suitable locking mechanism may be found in U.S. Patent No. 5,344,426 to Lau, U.S. Patent Nos. 5,735,872 and 5,876,419 to Carpenter, U.S. Pat. No. 5,741,293 to Wijay, U.S. Patent No. 5,984,963 to Ryan, U.S. Patent Nos. 5,441,515 and 5,618,299 by Khosravi, U.S. Patent No. 5,306,286 to Stack, U.S. Patent No. 5,443,500 to Sigwart, U.S. Patent No. 5,449,382 to Dayton, U.S. Patent No. 6,409,752 to Boatman, and the like. In addition, many of the slide and lock mechanisms disclosed in the above patents may be suitable for use with stents embodiments comprising slidable interconnected elements of the type described above.

**Therapeutic Agents and Stent Coatings**

A stent may further an amount of a therapeutic agent (for example, a pharmaceutical agent and/or a biologic agent) sufficient to exert a selected therapeutic effect. The term "pharmaceutical agent", as used herein, encompasses a substance intended for mitigation, treatment, or prevention of disease that stimulates a specific physiologic (metabolic) response. The term "biological agent", as used herein, encompasses any substance that possesses structural and/or functional activity in a biological system, including without limitation, organ, tissue or cell based derivatives, cells, viruses, vectors, nucleic acids (animal, plant, microbial, and viral) that are natural and recombinant and synthetic in origin and of any sequence and size, antibodies, polynucleotides, oligonucleotides, cDNA’s, oncogenes, proteins, peptides, amino acids, lipoproteins, glycoproteins, lipids, carbohydrates, polysaccharides, lipids, liposomes, or other cellular components or organelles for instance receptors and ligands. Further the term "biological agent", as used herein, includes virus, serum, toxin, antitoxin, vaccine, blood, blood component or derivative, allergenic product, or analogous product, or arsphenamine or its derivatives (or any trivalent organic arsenic compound) applicable to the prevention, treatment, or cure of diseases or injuries of man (per Section 351(a) of the Public Health Service Act (42 U.S.C. 262(a)). Further the term "biological agent" may include 1) "biomolecule", as used herein, encompassing a biologically active peptide, protein,

carbohydrate, vitamin, lipid, or nucleic acid produced by and purified from naturally occurring or recombinant organisms, antibodies, tissues or cell lines or synthetic analogs of such molecules; 2) "genetic material" as used herein, encompassing nucleic acid (either deoxyribonucleic acid (DNA) or ribonucleic acid (RNA), genetic element, gene, factor, allele, operon, structural gene, regulator gene, operator gene, gene complement, genome, genetic code, codon, anticodon, messenger RNA (mRNA), transfer RNA (tRNA), ribosomal extrachromosomal genetic element, plasmagene, plasmid, transposon, gene mutation, gene sequence, exon, intron, and, 3) "processed biologics", as used herein, such as cells, tissues or organs that have undergone manipulation. The therapeutic agent may also include vitamin or mineral substances or other natural elements.

The amount of the therapeutic agent is preferably sufficient to inhibit restenosis or thrombosis or to affect some other state of the stented tissue, for instance, heal a vulnerable plaque, and/or prevent rupture or stimulate endothelialization. The agent(s) may be selected from the group consisting of antiproliferative agents, anti-inflammatory, anti-matrix metalloproteinase, and lipid lowering, cholesterol modifying, anti-thrombotic and antiplatelet agents, in accordance with preferred embodiments of the present invention. In some preferred embodiments of the stent, the therapeutic agent is contained within the stent as the agent is blended with the polymer or admixed by other means known to those skilled in the art. In other preferred embodiments of the stent, the therapeutic agent is delivered from a polymer coating on the stent surface. In another preferred variation the therapeutic agent is delivered by means of a non-polymer coating. In other preferred embodiments of the stent, the therapeutic agent is delivered from at least one region or one surface of the stent. The therapeutic can be chemically bonded to the polymer or carrier used for delivery of the therapeutic from at least one portion of the stent and/or the therapeutic can be chemically bonded to the polymer that comprises at least one portion of the stent body. In one preferred embodiment, more than one therapeutic agent may be delivered.

In addition to a stent that may deliver a therapeutic agent, for instance delivery of a biological polymer on the stent such as a repellant phosphorylcholine, the stent may be coated with other bioresorbable polymers predetermined to promote biological responses in the vessel lumen desired for certain clinical effectiveness. Further the coating may be used to mask the surface properties of the polymer used to comprise the stent embodiment. The coating may be selected from the broad class of any biocompatible bioresorbable polymer which may include any one or combination of halogenated and/or non-halogenated which may or may not comprise any poly(alkylene glycol). These polymers may include compositional variations including homopolymers and heteropolymers, stereoisomers and/or a blend of such polymers. These polymers may include for example, but are not limited to, polycarbonates, polyarylates, poly(ester amides), poly(amide carbonates), trimethylene carbonates, polycaprolactones, polydioxanes, polyhydroxybutyrates, polyhydroxyvalerates, polyglycolides, polylactides and stereoisomers and copolymers thereof, such as glycolide/lactide copolymers. In a preferred embodiment, the stent is coated with a polymer that exhibits a negative charge that repels the negatively charged red blood cells' outer membranes thereby reducing the risk of clot formation. In another preferred embodiment, the stent is coated with a polymer that exhibits an affinity for cells, (e.g., endothelial cells) to promote healing. In yet another preferred embodiment, the stent is coated with a polymer that repels the attachment and/or proliferation of specific cells, for instance arterial fibroblasts and/or smooth muscle cells in order to lessen restenosis and/or inflammatory cells such as macrophages.

Therapeutic agents can be incorporated into the bioresorbable stent and/or coated on at least one region of the stent surface, thereby providing local release of such agents. In preferred embodiments, the therapeutic agent is contained within the stent as the agent is blended with the polymer or admixed by other means known to those skilled in the art. In other preferred embodiments of the stent, the therapeutic agent is delivered from a polymer coating on the stent surface. In another preferred variation the therapeutic agent is delivered by means of no polymer coating. In other preferred embodiments of the stent, the therapeutic agent is delivered from at least one region or one surface of the stent.

The preferred therapeutic agent(s) control restenosis (including neointimal thickening, intimal hyperplasia and in-stent restenosis or limits vascular smooth muscle cell overgrowth) in the lumen of a stented vessel. Vascular stent applications and other body applications may require a different therapeutic or more than one therapeutic.

A variety of compounds are considered to be useful in controlling vascular restenosis and in-stent restenosis. Some of these preferred agents that improve vascular patency include without limitation paclitaxel, Rapamycin, ABT-578, everolimus, dexamethasone, nitric oxide modulating molecules for endothelial function, tacrolimus, estradiol, mycophenolic acid, C6-ceramide, actinomycin-D and epothilones, and derivatives and analogs of each.

The preferred therapeutic agent can also limit or inhibit thrombosis or affect some other state of the stented tissue, for instance, heal a vulnerable plaque, inhibit plaque rupture, stimulate endothelialization or limit other cell types from proliferating and from producing and depositing extracellular matrix molecules. The agent(s) may be selected from the group consisting of but not limited to: antiproliferative agents, anti-inflammatory, anti-matrix metalloproteinase, and lipid lowering, anti-thrombotic and antiplatelet agents, in accordance with preferred embodiments of the present invention.

In a variation the device delivers a therapeutic agent(s) to treat the vulnerable plaque lesion such as an anti-inflammatory, a lipid lowering/matrix altering therapeutic and/or an antiproliferative. The anti-inflammatory may include aspirin, an effective neutralizer of inflammation, losartan, an angiotensin receptor blocker or pravastatin, a 3-Hydroxy-3-Methyl-Glutaryl Coenzyme A (HMG-CoA) reductase inhibitor. Further delivery of statins, such as pravastatin and

fluvastatin, which are 3- HMG-CoA reductase inhibitors may interstitial collagen gene expression and lower matrix metalloproteinases (MMP-1, MMP-3, and MMP-9) expression to effectively stabilize the vulnerable plaque lesions. Local stent delivery of lipid-lowering agent, for example Pravastatin, may also improve plaque stability.

A stent may deliver an antiplatelet agent that acts by glycoprotein IIb/IIIa receptor inhibition or other means such as but not limited to aspirin, Plavix (clopidogrel bisulfate), ticlopidine, integrelin, and dipyridamole. In another variation the device delivers an antithrombin agent that acts by thrombin inhibition or other means such as heparin, low molecular weight heparin (LMWH), polyamine to which dextran sulfate and heparin are covalently bonded, heparin-containing polymer coating for indwelling implants (MEDI-COAT by STS Biopolymers), polyurethane urea/heparin, R-Hirudin, Hirulog, hirudin/prostacyclin and analogues, argatroban, efegatran, and tick anticoagulant peptide. Additional anti-thrombogenic substances and formulations may include but are not limited to endothelium-derived relaxing factor, prostaglandin I.sub.2, plasminogen activator inhibitor, tissue-type plasminogen activator (tPA), ReoPro: antiplatelet glycoprotein IIb/IIIa integrin receptor, fibrin and fibrin peptide A, lipid-lowering drugs, e.g., Omega-3 fatty acids, and Chrysalin (aka TRAP-508) by Chrysalis Vascular Technologies.

Various compounds address other pathologic events and/or vascular diseases. Some of these therapeutic target compounds are agents to treat endothelial injury (e.g., VEGF; FGF), agents to modulate cell activation and phenotype (e.g., MEF-2 & Gax modulators; NFKB antagonists; cell cycle inhibitors), agents for dysregulated cell growth (e.g., E2F decoys; RB mutants; cell cycle inhibitors), agents for dysregulated apoptosis (e.g., Bax or CPP32 inducers; Bcl-2 inhibitors; integrin antagonists) and agents for abnormal cell migration (e.g., integrin antagonists; PDGF blockers; plasminogen activator inhibitors).

The therapeutic agents to be coated or incorporated within the stent polymer may be classified in terms of their sites of action in the host. The following agents are believed to exert their actions extracellularly or at specific membrane receptor sites. These include corticoids and other ion channel blockers, growth factors, antibodies, receptor blockers, fusion toxins, extracellular matrix proteins, peptides, or other biomolecules (e.g., hormones, lipids, matrix metalloproteinases, and the like), radiation, anti-inflammatory agents including cytokines such as interleukin-1 (IL-1), and tumor necrosis factor alpha (TNF-$\alpha$), gamma interferon (interferon-$\gamma$), and Tranilast, which modulate the inflammatory response.

Other groups of agents exert their effects at the plasma membrane. These include those involved in the signal transduction cascade, such as coupling proteins, membrane associated and cytoplasmic protein kinases and effectors, tyrosine kinases, growth factor receptors, and adhesion molecules (selectins and integrins).

Some compounds are active within the cytoplasm, including for example, heparin, ribozymes, cytoxins, antisense oligonucleotides, and expression vectors. Other therapeutic approaches are directed at the nucleus. These include gene integration, proto-oncogenes, particularly those important for cell division, nuclear proteins, cell cycle genes, and transcription factors.

Other therapeutic substances that may be useful as stent coatings and/or depot formulations incorporated within bioresorbable stents include: antibodies e.g., ICAM-1 antibodies for inhibition of monocyte chemotactic recruitment and adhesion, macrophage adhesion and associated events (Yasukawa et al, 1996, Circulation); toxin based therapies such as chimeric toxins or single toxins to control vascular SMC proliferation (Epstein et al., 1991, Circulation); bFGF-saporin to selectively stop SMC proliferation among those cells with a large number of FGF-2 receptors (Chen et al, 1995, Circulation), suramin inhibits migration and proliferation by blocking PDGF-induced and/or mitogen activated protein kinase (MAPK-AP-1)-induced signaling (Hu et al, Circulation, 1999); Beraprost Sodium, a chemically stable prostacyclin analogue ($PGI_2$), suppresses intimal thickening and luminal narrowing of coronary arteries. (Kurisu et al., Hiroshima J. Med Sci, 1997); Verapamil inhibits neointimal smooth muscle cell proliferation (Brauner et al., J Thorac Cardiovasc Surg 1997), agents that block the CD 154 or CD40 receptor may limit the progression of atherosclerosis (E Lutgens et al., Nature Medicine 1999), agents that control responses of shear stress response elements or mechanical stress or strain elements or heat shock genes; and anti-chemoattractants for SMC and inflammatory cells.

In addition or in the alternative, cells could be encapsulated in a bioresorbable microsphere, or mixed directly with polymer, or hydrogel. Living cells could be used to continuously deliver molecules, for instance, cytokines and growth factors. Cells of any origin may be used in accordance with this aspect of the present invention. Further, nonliving cells may be used and preserved or dehydrated cells which retain their purpose when rehydrated may be used. Native, chemically modified (processed), and/or genetically engineered cells may be used.

Therapeutic agents may be polar or possess a net negative or positive or neutral charge; they may be hydrophobic, hydrophilic or zwitterionic or have a great affinity for water. Release may occur by controlled release mechanisms, diffusion, interaction with another agent(s) delivered by intravenous injection, aerosolization, or orally. Release may also occur by application of a magnetic field, an electrical field, or use of ultrasound.

A stent may also incorporate or deliver a hydrogel or other material such as phosphorylcholine (PC) that acts to prevent adhesions of blood cells, blood proteins or blood molecules, extracellular matrix or other cell types. The hydrogel may deliver a therapeutic agent.

Use of synthetic, natural (plant, microbial, viral or animal-derived) and recombinant agents having selected functions or chemical properties can be mixed with complementary substances (e.g., anti-thrombotic and anti-restenosis

substances; nucleic acids and lipid complexes). Pharmacologic agents may also incorporate use of vitamins or minerals. For instance, those that function directly or indirectly through interactions or mechanisms involving amino acids, nucleic acids (DNA, RNA), proteins or peptides (e.g., ROD peptides), carbohydrate moieties, polysaccharides, liposomes, or other cellular components or organelles for instance receptors and ligands.

Genetic approaches to control restenosis include without limitation: use of antisense oligonucleotides to PDGFR-ββ mRNA to control PDGF expression; use of antisense oligonucleotides for nuclear antigens c-myb or c-myc oncogenes (Bauters et al., 1997, Trends CV Med); use of antisense phosphorothioate oligodeoxynucleotides against cdk 2 kinase (cyclin dependent kinase) to control the cell cycle of vascular smooth muscle cells (Morishita et al, 1993, Hypertension); use of VEGF gene (or VEGF itself) to stimulate reconstructive wound healing such as endothelialization and decrease neointima growth (Asahara et al 1995); delivery of the nitric oxide synthetase gene (eNOS) to reduce vascular smooth muscle cell proliferation (Von Der Leyen et al., 1995, Proc Natl Acad Sci); use of adenovirus expressing plasminogen activator inhibitor-1 (PAI-1) to reduce vascular SMOOTH MUSCLE CELL migration and thereby diminish restenosis (Carmeliet et al., 1997, Circulation); stimulation of apolipoprotein A-1 (ApoAl) over-expression to rebalance serum levels of LDL and HDL; use of apoptosis gene products to promote cell death (e.g., of smooth muscle cells) and cytotactic gene products to that regulate cell division (tumor suppressor protein p53 and Gax homeobox gene product to suppress ras; p21 over expression); and inhibition of NF-κB activation (e.g., p65) to control smooth muscle cell proliferation (Autieri et al., 1994, Biochem Biophys Res Commun).

**Methods of Manufacture**

A method is disclosed for manufacture of an inherently radiopaque, biocompatible, bioresorbable stent. One aspect of the method involves the selective removal of tert-butyl ester groups from a hydrolytically unstable polymer to form a new polymer composition having free carboxylic acid groups in place of said tert-butyl ester groups. The disclosed methods comprise dissolving a hydrolytically unstable polymer having at least one t-butyl ester group in a solvent comprising an amount of an acid having a pKa from about 0 to about 4 that is effective to selectively remove by acidolysis at least one t-butyl group to form a free carboxylic acid group.

In one preferred embodiment of the method, the polymer is soluble in the acid and the solvent consists essentially of the acid.

In another variation, the solvent is selected from the group consisting of chloroform, methylene chloride, tetrahydrofuran, dimethylformamide, and mixtures of two or more thereof. In a further variation to the method, the acid is selected from the group consisting of formic acid, trifluoroacetic acid, chloroacetic acid, and mixtures of two or more thereof. In one preferred embodiment of the method, the acid is formic acid.

The polymer compositions of Formula I may be prepared via any of a variety of methods. As noted above, the polymers described by Formula I are halogen ring-substituted diphenolic polycarbonates or polyarylates comprising diphenolic acid ester units, diphenolic free carboxylic acid units, and poly(alkylene glycol) units in the defined relative amounts. Accordingly, the halogen may be iodine and the poly(alkylene glycol) units are poly(ethylene glycol) ("PEG"), the polymers may be prepared by methods comprising polymerizing a desired ratio of one or more iodine ring-substituted diphenol monomer compounds (including monomer compounds for which the subgroup $R_1$ from Formula I is a tert-butyl ester group) and PEG, followed by a deprotection reaction to remove the tert-butyl ester protecting groups to form a polymer composition of Formula I.

Examples of methods adaptable for use to prepare polycarbonate or polyarylate polymers are disclosed in U.S. Patent Nos. 5,099,060, 5,587,507, 5,658,995, 5,670,602, 6,120,491, and 6,475,477 Other suitable processes, associated catalysts and solvents are known in the art and are taught in Schnell, Chemistry and Physics of Polycarbonates, (Interscience, New York 1964).

Polycarbonates may also be prepared by dissolving the diphenol monomers and poly(ethylene glycol) in methylene chloride containing 0.1M pyridine or triethylamine. A solution of phosgene in toluene at a concentration between about 10 and about 25 wt%, and preferably about 20 wt%, is added at a constant rate, typically over about two hours, using a syringe pump or other means. The reaction mixture is quenched by stirring with tetrahydrofuran (THF) and water, after which the polymer is isolated by precipitation with isopropanol (IPA). Residual pyridine (if used) is then removed by agitation of a THF polymer solution with a strongly acidic resin, such as AMBERLYST 15.

The foregoing process improves upon prior art methods using gaseous phosgene, which requires special handling, and also requires the careful co-addition of sodium hydroxide at a controlled rate to maintain the reaction mixture at a pH between 6 and 8 to prevent polymer backbone degradation. The prior art methods also required a significant excess of phosgene, because under the conditions employed considerable quantities were hydrolyzed. The present process also advantageously provides for a more complete removal of residual pyridine or replacement of pyridine entirely with triethylamine, which has a more favorable toxicity profile.

Methods for preparing diphenol monomers for use in making polymers are disclosed, for example, in U.S. Patent Nos. 5,587,507, and 5,670,602. In particular, such references disclose the preparation of non-ester desaminotyrosyl-tyrosine free carboxylic acid (DT), as well as, desaminotyrosyl-tyrosine esters, including the ethyl (DTE), butyl (DTB), hexyl (DTH), octyl (DTO), benzyl (DTBn), and other esters. Iodine-substituted diphenol monomers may be prepared, for example, by coupling together, via any of the procedures disclosed herein, two phenol compounds in which either or both of the phenol rings are iodine substituted, or forming a diphenol, which is iodinated after coupling via any suitable iodination method.

While any of the aforementioned processes are adaptable for use herein, as noted above, it may be difficult to prepare the preferred polycarbonates and polyarylates having pendant free carboxylic acid groups from monomers having free carboxylic acid groups (such as DT monomers) without cross-reaction of the monomer free carboxylic acid groups with co-monomers. Applicants found, however, that free acid polymers, including the preferred polymers of the present invention, can be produced from protected polymers as detailed below in the absence of palladium catalysts, thus avoiding any disadvantages associated with conventional methods. Applicants discovered, contrary to conventional teachings in the art, that t-butyl (tB) groups can be used to great advantage as free acid protecting groups that are readily and selectively removed from polymers without the need for palladium, or other difficult to remove catalysts.

As used herein, the term "selectively removed" refers to a deprotecting reaction wherein over 99% of all t-butyl protecting groups are removed with less than a 10% reduction of the polymer molecular weight. Applicants have discovered that the present methods are capable of removing t-butyl protecting groups from provided polymers with a selectivity of about 99.99% or greater, and preferably about 99.995% or greater.

More particularly, the preferred polymers can be prepared advantageously by polymerizing iodine-ring substituted alkyl ester monomers with poly(ethylene glycols) and temporarily protected free carboxylic acid monomers (monomers wherein the free acid functionality is masked using a temporary protecting group) to form a polycarbonate or polyarylate polymeric unit from which the temporary protecting groups are selectively removable to produce the corresponding free carboxylic acid groups.

Any of a wide variety of suitable protection/deprotection methods can be adapted for use in the preparation of the polymeric devices of the present invention, including the methods for converting benzyl carboxylate esters to free carboxylic acid moieties as described, for example, in U.S. Patent 6,120,491.

Another method that can be used is the novel deprotection method of the present invention in which t-butyl protecting groups on hydrolytically unstable polymers are selectively removed to provide new polymers with free carboxylic acid groups in place of the t-butyl groups. The method contacts a hydrolytically unstable polymer having at least one t-butyl ester group with an amount of an acid having a pKa from about 0 to about 4 effective to selectively remove by acidolysis at least one t-butyl group to form a free carboxylic acid group.

Preferred polymeric starting materials have at least one repeating unit comprising a t-butyl protected free acid. One example of t-butyl protected polymers suitable for use in the deprotection method of the present invention comprises one or more units described by Formula II. Preferred diphenolic polymers comprise one or more diphenol units described by Formula III, and in particular, the t-butyl protected polymers of Formula I.

In general, the polymer having one or more repeating t-butyl (tB) ester groups to be deprotected according to the present method can be provided via any of a wide variety of methods. When radiopacity is desired, the monomer is appropriately ring-iodinated or brominated. Formula II includes polycarbonates and polyarylates that may be prepared as described herein, including copolymers thereof with poly(alkylene oxides). In certain preferred embodiments, a polyarylate or polycarbonate comprising t-butyl (tB) ester repeating units is prepared and provided by reacting t-butyl ester protected carboxylate monomers with other monomers.

Formula II also includes poly(amide carbonates) and poly(ester amides) prepared according to the method described by U.S. Patent No. 6,284,462, using species of monomers disclosed therein having pendant t-butyl ester groups. Formula II also includes polyiminocarbonates that may be prepared, for example, by reacting t-butyl ester protected carboxylate monomers with other monomers according to a method disclosed by U.S. Patent No. 4,863,735.

Formula II also includes the phosphorus-containing polymers disclosed by U.S. Patent Nos. 6,238,687 and 5,912,225, that may be prepared, for example, by reacting monomers disclosed therein having pendant t-butyl ester groups with other monomers according to the polymerization method disclosed therein. Aliphatic-aromatic monomers with pendant t-butyl ester groups disclosed by the above-referenced U.S. Patent No. 6,284,462 may be substituted for the tyrosine derived diphenols to prepare other polymers according to Formula II.

Formula II also includes strictly alternating poly(alkylene oxide ether) copolymers that may be prepared, for example, by reacting t-butyl ester protected carboxylate monomers with other monomers according to a method disclosed by U.S. Patent No. 6,602,497.

Thus, polymers with units described by Formula II can be polymerized from monomers described by Formula IIa, for which $R_4$, X and Y have already been herein defined:

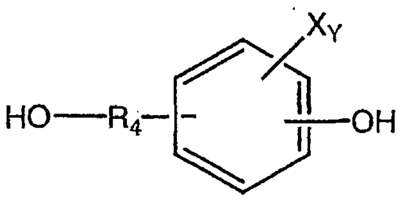

(IIa)

Monomer preparation is described in the patents referenced herein. See in particular:

U.S. Patent Nos. 6,284,462, 4,863,735, 6,238,687 5,912,225, and 6,602,497.

Polymers with diphenol units described by Formulae I and III can be polymerized from diphenol monomers described by Formula IIIa, for which $R_2$, X, Y1 and Y2 have already been herein defined:

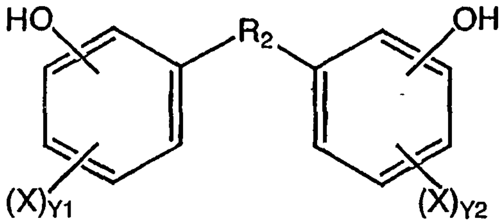

(IIIa)

The polymer may be contacted with the acid by dissolving the polymer in a suitable solvent containing an effective amount of the acid. Any suitable inert solvent in which the polymer to be deprotected is soluble may be used in the reaction mixture of the providing step of the present method. Examples of suitable solvents include chloroform, methylene chloride, THF, dimethylformamide, and the like. In certain preferred embodiments, the solvent comprises methylene chloride.

Any suitable weak acid capable of facilitating the selective removal of a t-butyl protecting group from the carboxylic acid group of a provided polymer by acidolysis can be used according to the present method. Examples of certain suitable weak acids include acids having a pKa of from about 0 to about 4, including formic acid, trifluoroacetic acid, chloroacetic acid, and the like. In certain preferred embodiments the weak acid is formic acid.

Using this method complete deprotection can be achieved with minimal molecular weight loss (<1%). Accordingly, the amount of weak acid used should be the maximum quantity that can be added to the solvent without interfering with polymer solubility. Depending on the specific polymer formulation, complete deprotection will occur within one to four days. The polymer is then recovered by precipitation in either isopropanol or water. Re-dissolving in the solvent (without acid) and re-precipitating will remove any residual acid.

The weak acid can serve as the solvent for polymers soluble therein. Under such circumstances, deprotection occurs more rapidly, within four to eight hours, and eliminates the need for a separate process solvent. In this embodiment the preferred acid is formic acid. The same precipitation and re-precipitation steps may be employed to recover and purify the polymer.

The contacting step, or portions thereof, may be conducted under any suitable conditions effective to selectively remove t-butyl protecting groups via acidolysis. Those of skill in the art will be readily able to adapt any of the wide range of acidolysis methods for use in the contacting step of the present invention to selectively remove t-butyl groups without undue experimentation. For example, in certain preferred embodiments, the contacting step is conducted at about 25° C and about 1 atm.

In light of the disclosure herein, those of skill in the art will be readily able to produce a variety of hydrolytically unstable polymers with free carboxylic acid groups, and especially polymers of the instant invention, for use in a variety of medical devices, from corresponding polymers comprising t-butyl protected free carboxylic acid repeating units.

After polymerization and deprotection, appropriate work up of the polymers in accordance with preferred embodiments of the present invention may be achieved by any of a variety of known methods to produce a variety of stents or other medical devices, suitable for various applications. For example, in certain preferred embodiments, the present polymers are shaped into stents via methods comprising extrusion, compression molding, injection molding, solvent casting, spin casting, combinations of two or more thereof, and the like. Further, stents may be comprised of at least one fiber material, curable material, laminated material, and/or woven material.

Such processes may further include two-dimensional methods of fabrication such as cutting extruded sheets of polymer, via laser cutting, etching, mechanical cutting, or other methods, and assembling the resulting cut portions

into stents, or similar methods of three-dimensional fabrication of devices from solid forms. In certain other embodiments, the polymers are formed into coatings on the surface of an implantable device, particularly a stent, made either of a polymer of the present invention or another material, such as metal. Such coatings may be formed on stents via techniques such as dipping, spray coating, combinations thereof, and the like.

### Other Applications

The highly beneficial combination of properties associated with polymers produced in accordance with embodiments of the present invention are well-suited for use in producing a variety of medical devices besides stents, especially implantable medical devices that are preferably radiopaque, biocompatible, and have various times of bioresorption. For example, applicants have recognized that, in certain embodiments, the polymers are suitable for use in producing implantable devices for orthopedics, tissue engineering, dental applications, wound closure, gastric lap bands, drug delivery, cancer treatment, other cardiovascular applications, non-cardiovascular stents such as biliary, esophagus, vaginal, lung-trachea/bronchus, and the like. In addition, the polymers are suitable for use in producing implantable, radiopaque discs, plugs, and other devices used to track regions of tissue removal, for example, in the removal of cancerous tissue and organ removal, as well as, staples and clips suitable for use in wound closure, attaching tissue to bone and/or cartilage, stopping bleeding (homeostasis), tubal ligation, surgical adhesion prevention, and the like. Applicants have also recognized that the polymers of the present invention are well-suited for use in producing a variety of coatings for medical devices besides stents, especially implantable medical devices.

Furthermore, the polymers may be advantageously used in making various orthopedic devices including, for example, radiopaque biodegradable screws (interference screws), radiopaque biodegradable suture anchors, and the like for use in applications including the correction, prevention, reconstruction, and repair of the anterior cruciate ligament (ACL), the rotator cuff/rotator cup, and other skeletal deformities.

Other devices, which can be advantageously formed from the polymers of the present invention, include devices for use in tissue engineering. Examples of suitable devices include tissue engineering scaffolds and grafts (such as vascular grafts, grafts or implants used in nerve regeneration). The present polymers may also be used to form a variety of devices effective for use in closing internal wounds. For example, biodegradable sutures, clips, staples, barbed or mesh sutures, implantable organ supports, and the like, for use in various surgery, cosmetic applications, and cardiac wound closures can be formed.

Various devices finding use in dental applications may advantageously be formed. For example, devices for guided tissue regeneration, alveolar ridge replacement for denture wearers, and devices for the regeneration of maxilla-facial bones may benefit from being radiopaque so that the surgeon/dentist can ascertain the placement and continuous function of such implants by simple X-ray imaging.

The present polymers are also useful in the production of gastric lap bands for use in the treatment of obesity. The production of radiopaque lap bands allows for more effective monitoring of the devices in the human body, and more effective treatment of obesity.

In addition to intravascular stents and non-cardiovascular stents, the present polymers are useful in a number of other cardiovascular and vascular devices. For example, valves, chordae tendinea replacements, annuloplasty rings, leaflet repair patches, vascular grafts, vascular tubes, patches for septal defects, arterial and venous access closure devices (plugs), and the like can be formed for use in replacement repair of heart valves, tubes, and the like. In addition, portions of an artificial heart, such as the rough surface/fibroid layer (bellow pumps) may be formed from the polymers of the instant invention

The present polymers are further useful in the production of a wide variety of therapeutic delivery devices. Such devices may be adapted for use with a variety of therapeutics including, for example, pharmaceuticals (i.e., drugs) and/or biological agents as previously defined and including biomolecules, genetic material, and processed biologic materials, and the like. Any number of transport systems capable of delivering therapeutics to the body can be made, including devices for therapeutics delivery in the treatment of cancer, intravascular problems, dental problems, obesity, infection, and the like. In certain embodiments, any of the aforementioned devices described herein can be adapted for use as a therapeutic delivery device (in addition to any other functionality thereof). Controlled therapeutic delivery systems may be prepared, in which a biologically or pharmaceutically active and/or passive agent is physically embedded or dispersed within a polymeric matrix or physically admixed with a polycarbonate or polyarylate of the present invention. Controlled therapeutic delivery systems may also be prepared by direct application of the therapeutic to the surface of a bioresorbable stent device (comprised of at least one of the present polymers) without the use of these polymers as a coating, or by use of other polymers or substances for the coating.

One major advantage of using the radiopaque, bioresorbable polymers produced with methods of the instant invention in therapeutic delivery applications is the ease of monitoring the release of a therapeutic and the presence of the implantable therapeutic delivery system. Because the radiopacity of the polymeric matrix is due to covalently attached halogen substituents, the level of radiopacity is directly related to the residual amount of the degrading therapeutic

delivery matrix still present at the implant site at any given time after implantation. In preferred embodiments, the rate of therapeutic release from the degrading therapeutic delivery system will be correlated with the rate of polymer resorption. In such preferred embodiments, the straightforward, quantitative measurement of the residual degree of radio-opacity will provide the attending physician with a way to monitor the level of therapeutic release from the implanted therapeutic delivery system.

The following non-limiting examples set forth below illustrate certain aspects of the invention. All parts and percentages are by weight unless otherwise noted and all temperatures are in degrees Celsius.

## EXAMPLES

### Nomenclature and Abbreviations Used

The following abbreviations are used to identify the various iodinated compounds. TE stands for tyrosine ethyl ester, DAT stands for desaminotyrosine and DTE for desaminotyrosyl tyrosine ethyl ester. The polymer obtained by phosgenation of DTE is denoted as poly(DTE carbonate). An "I" before the abbreviation shows mono-iodination (e.g. ITE stands for mono-iodinated TE) and an $I_2$ before the abbreviation shows di-iodination (e.g. $I_2$DAT stands for di-iodinated DAT). In DTE, if the "I" is before D, it means the iodine is on DAT and if "I" is after D, it means the iodine is on the tyrosine ring (e.g. $DI_2$TE stands for DTE with 2 iodine atoms on the tyrosine ring). The following diagram illustrates this nomenclature further.

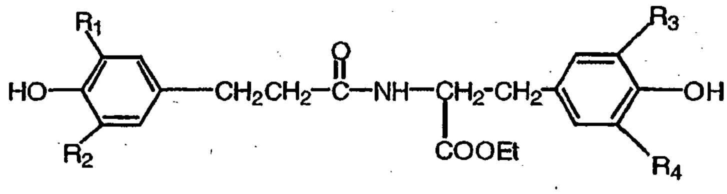

General Structure of iodinated DTE monomer
$R_1$=I, $R_2$, $R_3$, $R_4$ = H; IDTE
$R_1$, $R_2$ = I , $R_3$, $R_4$ = H ; $I_2$DTE
$R_1$,$R_2$=H, $R_3$, $R_4$ = I; $DI_2$TE
$R_1$, $R_3$=I, $R_2$, $R_4$ =H; IDITE

### Tensile Testing

Testing was performed using an Endura TEC EMS with appropriate load cell, running WinTest Software Version 2.22 (Minnetonka, MN) and per the ASTM D882-02 Standard Test Method for Tensile Properties of Thin Plastic Sheeting. Briefly, to simulate in vivo conditions, thin film samples were produced using a Thermal Press Method using a PHI Tulip lab press (Model Q230) with a calibrated temperature range of 0-315°C. The thin films were hydrated in 7.4 pH phosphate buffed saline for 30 minutes then tensile tested while submerged. Data was collected and analyzed per ASTM D 882-02 to obtain the modulus of elasticity, yield point, yield strength, percent elongation at yield, maximum tensile, and maximum elongation.

### Resorption Testing

Polymer degradation rate was measured *in vivo* and *in vitro* using the materials and methods described in Abramson et al., " Small changes in polymer structure can dramatically increase degradation rates: the effect of free carboxylate groups on the properties of tyrosine-derived polycarbonates," Sixth World Biomaterials Congress Transactions, Society for Bioinaterials 26th Annual Meeting, Abstract 1164 (2000), the disclosure of which is incorporated by reference.

### Example 1: Preparation of 3,5-diiodo-4-hydroxyphenyl propionic acid (3,5-di-iodo-desaminotyrosine, $I_2$DAT)

Dissolve 50 g (0.300 mol) of DAT in 500 mL of 95% ethanol. To the solution with stirring was added 146 g (0.605 mol) of PyICl. The solution was stirred for 30 min when the solid slowly dissolved to give a light yellow solution. This was added over 30 min to 2.5 liters of water containing 10 g sodium thiosulfate. The water was stirred during this addition. An off-white solid separated and was isolated by filtration and washed with several portions of deionized water.

The solid was transferred to a large beaker along with 2 L of deionized water and 24 g of sodium hydroxide and stirred to dissolve. The filtrate was acidified with 35 mL acetic acid (pH about 4). The white solid formed was isolated by filtration and washed with several portions of water. A rubber dam was used to squeeze out all the water. The solid was dried under nitrogen and then under vacuum. The dry solid was purified by recrystallization from 1:2 acetone-water. 50 g of crude $I_2$DAT was obtained and characterized by HPLC and $^1$H NMR.

**Examples 2 and 3: Preparation of Diiodinated-DTE ($I_2$DTE): (not claimed)**

Diiodinated monomer ($I_2$DTE) was prepared using procedures similar to those published in the literature by substituting $I_2$DAT in the place of DAT. In a typical procedure 53.3 g (0.255 mol) of tyrosine ethyl ester, 104 g (0.250 mol) of $I_2$DAT and 3 g (0.025 mol) 1-hydroxybenzotriazole were stirred with 500 mL of tetrahydrofuran in a 1 liter round-bottomed flask. The flask was cooled in ice-water bath to 10-18 °C and 50 g (0.255 mol) of EDCI was added and stirred for 1 h at 15-22 °C. This was followed by stirring at ambient temperature for 5 h. The reaction mixture was concentrated to 250 mL and then stirred with 1 L of water and 1 L of ethyl acetate. The lower aqueous layer was separated and discarded using a separatory funnel. The organic layer was sequentially washed with 500 mL each of 0.4 M HCl, 5% sodium bicarbonate solution and 20% sodium chloride solution. After drying over anhydrous sodium sulfate, the organic layer was concentrated to syrup and triturated by stirring with hexane. Alight yellow solid is obtained. The product is characterized by HPLC and $^1$H NMR. Using similar procedures $I_2$DTtBu was prepared by coupling $I_2$DAT and tyrosine t-butyl ester (TtBu), which is commercially available.

**Examples 4 and 5: Preparation of iodinated tyrosine esters (not claimed)**

3-Iodotyrosine ethyl ester (ITE), and 3,5-diiodotyrosine ethyl ester ($I_2$TE) were prepared from the corresponding iodinated tyrosine by esterification with ethanol and thionyl chloride. The iodinated tyrosines were prepared by the method of Example 1.

**Examples 6 -11: Other iodinated diphenolic monomers (not claimed)**

A number of other iodinated monomers were prepared by coupling the following combinations of two phenolic reagents according to the methods of Examples 2 - 3. The following lists monomers that were prepared:

DITE: DAT and ITE
IDITE: IDAT and ITE
$I_2$DTE: $I_2$DAT and TE
D$I_2$TE: DAT and $I_2$TE
$I_2$DTtB: $I_2$DAT and TtB
$I_2$DITE: $I_2$DAT and ITE

FIG. 4a-b show X-ray comparisons of radiopaque bioresorbable di -iodinated and tri-iodinated tyrosine-derived polycarbonate films. The poly(I2DITE-co-20%PEG2k) carbonate 114 micron films have a photo-density equivalent to human bone. That of poly(80%I2DTE-co-20%PEG2k) carbonate has a lower photo-density.

**Example 12: Polymer containing iodine and poly(ethylene glycol) (not claimed)**

A polymer containing 97.5% mole percent $I_2$DTE and 2.5% poly(ethylene glycol) of molecular weight 2000 (poly(97.5%$I_2$DTE-co-2.5%PEG2K carbonate) )was prepared as follows. Into a three necked round-bottomed flask, equipped with a mechanical stirrer, a thermometer, a reflux condenser and a rubber septum, were added 29.7 g (0.0488 mol) of $I_2$DTE, 2.5 g (0.00125 mol) of PEG2000, and 215 mL of methylene chloride. On stirring a clear light yellow solution was obtained. To this was added 15.1 mL (0.15 mol) of pyridine. In a gas tight plastic syringe was placed 30 mL of a 20% solution of phosgene in toluene (0.0576 mol), which was added to the reaction flask over 3 h using a syringe pump. The molecular weight was determined by analyzing an aliquot of the reaction mixture by GPC. Additional phosgene solution (up to 10%) was added to achieve the desired molecular weight. The reaction mixture was quenched with 110 mL of tetrahydrofuran and 10 mL of water. The polymer was precipitated by adding the reaction mixture to 1.5 L of cold 2-propanol in high speed Waring blender. The resulting polymer was ground with two portions of 0.5 L 2-propanol. The fine granular polymer particles were isolated by filtration and dried in a vacuum oven.

**Examples 13 and 14: Preparation of Poly(DTE carbonate) and Poly(I-DTE carbonate) (not claimed)**

Poly(DTE carbonate) and Poly(I-DTE carbonate) were prepared by the method of Example 12, substituting DTE and I-DTE for $I_2$-DTE, respectively.

**Example 15: Polymer for stent preparation**

The following example describes the preparation of poly(87.5%$I_2$DTE-co-10%$I_2$DTtBu-co-2.5%PEG2K carbonate) that is typically used in stent preparation. Into a three necked round-bottomed flask, equipped with a mechanical stirrer, a thermometer, a reflux condenser and a rubber septum were added 26.6 g (0.044 mol) of $I_2$DTE, 3.20 g (0.005 mol) of $I_2$DTtBu, 2.5 g (0.00125 mol) of PEG2000, and 215 mL of methylene chloride. On stirring a clear light yellow solution was obtained. To this was added 15.1 mL (0.15 mol) of pyridine. In a gas tight plastic syringe was placed 30 mL of a 20% solution of phosgene in toluene (0.0576 mol) and added to the reaction flask over 3 h using a syringe pump. The molecular weight was determined by analyzing an aliquot of the reaction mixture by GPC. Additional phosgene solution (up to 10%) was needed to achieve desired molecular weight. The reaction mixture was quenched with 110 mL of tetrahydrofuran and 10 mL of water. The polymer was precipitated by adding the reaction mixture to 1.5 L of cold 2-propanol in high speed Waring blender. The resulting gluey polymer was ground with two portions of 0.5 L 2-propanol. The fine granular polymer particles were isolated by filtration and dried in a vacuum oven.

**Example 16: Deprotection of t-butyl side chain**

To remove the t-Butyl protecting group, 25 g of poly(87.5%$I_2$DTE-co-10%$I_2$DTtBu-co-2.5%PEG2K carbonate) prepared above was stirred with 125 mL of trifluoroacetic acid (TFA) to obtain a 20% solution. After all the polymer particles went into solution, the stirring was continued for 4 h at room temperature. The polymer was precipitated by adding the solution to 1 liter of 2-propanol in a high speed Waring Blender. The resulting polymer particles were ground with 500 mL of 2-propanol twice to remove trace TFA. The product was isolated by filtration, washed with IPA and dried in vacuum oven at 40 °C.

**Examples 17 -19: Preparation of Poly(DTE-co-PEG carbonates) (not claimed)**

Poly(DTE-co-5%PEG1k carbonate) was prepared according to the method of Example 15, substituting DTE for both $I_2$-DTE and $I_2$-DttBu and PEG1000 for PEG2000 and adjusting the stoichiometry to increase the molar ratio of PEG. Using the same procedure poly($I_2$DTE-co-2.5%PEG2000-carbonate) and poly($I_2$DTE-co-3.4%PEG2000-carbonate) were prepared.

**Examples 20 - 25: Preparation of Poly(DTE-co-DT carbonates) (not claimed)**

Poly(63% DTE-co-37%DT carbonate) was prepared according to the method of Example 15, omitting the use of PEG and adjusting the stoichiometry to obtain the desired ratio of DTE to DT. The t-butyl groups were deprotected according to the method of Example 16. By the method poly(90% DTE-co-10%DT carbonate), poly(85% DTE-co-15%DT carbonate), poly(83% DTE-co-1,7%DT carbonate), poly(76% DTE-co-24%DT carbonate) and poly(75% DTE-co-25%DT carbonate) were also prepared.

**Example 26: Preparation of poly($I_2$DTE-co-2.5mole%$PEG_{2k}$ adipate) (not claimed)**

The diphenol $I_2$DTE (2.97 g, 4.87 mmol), PEG2000 ( 0.250 g, 0.125 mmol) and adipic acid (0.731 g, 5.04 mmol) and 0.4 g of DPTS (dimethylamonopyridyl-paratoluene sulfonate, catalyst) were weighed into a 100 mL brown bottle with Teflon-lined cap. To the bottle is also added 40 ml of methylene chloride, and securely capped. The bottle is agitated for 10-15 min and then 2.5 mL (2.02 g, 16 mmol) of diisopropylcarbodiimide is added and continued to agitate for 2 h. An aliquot of the sample is withdrawn and after proper treatment analyzed by GPC. A Mw of about 100,000 is desirable. Once the desired Mw is reached, 200 mL of 2-propanol is added to the reaction mixture with stirring. The precipitate is collected and dried in a stream of nitrogen. The precipitate is then dissolved in 20 mL of methylene chloride and precipitated with 200 mL of methanol. Then the polymer is dried under nitrogen, followed by drying in a vacuum oven.

**Example 27: Polymerization of poly(60 % $I_2$DTE-co-20% $I_2$DT-co-20%$PEG_{2k}$ adipate)**

The diolic components (1.83 g, 3.00 mmol of $I_2$DTE, 0.638 g , 1.00 mmol $I_2$DTtB, and 2.000 g 1.00 mmol of PEG2000), and the diacid (0.731 g, 5 mmol adipic acid) and 0.4 g, of DPTS were weighed into a 100 mL brown bottle

with Teflon-lined cap. To the bottle is also added 40 ml of methylene chloride, and securely capped. The bottle is agitated for 10-15 min and then 2.5 mL (2.02 g, 16 mmol) of diisopropylcarbodiimide is added and continued to agitate for 2 h. An aliquot of the sample is withdrawn and after proper treatment analyzed by GPC. A Mw of about 100,000 is desirable. Once the desired Mw is reached, 200 mL of 2-propanol is added to the reaction mixture, with stirring. The precipitate is collected and dried in a stream of nitrogen. The precipitate is then dissolved in 20 mL of methylene chloride and precipitated with 200 mL of methanol. Then the polymer is dried under nitrogen, followed by drying in a vacuum oven.

Deprotection: The resulting polymer is dissolved in trifluoroacetic acid (10% w/v) and allowed to stir overnight. The following day, the polymer is precipitated in isopropanol using a blender for mixing. The polymer is then ground twice with fresh isopropanol, filtering with a fritted filter between washes. Then the polymer is dried under nitrogen, followed by drying in a vacuum oven.

**Example 28: Preparation of poly($I_2$DTE-co-2.5mole%$PEG_{2k}$ sebacate) (not claimed)**

The diphenol $I_2$DTE (2.98 g, 4.89 mmol), PEG2000 ( 0.250 g, 0.125 mmol). and sebacic acid (1.01 g, 5.00 mmol) and 0.4 g of DPTS are weighed into a 100 mL brown bottle with Teflon-lined cap. To the bottle is also added 40 ml of methylene chloride, and securely capped. The bottle is agitated for 10-15 min and then 2.5 mL (2.02 g, 16 mmol) of diisopropylcarbodiimide is added and continued to agitate for 2 h. An aliquot of the sample is withdrawn and after proper treatment analyzed by GPC. A Mw of about 100,000 is desirable. Once the desired Mw is reached, 200 mL of 2-propanol is added to the reaction mixture, with stirring. The precipitate is collected and dried in a stream of nitrogen. The precipitate is then dissolved in 20 mL of methylene chloride and precipitated with 200 mL of methanol. Then the polymer is dried under nitrogen, followed by drying in a vacuum oven.

Applicants conducted detailed studies, aimed at discovering optimized polymer compositions that can fulfill all of the above requirements. A key finding is documented in Table 1 below.

**Table 1: Effect of Iodination and PEG on Mechanical Properties**

| **Test Material** (average of 5 repetitive measurements | **Elongation at Yield (%)** | **Yield strength (PSI)** | **Strength at break (PSI)** | **Elastic Modulus (PSI)** |
|---|---|---|---|---|
| Poly(DTE carbonate) | 2.8% | 5400 | 6700 | 198,500 |
| Poly(I-DTE carbonate) | 2.8% | 5000 | 5800 | 183,000 |
| Poly($I_2$-DTE carbonate) | 1.1% | 2000 | 2000 | 183,000 |
| Poly(DTE-co-5%PEG1k carbonate) | 500% | 2200 | 2800 | 84,000 |
| Poly($I_2$-DTE-co-2.5mole%PEG2K carbonate) | 2.6% | 5400 | 7400 | 216,000 |

**N= 5 each, soaked for 30 minutes in 7.4 pH PBS at 37°C**
**(Rounded to significant digits)**

Table 1 illustrates that poly(DTE carbonate) (defined by Formula 1 when f = 0, g = 0, X = Y = 0, $R_1$ = ethyl, and A = -C(=O)- ) is sufficiently strong to be a promising candidate material for use in the fabrication of a bioresorbable stent (see Row 1). However, this material is not radiopaque and not sufficiently hemocompatible. As outlined above, the incorporation of iodine substituents reduces the polymer mechanical strength. At mono-iodination (Row 2, the polymer is still strong enough to be a useful stent material, but is not sufficiently radiopaque to be visible by X-ray fluoroscopy. When two iodine atoms are incorporated into the polymer structure, the polymer is sufficiently radiopaque, but its mechanical strength is now insufficient (Row 3). Likewise, when PEG is incorporated into the polymer backbone (f = 0.05), the polymer is dramatically weakened (Row 4). In fact, as little as 5 mol% of PEG result in a 50% reduction in polymer strength and stiffness and completely disqualifies the corresponding polymer from further consideration as a stent material if used in and of itself; laminate and multi-polymer stent designs may use such a polymer for a specific design purpose.

Against this background, applicants have now discovered that the incorporation of both iodine AND a low percentage of PEG into the polymer has the non-obvious and entirely unexpected effect of significantly improving the mechanical properties of the polymer (see Row 5 of Table 1). Those skilled in the art of polymer science would predict with confidence a synergistic effect: Since PEG and iodine each individually reduce the mechanical strength of the polymer, the simultaneous incorporation of both PEG and iodine within the same polymer should have resulted in a pronounced reduction in the mechanical strength of the polymer containing both iodine and PEG. Contrary to this expectation, the combination of iodine and PEG incorporation has an "anti-synergistic" effect resulting in a polymer

composition that surpassed poly(DTE carbonate) in strength and stiffness.

FIG. 1 illustrates that the polymer composition of Row 5 in Table 1 is indeed sufficiently strong to be useful in the fabrication of a fully functional stent and sufficiently radiopaque to be visible by X-ray fluoroscopy in an animal heart. Comparison with a clinically used stainless steel stent shows a virtually identical level of visibility. FIGS 2A and 2B are light micrographs demonstrating that polymer compositions containing iodine substituents without PEG may be too brittle to allow fabrication of functional stents. FIG. 2A shows that a stent frame formed from a poly($I_2$-DTE carbonate) (without PEG) broke (see arrows) at multiple sites under gentle manipulation. The mechanical properties of the polymer were dramatically improved by the simultaneous incorporation of both PEG and iodine into the polymer. FIG. 2B shows that poly($I_2$-DTE-co-2.5%PEG2K carbonate) had sufficient stiffness and ductility to be readily fabricated into stents. The polymer composition of Row 5 in Table 1 was thus sufficiently strong and ductile for stent fabrication.

Table 2 illustrates the minor effect of DT incorporation on polymer mechanical properties. While there is a trend to reduce strength at yield and break, the elongation and elastic modulus remain virtually unchanged - in spite of a dramatic reduction in the time to device resorption.

**Table 2: Effect of DT Units on Mechanical Properties and Resorption Time (Rounded to significant digits)**

| **Test Material** (average of 5 repetitive measurements | **Elongation at yield (%)** | **Yield strength (PSI)** | **Strength at break (PSI)** | **Elastic Modulus (PSI)** | **Expected time to full resorption** |
|---|---|---|---|---|---|
| Poly(DTE Carbonate) | 2.8% | 5400 | 5600 | 231,400 | 4 years |
| Poly(90% DTE-co-10%DT Carbonate) | 2.7% | 5100 | 5300 | 190,000 | 2 years |
| Poly(85% DTE-co-15%DT Carbonate) | 2.4% | 4800 | 4900 | 204,000 | 1.5 years |
| Poly(83% DTE-co-17%DT Carbonate) | 2.3% | 5000 | 5400 | 221,000 | 1.4 years |
| Poly(75% DTE-co-25%DT Carbonate) | 2.1% | 4600 | 4800 | 225,000 | 0.5 years |
| Poly(63% DTE-co-37%DT Carbonate) | 2.4% | 4300 | 4400 | 181,000 | 0.1 years |

**Example 29: Fibrinogen adsorption to polymeric surfaces (not claimed)**

The time course of human fibrinogen adsorption to the test polymer and stainless steel surfaces were measured using a Quartz Crystal Microbalance with Dissipation monitoring (QCM-D, Q-Sense AB, model D300, Goeteborg, Sweden).

QCM-D is a gravimetric technique and useful for measuring in real-time the mass of material in liquid adhering to a surface. An increase in mass bound to the quartz surface causes the crystal's oscillation frequency to decrease. Moreover, this device can measure the change of dissipation induced by the surface-adsorbed mass.

Quartz crystals (Q-Sense, Cat # QSX-301) were spin-coated with polymer solutions (1% polymer in methylene chloride). Commercially available quartz crystals coated with a thin layer of stainless steel (Q-Sense, Cat # QSX-304) were included, too. To start a typical experiment, the crystals were inserted into the QCM-D instrument and incubated in phosphate-buffered saline (PBS) at 37°C. After reaching a stable baseline, the fibrinogen solution was injected and the frequency and dissipation shifts induced by adsorbed mass, were recorded in real-time. The fibrinogen solution was incubated until the binding saturation was reached (as indicated by absence of further significant changes in frequency and dissipation values). PBS without fibrinogen was used for all rinsing steps to remove non-bound fibrinogen from the sensor surface after the adsorption process. Human fibrinogen was purchased from Calbiochem (Cat # 341576) and diluted in PBS to a final concentration of 3 mg/mL. All experiments were performed in triplicate with a standard deviation of less than 12 % (standard error mean).

The quartz crystals could be reused up to 10 times by applying the following cleaning procedure: Quartz crystals were treated with a cleaning solution (80°C, 15 min) consisting of $H_2O_2$ (30%), $NH_4OH$ and ultrapure water in a 1:1:5 ratio. Thereafter, crystals were extensively rinsed with ultrapure water and blow dried with nitrogen. Finally, the crystals were exposed to UV and ozone for 15 min (UVO cleaner, Jelight Company, Irvine, CA, USA).

Table 3 summarizes the comparative evaluation of different stent polymer formulations with respect to fibrinogen adsorption in vitro. Fibrinogen is a key blood protein. The degree of fibrinogen adsorption on an artificial surface in contact with blood is widely regarded as a reliable indicator of the tendency of said surface to be hemocompatible. As a general rule, known to those skilled in the art of biomedical engineering, the lower the level of fibrinogen adsorption

onto a material, the higher the hemocompatibility of that material.

**Table 3: Relative levels of fibrinogen adsorption on test surfaces as measured in vitro by the frequency shift of a quartz microbalance (Q-sense)**

| Item | Test material | Fibrinogen adsorption (relative units) |
|---|---|---|
| 1 | Stainless Steel, SS2343 | 83 |
| 2 | PET (Dacron) | 179 |
| 3 | poly(DTE-carbonate) | 158 |
| 4 | poly($I_2$DTE-carbonate) | 133 |
| 5 | poly(76%DTE-co-24%DT-carbonate) | 125 |
| 6 | poly($I_2$DTE-co-2.5%PEG2000-carbonate) | 100 |
| 7 | poly($I_2$DTE-co-3.4%PEG2000-carbonate) | 72 |

In reference to Table 3, item 1 (stainless steel) represents a clinically used stent material, which is known for its low level of thrombogenicity and its good hemocompatibility. Stainless steel serves as a control and has an acceptable level of fibrinogen adsorption. Item 2 in Table 3 is Dacron, a known thrombogenic material which has only limited clinical utility in vascular applications. Dacron has the highest level of fibrinogen adsorption of all test materials. Item 3 is poly(DTE carbonate), the base material among the polymers represented by Formula I. Its high level of fibrinogen adsorption indicates that this polymer is not a promising candidate for use in a blood-contacting medical device. Either incorporation of iodine alone (Item 4) or incorporation of DT Units alone (Item 5) tend to reduce the level of fibrinogen adsorption, however, the reduction is not sufficient to qualify either of these polymer compositions as promising materials for use in stents.

The foregoing demonstrates that the simultaneous incorporation of iodine, DT, and PEG results in a major reduction in fibrinogen adsorption - at PEG levels that are still compatible with the need to provide a mechanically strong polymer. Within this general regimen, applicants now provide yet another unexpected observation: Comparison of items 6 and 7 shows that a very small, incremental increase in the amount of PEG within the polymer composition can have a non-obvious and non-predictable effect on protein adsorption. Fibrinogen adsorption to polymer composition 6 is sufficiently low to qualify this composition as a promising candidate material for use in stents while as little as 0.9 mol% of additional PEG added to polymer composition 7 provided a polymer composition which appears to be superior in terms of its hemocompatibility to the clinically used stainless steel.

Polymer composition 7 in Table 3 illustrates another key design principle recognized for the first time by the applicants: When iodine and PEG are incorporated concomitantly into a polymer composition covered by Formula I, a very low molar ratio of PEG is sufficient to reduce dramatically the level of fibrinogen surface adsorption. In combination with the previously described effect of iodine and PEG on the mechanical properties of the polymer composition, applicants have discovered a method to simultaneously optimize both the mechanical and biological properties of polymers for use in stent applications.

**Example 30: In-Vitro Drug Elution Kinetics (not claimed)**

This is determined for the release of drug out of certain polymers, based on physiochemical characteristics and solvent extraction requirements at 37°C under "sink" conditions, and with agitation to ensure dissolution homogeneity. The therapeutic substance (e.g., drug) in a polymer (see table below) may be coated on to the surface of a polymer film, on metal stents or metal surfaces and it may be embedded or blended with the polymer prior to pressing the film.

Film size is adjusted to accommodate drug load and detection limits for quantitation. A typical procedure might include compound extraction or precipitation, followed by quantitation using high performance liquid chromatography (HPLC). An appropriate dissolution media such as 3% Bovine Serum Albumin (BSA) or 35% Tween 20 in a phosphate buffer saline (PBS) is used. Dissolution may be determined from 24 hours out to 28 days. After dissolution, the drug content of films and/or media is analyzed. Dissolution rate is calculated for each drug using a mass balance determination from this HPLC assay. The percent dissolved is calculated by using the quantities measured at each time point for the overall dissolution profile.

**Table 4 - Summary of Testing of Tyrosine-Derived Polycarbonate Coatings**

| Test Material |
|---|
| Poly (95% I2DTE-co-5% PEG 1K) Carbonate[1,2] |

(continued)

| Test Material |
|---|
| Poly (97.5% I2DTE-co-2.5% PEG 2K) Carbonate |
| Poly (77.5% I2DTE-co-20%I2DT-2.5% PEG 2K) Carbonate |
| Poly (67.5% I2DTE-co-30%I2DT-2.5% PEG 2K) Carbonate |
| Poly (70% I2DTE-co-20%I2DT-10% PEG 2K) Carbonate |
| Poly (80% I2DTE-co-20% PEG 2K) Carbonate |
| |
| **Control Material for Comparison** |
| Poly (95% DTE-co-5% PEG 1K) Carbonate |
| [1] Polymer only was applied to a steel stent to determine by scanning electron microscopy (SEM) the surface characteristics of the polymer coating.<br>[2]Polymers with a drug were applied to metal stents and the biocompatibility and drug elution effects were determined in an in vivo system, pig coronary arteries. |

Drug elution with the various polymers that were coated onto a surface or embedded in the polymer and compressed into a film has demonstrated drug elution. FIG. 3 shows that elution of drug out of poly-DTE-carbonates can be tailored by modifying the polymer with iodines on the DAT ring and by adding PEG to the back bone of the polymer. SEM studies showed that polymer applied directly to a steel stent adhered to and remained intact after stent deployment. SEM has also demonstrated that polymer-drug applied to the steel or polymer stents will adhere to and remain intact after stent deployment. Polymers with a drug applied to metal and to polymer stents demonstrated biocompatibility in pig coronary arteries when tested in vivo for 28 days and a decrease of restenosis (versus non-drug coated stents) was shown as the drug, an anti-proliferative, eluted and implemented its effect.

The foregoing description of the preferred embodiment should be taken as illustrating, rather than as limiting, the present invention as defined by the claims. As would be readily appreciated, numerous variations and combinations of the features set forth above can be utilized without departing from the present invention as set forth in the claims. Such variations are not regarded as a departure from the spirit and scope of the invention, and all such variations are intended to be included within the scope of the following claims.

## Claims

1. A method for the selective removal of a tert-butyl protection group from a hydrolytically unstable polymer to form a new polymer composition having a free carboxylic acid group in place of said tert-butyl protection group, said method comprising dissolving said hydrolytically unstable polymer in a solvent comprising an amount of an acid having a pKa from 0 to 4, wherein said hydrolytically unstable polymer comprises one or more units described by Formula III:

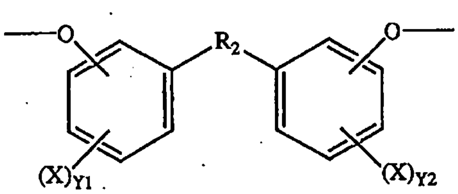

(III)

wherein X for each polymer unit is independently Br or I, Y1 and Y2 are independently between 0 and 4 inclusive, Y1 + Y2 is between 0 and 8, inclusive, and $R_2$ for each unit is independently an alkyl, aryl or alkylaryl group containing up to 18 carbon atoms and from 0 to 8 heteroatoms selected from O and N, and $R_2$ further comprises a pendant tert-butyl protection group.

2. The method of claim 1, wherein said hydrolytically unstable polymer is soluble in said solvent.

3. The method of claim 1, wherein said solvent consists essentially of said acid.

4. The method of claim 1, wherein said solvent is selected from the group consisting of chloroform, methylene chloride, tetrahydrofuran, dimethylformamide, and mixtures thereof.

5. The method of claim 1, wherein said acid is selected from the group consisting of formic acid, trifluoroacetic acid, chloroacetic acid, and mixtures thereof.

6. The method of claim 1, wherein said acid is formic acid.

7. The method of claim 1, wherein said polymer is copolymerized with up to 75 wt% of a poly($C_1$-$C_4$ alkylene glycol).

8. The method of claim 7, wherein said poly($C_1$-$C_4$ alkylene glycol) weight fraction is less than 50 wt%.

9. The method of claim 1, wherein $R_2$ comprises:

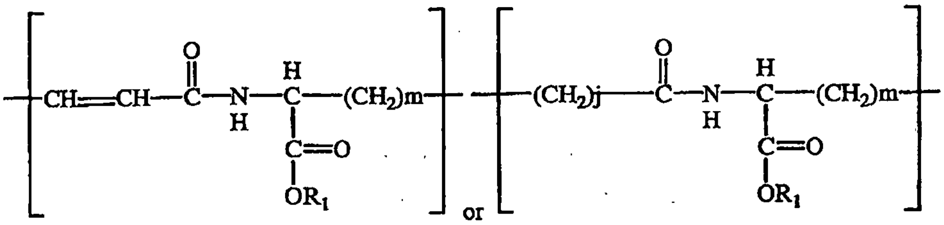

wherein j and m are independently an integer from 1 to 8, inclusive, and $R_1$ is a tert-butyl group.

10. The method of claim 1, wherein said polymer is copolymerized with up to 75 wt% of a poly($C_1$-$C_4$ alkylene glycol).

11. The method of claim 10, wherein said poly($C_1$-$C_4$ alkylene glycol) weight fraction is less than 40 wt%.

12. The method of claim 1, wherein all X groups are ortho-directed, and Y1 +Y2 = 1, 2, 3 or 4.

13. The method of claim 1, wherein every X is iodine.

14. The method of claim 1, wherein said hydrolytically unstable polymer comprises one or more units defined by Formula I:

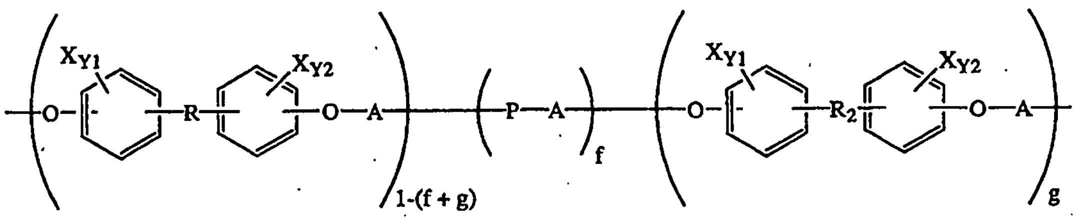

(I)

wherein each X is independently I or Br, Y1 and Y2 for each diphenol unit are independently between 0 and 4, inclusive, and Y1 + Y2 for each diphenol unit is between 0 and 8, inclusive,
wherein R and $R_2$ for each unit are independently an alkyl, aryl or alkylaryl group containing up to 18 carbon atoms and from 0 to 8 heteroatoms selected from O and N, and $R_2$ further comprises a pendant t-butyl ester group; wherein A is either:

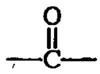 or 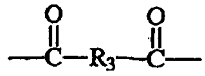 ;

wherein $R_3$ is a saturated or unsaturated, substituted or unsubstituted alkyl, aryl, or alkylaryl group containing up to 18 carbon atoms and 0 to 8 heteroatoms selected from O and N;
wherein P is a poly($C_1$-$C_4$ alkylene glycol) unit; f ranges from 0 to less than 1; g ranges from 0 to 1, inclusive; and f + g ranges from 0 to 1, inclusive.

**15.** The method of claim 14, wherein $R_2$ comprises:

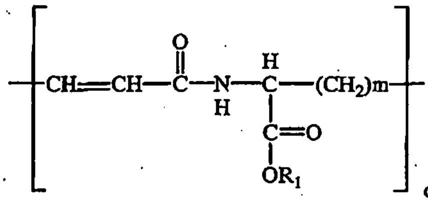 or 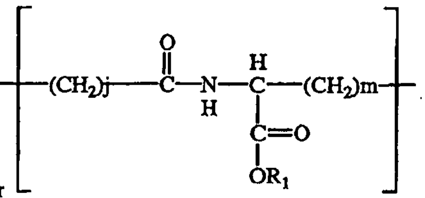

wherein j and m are independently an integer from 1 to 8, inclusive; and $R_1$ is a tert-butyl (tB) group.

**16.** The method of claim 14, wherein all X groups are ortho-directed, and Y1 + Y2 = 1, 2, 3 or 4.

**17.** The method of claim 14, wherein every X is iodine.

**18.** The method of claim 1, wherein said polymer is a polycarbonate, polyarylate, polyiminocarbonate or a phosphorus-containing polymer, wherein phosphorous containing polymers have the structure as follows:

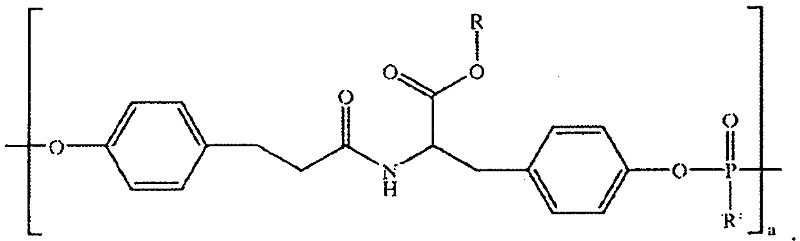

wherein either:

R is tert-butyl,
R' is selected from alkyl, alkoxy, aryl, aryloxy, heterocyclic or heterocycloxy, and
n is between 2 to 500;

or:

R is tert-butyl, R' is selected from H, alkyl, alkoxy, aryl, aryloxy, heterocyclic or heterocycloxy, and n is between 5 to 500.

**19.** The method of claim 18, wherein said polymer is a halogen-substituted radiopaque polymer.

**20.** The method of claim 18, wherein said polymer is a copolymerized with up to 75 wt % of a poly($C_1$-$C_4$ alkylene glycol).

## Patentansprüche

1. Verfahren zum selektiven Entfernen einer tert-Butyl-Schutzgruppe von einem hydrolytisch instabilen Polymer, um eine neue Polymerzusammensetzung zu bilden, welche statt der tert-Butyl-Schutzgruppe eine freie Carbonsäuregruppe aufweist, wobei das Verfahren das Auflösen des hydrolytisch instabilen Polymers in einem Lösungsmittel umfasst, welches eine Menge einer Säure umfasst, mit einem $pK_a$-Wert von 0 bis 4, wobei das hydrolytisch instabile Polymer eine oder mehrere Einheiten der Formel III umfasst:

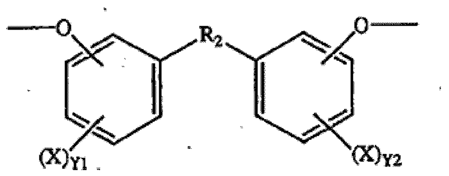

(III)

wobei X für jede Polymereinheit unabhängig für Br oder I steht, Y1 und Y2 unabhängig für 0 bis einschließlich 4 stehen, Y1 + Y2 0 bis einschließlich 8 beträgt und $R_2$ für jede Einheit unabhängig für eine Alkyl-, Aryl- oder Alkylarylgruppe steht, welche bis zu 18 Kohlenstoffatome und 0 bis 8 Heteroatome enthält, ausgewählt aus O und N, und $R_2$ ferner eine tert-Butyl-Schutzgruppe als Seitengruppe umfasst.

2. Verfahren nach Anspruch 1, wobei das hydrolytisch instabile Polymer in dem Lösungsmittel löslich ist.

3. Verfahren nach Anspruch 1, wobei das Lösungsmittel im Wesentlichen aus der Säure besteht.

4. Verfahren nach Anspruch 1, wobei das Lösungsmittel aus der Gruppe ausgewählt ist, die aus Chloroform, Methylenchlorid, Tetrahydrofuran, Dimethylformamid und Gemischen dieser besteht.

5. Verfahren nach Anspruch 1, wobei die Säure aus der Gruppe ausgewählt ist, die aus Ameisensäure, Trifluoressigsäure, Chloressigsäure und Gemischen dieser besteht.

6. Verfahren nach Anspruch 1, wobei die Säure Ameisensäure ist.

7. Verfahren nach Anspruch 1, wobei das Polymer mit bis zu 75 Gew.-% eines Poly-($C_{1-4}$-alkylenglykols) copolymerisiert ist.

8. Verfahren nach Anspruch 7, wobei der Gewichtsanteil des Poly-($C_{1-4}$-alkylenglykols) weniger als 50 % beträgt.

9. Verfahren nach Anspruch 1, wobei $R_2$ umfasst:

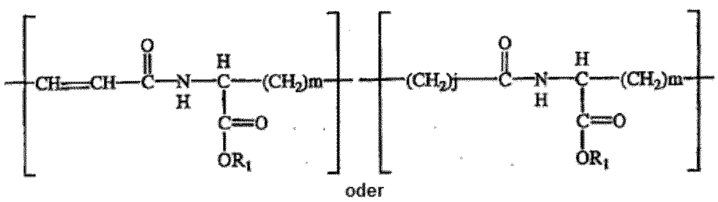

oder

wobei j und m unabhängig für eine ganze Zahl von 1 bis einschließlich 8 stehen und $R_1$ für eine tert-Butyl-Gruppe steht.

10. Verfahren nach Anspruch 1, wobei das Polymer mit bis zu 75 Gew.-% eines Poly-($C_{1-4}$-alkylenglykols) copolymerisiert ist.

11. Verfahren nach Anspruch 10, wobei der Gewichtsanteil des Poly-($C_{1-4}$-alkylenglykols) weniger als 40 % beträgt.

12. Verfahren nach Anspruch 1, wobei alle X-Gruppen in Orthoposition stehen und Y1 + Y2 = 1, 2, 3 oder 4.

**13.** Verfahren nach Anspruch 1, wobei jedes X für Iod steht.

**14.** Verfahren nach Anspruch 1, wobei das hydrolytisch instabile Polymer eine oder mehrere Einheiten umfasst, definiert durch die Formel I:

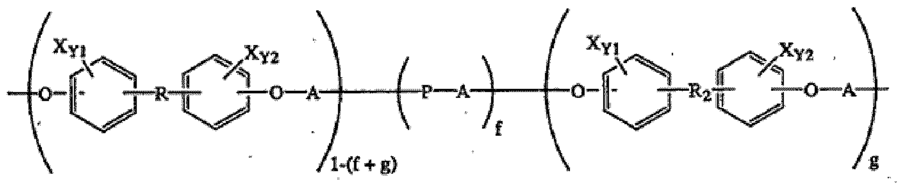

(I)

wobei jedes X unabhängig für I oder Br steht, Y1 und Y2 für jede Diphenoleinheit unabhängig für 0 bis einschließlich 4 stehen und Y1 + Y2 für jede Diphenoleinheit 0 bis einschließlich 8 beträgt,
wobei R und $R_2$ für jede Einheit unabhängig für eine Alkyl-, Aryl- oder Alkylarylgruppe steht, welche bis zu 18 Kohlenstoffatome und 0 bis 8 Heteroatome enthält, ausgewählt aus O und N, und $R_2$ ferner eine t-Butylestergruppe als Seitengruppe umfasst; wobei A entweder für

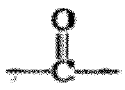 oder 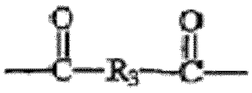

**steht;**
wobei $R_3$ für eine gesättigte oder ungesättigte, substituierte oder unsubstituierte Alkyl-, Aryl-oder Alkylarylgruppe steht, welche bis zu 18 Kohlenstoffatome und 0 bis 8 Heteroatome enthält, ausgewählt aus O und N;
wobei P für eine Poly-($C_{1-4}$-alkylenglykol)-Einheit steht; f im Bereich von 0 bis weniger als 1 liegt; g im Bereich von 0 bis einschließlich 1 liegt und f + g im Bereich von 0 bis einschließlich 1 liegt.

**15.** Verfahren nach Anspruch 14, wobei $R_2$ Folgendes umfasst:

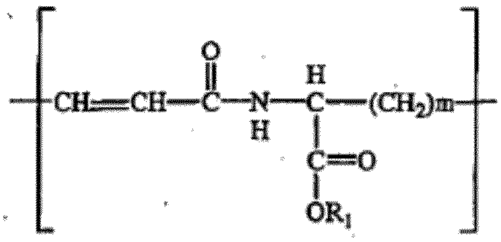 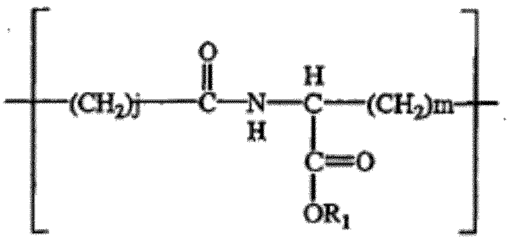

oder

wobei j und m unabhängig für eine ganze Zahl von 1 bis 8 stehen und $R_1$ eine tert-Butyl(tB)-Gruppe ist.

**16.** Verfahren nach Anspruch 14, wobei alle X-Gruppen in Orthoposition stehen und Y1 + Y2 = 1, 2, 3 oder 4.

**17.** Verfahren nach Anspruch 14, wobei jedes X für Iod steht.

**18.** Verfahren nach Anspruch 1, wobei das Polymer ein Polycarbonat, ein Polyarylat, ein Polyiminocarbonat oder ein phosphorhaltiges Polymer ist, wobei phosphorhaltige Polymere die folgende Struktur aufweisen:

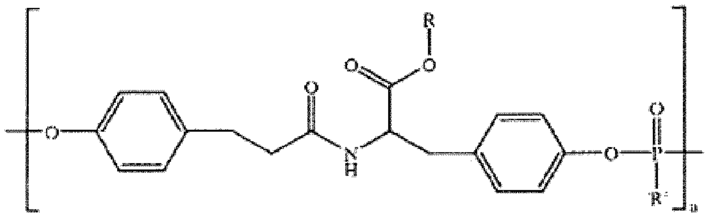

wobei entweder:

R für tert-Butyl steht,
R' aus Alkyl, Alkoxy, Aryl, Aryloxy, einer heterocyclischen Gruppe oder Heterocycloxy ausgewählt ist und
n 2 bis 500 beträgt;

oder

R für tert-Butyl steht, R' aus H, Alkyl, Alkoxy, Aryl, Aryloxy, einer heterocyclischen Gruppe oder Heterocycloxy ausgewählt ist und n 5 bis 500 beträgt.

**19.** Verfahren nach Anspruch 18, wobei das Polymer ein halogensubstituiertes röntgendichtes Polymer ist.

**20.** Verfahren nach Anspruch 18, wobei das Polymer mit bis zu 75 Gew.-% eines Poly-($C_{1-4}$-alkylenglykols) copolymerisiert ist.

**Revendications**

**1.** Procédé de retrait sélectif d'un groupe de protection tert-butyle d'un polymère hydrolytiquement instable pour former une nouvelle composition polymère ayant un groupe acide carboxylique libre à la place dudit groupe de protection tert-butyle, ledit procédé comprenant la dissolution dudit polymère hydrolytiquement instable dans un solvant comprenant une quantité d'un acide ayant une pKa de 0 à 4, dans lequel ledit polymère hydrolytiquement instable comprend une ou plusieurs unités décrites par la Formule III :

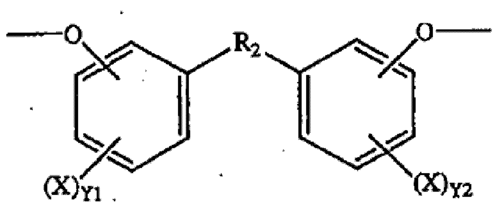
(III)

dans laquelle X pour chaque unité polymère représente indépendamment Br ou I, Y1 et Y2 sont indépendamment compris entre 0 et 4 inclus, et Y1 + Y2 est compris entre 0 et 8 inclus, et $R_2$ pour chaque unité représente indépendamment un groupe alkyle, un groupe aryle, ou un groupe alkylaryle contenant jusqu'à 18 atomes de carbone et de 0 à 8 hétéroatomes sélectionnés parmi O et N, et $R_2$ comprend en outre un groupe de protection tert-butyle pendant.

**2.** Procédé selon la revendication 1, dans lequel ledit polymère hydrolytiquement instable est soluble dans ledit solvant.

**3.** Procédé selon la revendication 1, dans lequel ledit solvant est sensiblement constitué dudit acide.

**4.** Procédé selon la revendication 1, dans lequel ledit solvant est sélectionné dans le groupe constitué du chloroforme, du chlorure de méthylène, du tétrahydrofurane, du diméthylformamide, et de mélanges de ceux-ci.

5. Procédé selon la revendication 1, dans lequel ledit acide est sélectionné dans le groupe constitué de l'acide formique, de l'acide trifluoroacétique, de l'acide chloroacétique, et de mélanges de ceux-ci.

6. Procédé selon la revendication 1, dans lequel ledit acide est l'acide formique.

7. Procédé selon la revendication 1, dans lequel ledit polymère est copolymérisé avec jusqu'à 75 % d'un poly(alkylène glycol en $C_1$-$C_4$).

8. Procédé selon la revendication 7, dans lequel la fraction en poids dudit poly(alkylène glycol en $C_1$-$C_4$) est inférieure à 50 % en poids.

9. Procédé selon la revendication 1, dans lequel $R_2$ comprend :

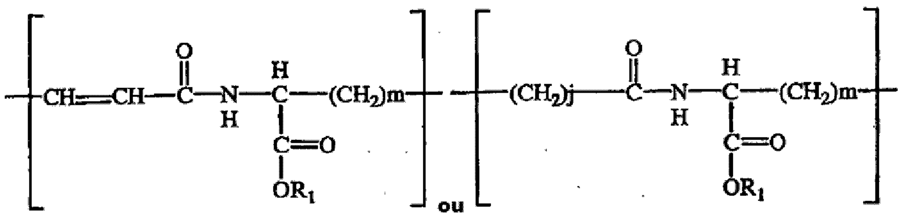

dans laquelle j et m sont indépendamment un nombre entier valant de 1 à 8 inclus, et $R_1$ représente un groupe tert-butyle.

10. Procédé selon la revendication 1, dans lequel ledit polymère est copolymérisé avec jusqu'à 75 % en poids d'un poly(alkylène glycol en $C_1$-$C_4$).

11. Procédé selon la revendication 10, dans lequel la fraction en poids dudit poly(alkylène glycol en $C_1$-$C_4$) est inférieure à 40 % en poids.

12. Procédé selon la revendication 1, dans lequel tous les groupes X sont ortho-dirigés, et Y1 + Y2 = 1, 2, 3 ou 4.

13. Procédé selon la revendication 1, dans lequel chaque X est de l'iode.

14. Procédé selon la revendication 1, dans lequel ledit polymère hydrolytiquement instable comprend une ou plusieurs unités définies par la formule I :

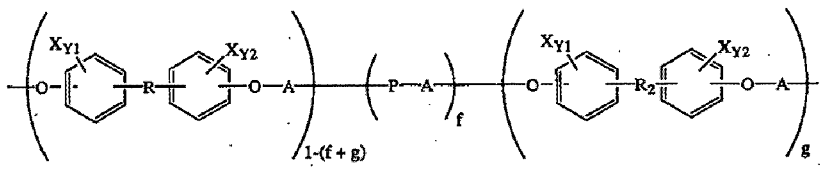
(I)

dans laquelle chaque X représente indépendamment I ou Br, Y1 et Y2 pour chaque unité diphénol sont indépendamment compris entre 0 et 4 inclus, et Y1 + Y2 pour chaque unité diphénol est compris entre 0 et 8 inclus, dans laquelle R et $R_2$ pour chaque unité représentent indépendamment un groupe alkyle, un groupe aryle, ou un groupe alkylaryle contenant jusqu'à 18 atomes de carbone et de 0 à 8 hétéroatomes sélectionnés parmi O et N, et $R_2$ comprend en outre un groupe t-butyl-ester pendant ; dans laquelle A est :

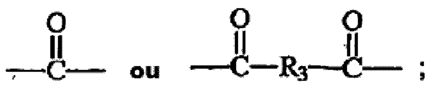

dans laquelle $R_3$ représente un groupe alkyle, aryle ou alkylaryle saturé ou insaturé, substitué ou non substitué contenant jusqu'à 18 atomes de carbone et de 0 à 8 hétéroatomes sélectionnés parmi O et N ;
dans laquelle P représente une unité poly(alkylène glycol en $C_1$-$C_4$) ; f se trouve dans la plage de 0 à moins de 1 ; g se trouve dans la plage de 0 à 1 inclus ; et f + g se trouve dans la plage de 0 à 1 inclus.

**15.** Procédé selon la revendication 14, dans lequel $R_2$ comprend :

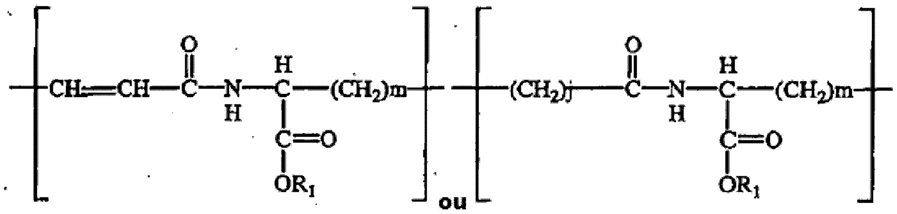

ou

dans laquelle j et m sont indépendamment un nombre entier de 1 à 8 inclus ; et $R_1$ est un groupe tert-butyle (tB).

**16.** Procédé selon la revendication 14, dans lequel tous les groupes X sont ortho-dirigés, et Y1 + Y2 = 1, 2, 3 ou 4.

**17.** Procédé selon la revendication 14, dans lequel chaque X est de l'iode.

**18.** Procédé selon la revendication 1, dans lequel ledit polymère est un polycarbonate, un polyarylate, un polyiminocarbonate ou un polymère contenant du phosphore, dans lequel les polymères contenant du phosphore ont la structure suivante :

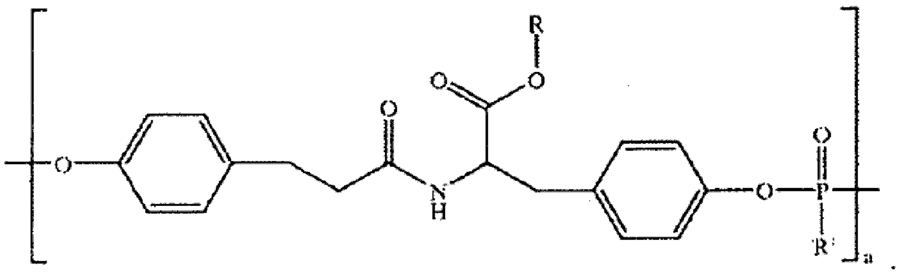

dans laquelle soit :

R est un groupe tert-butyle ;
R' est sélectionné parmi les groupes alkyle, alcoxy, aryle, aryloxy, hétérocyclique ou hétérocycloxy, et
n est compris entre 2 et 500 ;

soit :

R est un groupe tert-butyle, R' est sélectionné parmi H, un groupe alkyle, alcoxy, aryle, aryloxy, hétérocyclique ou hétérocycloxy et n est compris entre 5 et 500.

**19.** Procédé selon la revendication 18, dans laquelle ledit polymère est un polymère radio-opaque à substitution halogène.

**20.** Procédé selon la revendication 18, dans lequel ledit polymère est copolymérisé avec jusqu'à 75 % en poids d'un poly(alkylène glycol en $C_1$-$C_4$).

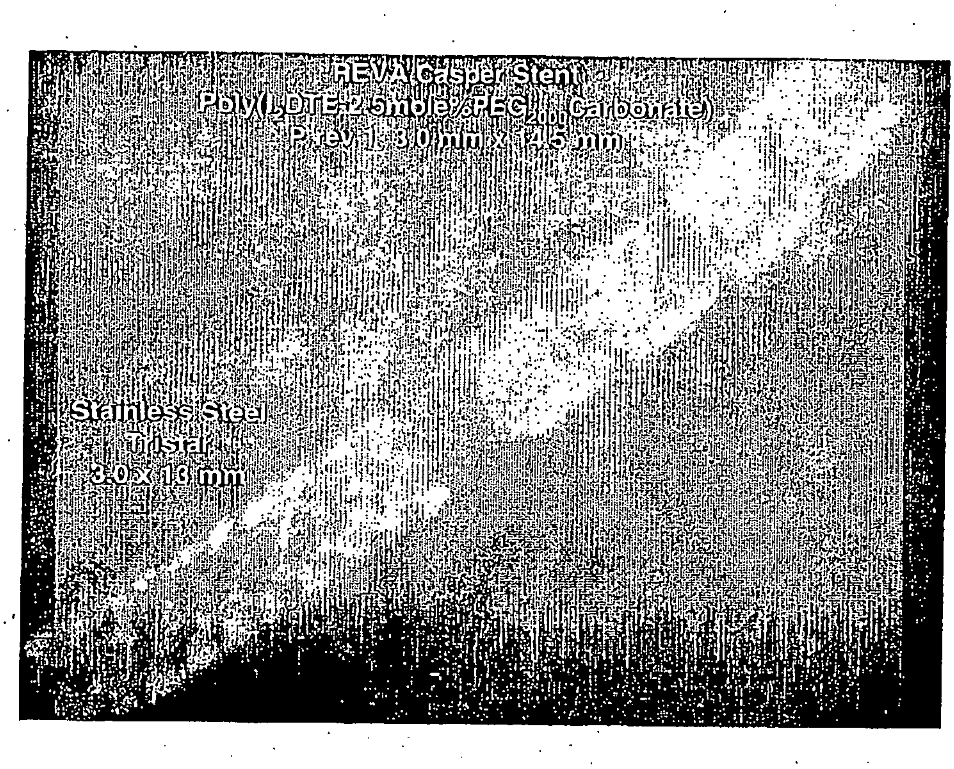


FIG. 1

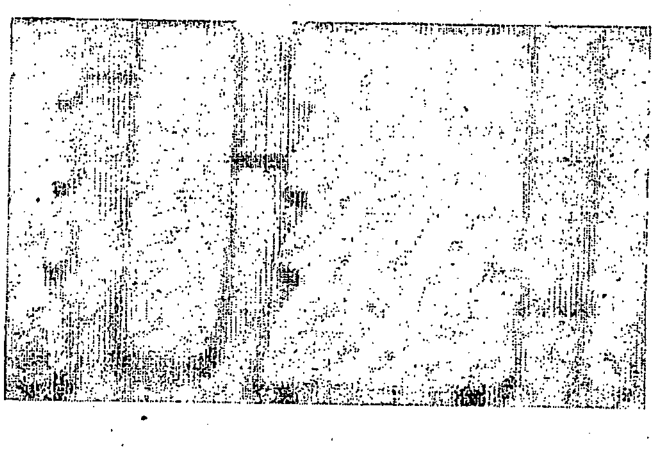

FIG. 2A

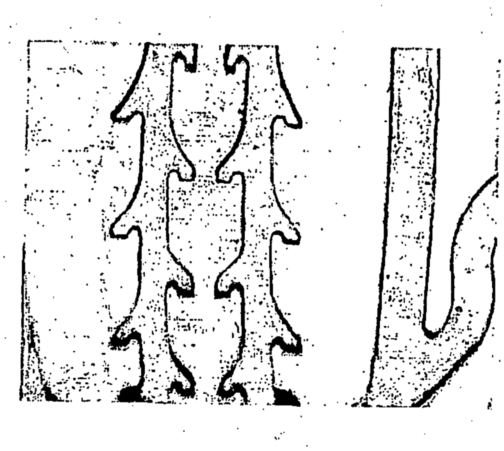

FIG. 2B

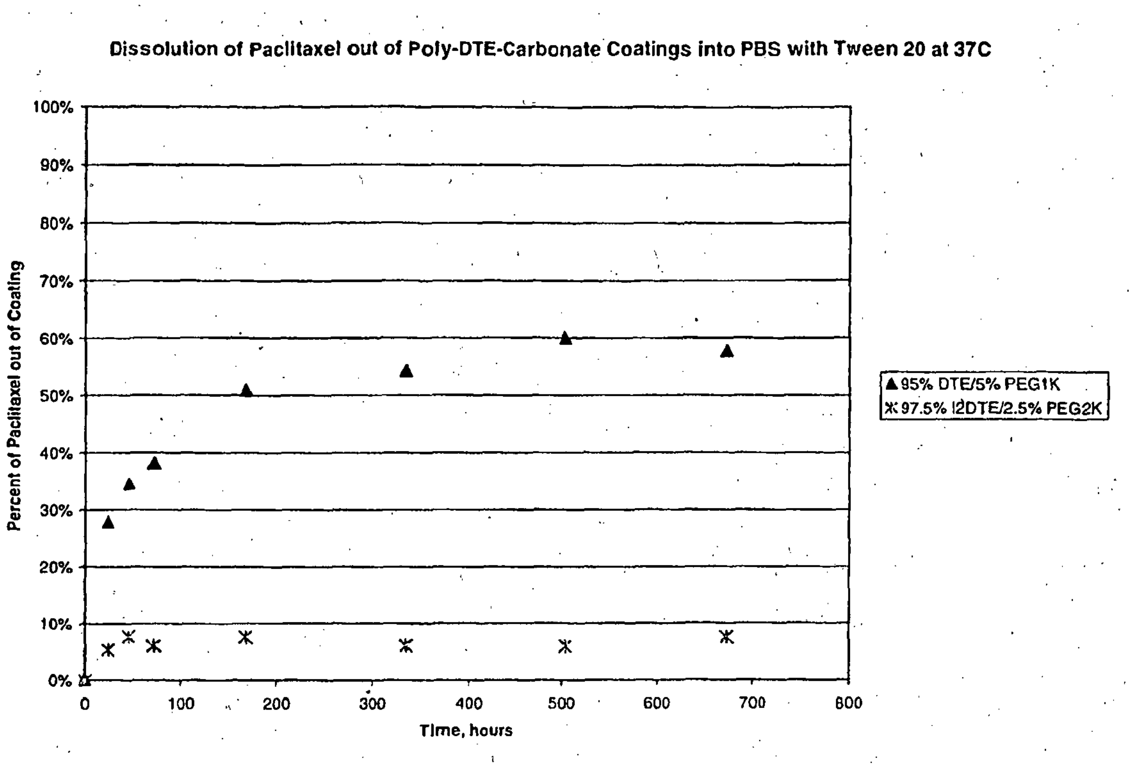
Dissolution of Paclitaxel out of Poly-DTE-Carbonate Coatings into PBS with Tween 20 at 37C
Percent of Paclitaxel out of Coating
100%
90%
80%
70%
60%
50%
40%
30%
20%
10%
0%
0
100
200
300
400
500
600
700
800
Time, hours
95% DTE/5% PEG1K
97.5% I2DTE/2.5% PEG2K

FIG. 3

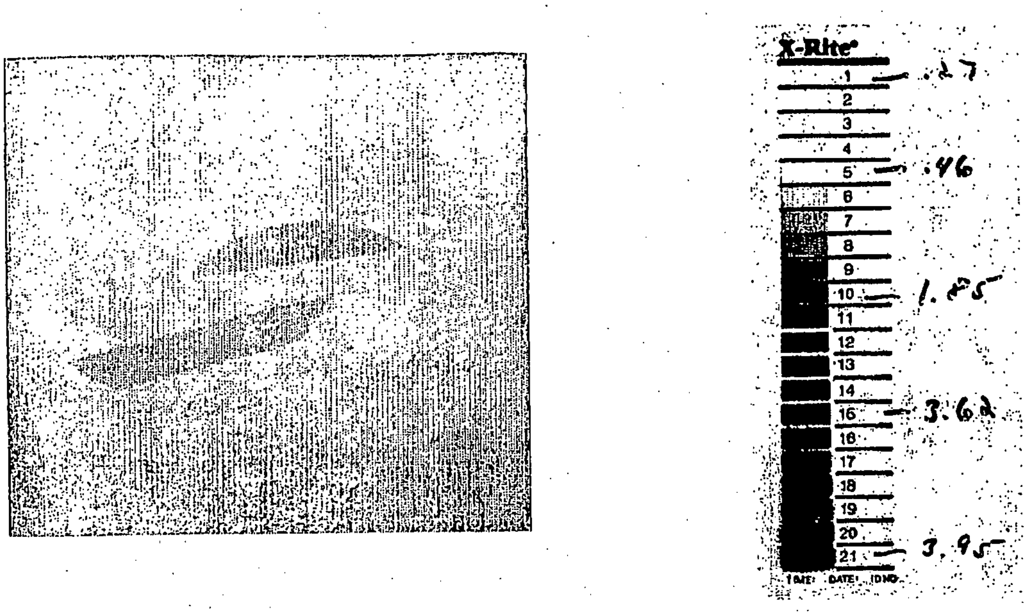
X-Rite
1
2
3
4
5
6
7
8
9
10
11
12
13
14
15
16
17
18
19
20
21
.27
.46
1.85
3.62
3.95

FIG. 4a

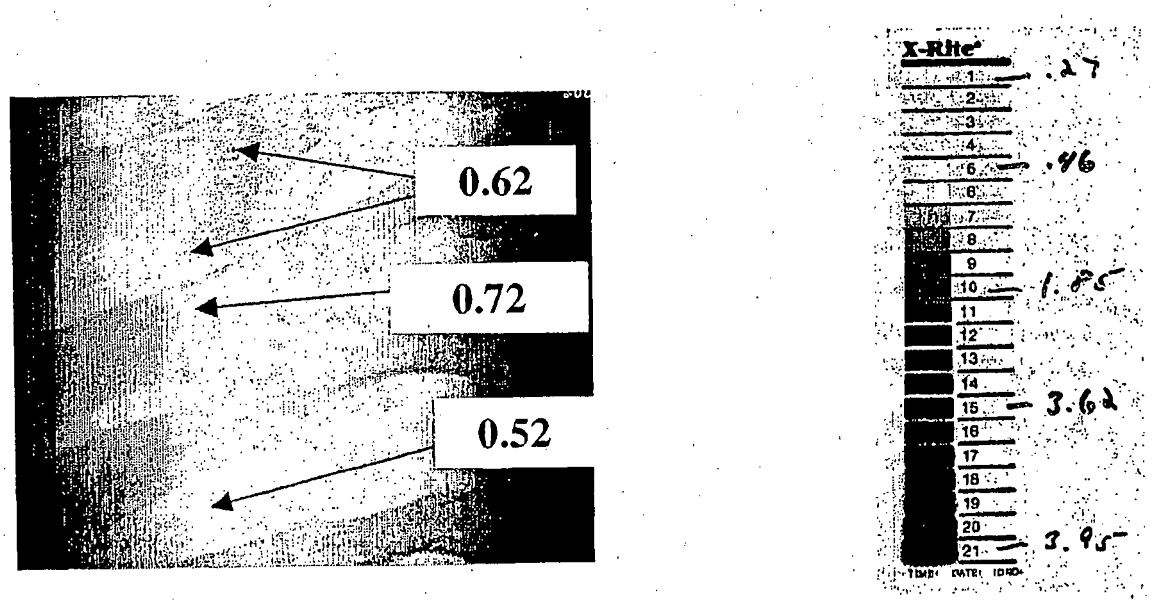
0.62
0.72
0.52
X-Rite
1
2
3
4
5
6
7
8
9
10
11
12
13
14
15
16
17
18
19
20
21
.27
.46
1.85
3.62
3.95

FIG. 4b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6475477 B
- US 4762129 A, Bonzel
- US 5232445 A
- US 4748982 A, Yock
- US 5496346 A
- US 5626600 A
- US 5040548 A
- US 5061273 A
- US 5350395 A
- US 5451233 A
- US 5749888 A
- US 5092877 A
- US 5108416 A
- US 5197978 A
- US 5300085 A
- US 5445646 A
- US 5496275 A
- US 5545135 A
- US 5545138 A
- US 5549556 A
- US 5755708 A
- US 5769868 A
- US 5800393 A
- US 5836965 A
- US 5989280 A
- US 6019785 A
- US 6036715 A
- US 5242399 A
- US 5158548 A
- US 6007545 A
- US 5151100 A
- US 5230349 A
- US 6447508 B
- US 6562021 B
- WO 9014046 A1
- US 5910816 A
- US 5423321 A
- US 6033436 A
- US 6224626 A
- US 6623521 A
- US 10897235 B
- US 4733665 A
- US 5344426 A, Lau
- US 5549662 A
- US 5733328 A, Fordenbacher
- US 5735872 A
- US 5876419 A, Carpenter
- US 5741293 A, Wijay
- US 5984963 A, Ryan
- US 5441515 A
- US 5618299 A, Khosravi
- US 5059211 A
- US 5306286 A
- US 5527337 A, Stack
- US 5443500 A, Sigwart
- US 5449382 A, Dayton
- US 6409752 B, Boatman
- US 4740207 A, Kreamer
- US 4877030 A, Beck
- US 5007926 A, Derbyshire
- US 4954126 A, Wallsten
- US 5192307 A, Wall
- US 6224626 B
- US 6623521 B, Steinke
- US 5099060 A
- US 5587507 A
- US 5658995 A
- US 5670602 A
- US 6120491 A
- US 6284462 B
- US 4863735 A
- US 6238687 B
- US 5912225 B
- US 6602497 B
- US 5912225 A


### Non-patent literature cited in the description


- Heart and Stroke Statistical Update. American Heart Association, 2001
- **M. BODANSZKY.** Principles of peptide synthesis. Springer Verlag, 1984
- **BALCON et al.** Recommendations on Stent Manufacture, Implantation and Utilization. *European Heart Journal,* 1997, vol. 18, 1536-1547
- **PHILLIPS et al.** The Stenter's Notebook. Physician's Press, 1998
- **YASUKAWA et al.** *Circulation,* 1996
- **CHEN et al.** *Circulation,* 1995
- **HU et al.** *Circulation,* 1999
- **KURISU et al.** *Hiroshima J. Med Sci,* 1997

- **BRAUNER et al.** *J Thorac Cardiovasc Surg,* 1997
- **E LUTGENS et al.** *Nature Medicine,* 1999
- **MORISHITA et al.** *Hypertension,* 1993
- **VON DER LEYEN et al.** *Proc Natl Acad Sci,* 1995
- **CARMELIET et al.** *Circulation,* 1997
- **AUTIERI et al.** *Biochem Biophys Res Commun,* 1994
- **SCHNELL.** Chemistry and Physics of Polycarbonates. Interscience, 1964
- **ABRAMSON et al.** Small changes in polymer structure can dramatically increase degradation rates: the effect of free carboxylate groups on the properties of tyrosine-derived polycarbonates. *Sixth World Biomaterials Congress Transactions, Society for Biomaterials 26th Annual Meeting,* 2000